# EUROPÄISCHE PATENTSCHRIFT

(11) **EP 3 712 618 B1**
(45) Veröffentlichungstag und Bekanntmachung des Hinweises auf die Patenterteilung: **04.10.2023**
(21) Anmeldenummer: 19163553.1
(22) Anmeldetag: 18.03.2019
(51) Int. Cl.: G01N 33/564, G06T 7/00

(54) **VERFAHREN ZUM DETEKTIEREN EINER BINDUNG VON ANTIKÖRPERN EINER PATIENTENPROBE AN DOPPELSTRÄNGIGE DNS UNTER VERWENDUNG VON CRITHIDIA LUCILIAE ZELLEN UND FLUORESZENZMIKROSKOPIE**
METHOD FOR DETECTING A BINDING OF ANTIBODIES OF A PATIENT SAMPLE TO DOUBLE-STRANDED DNA USING CRITHIDIA LUCILIAE CELLS AND FLUORESCENCE MICROSCOPY
PROCÉDÉ DE DÉTECTION D'UNE LIAISON D'ANTICORPS D'UN ÉCHANTILLON DE PATIENT À L'ADN DOUBLE BRIN À L'AIDE DES CELLULES DE CRITHIDIA LUCILIAE ET MICROSCOPIE PAR FLUORESCENCE

(43) Veröffentlichungstag der Anmeldung: 23.09.2020
(73) Patentinhaber: Euroimmun Medizinische Labordiagnostika AG, 23560 Lübeck (DE)
(72) Erfinder: GERLACH, Stefan, 23627 Groß Grönau (DE); MARZAHL, Christian, 91052 Erlangen (DE); DANCKWARDT, Maick, 23919 Rondeshagen (DE); VOIGT, Jörn, 23558 Lübeck (DE)

(56) Entgegenhaltungen:
- GABRIELLA LAKOS ET AL: "Detection of anti-dsDNA antibodies by computer-aided automated immunofluorescence analysis", JOURNAL OF IMMUNOLOGICAL METHODS., Bd. 433, 1. Juni 2016 (2016-06-01), Seiten 17-22, XP055501252, NL ISSN: 0022-1759, DOI: 10.1016/j.jim.2016.02.019
- SODA P ET AL: "A decision support system for Crithidia Luciliae image classification", ARTIFICIAL INTELLIGENCE IN MEDICINE, ELSEVIER, NL, Bd. 51, Nr. 1, 1. Januar 2011 (2011-01-01) , Seiten 67-74, XP027587179, ISSN: 0933-3657 [gefunden am 2010-07-14]
- GERLACH S ET AL: "Evaluation of Crithidia luciliae IFT can be reliably automated with EUROPattern", CLINICAL CHEMISTRY, AMERICAN ASSOCIATION FOR CLINICAL CHEMISTRY, US, Bd. 64, Nr. Supplement 1, 1. Januar 2018 (2018-01-01), Seite S125, XP009513229, ISSN: 1530-8561
- STEFAN GERLACH ET AL: "Automated Evaluation of Crithidia luciliae Based Indirect Immunofluorescence Tests: A Novel Application of the EUROPattern-Suite Technology", JOURNAL OF IMMUNOLOGY RESEARCH, Bd. 2015, 1. Januar 2015 (2015-01-01), Seiten 1-8, XP055588770, ISSN: 2314-8861, DOI: 10.1155/2015/742402
- BAYRAMOGLU NESLIHAN ET AL: "Human Epithelial Type 2 cell classification with convolutional neural networks", 2015 IEEE 15TH INTERNATIONAL CONFERENCE ON BIOINFORMATICS AND BIOENGINEERING (BIBE), IEEE, 2. November 2015 (2015-11-02), Seiten 1-6, XP032839513, DOI: 10.1109/BIBE.2015.7367705 [gefunden am 2015-12-28]

## Beschreibung

Die Erfindung betrifft ein Verfahren und eine Vorrichtung zum Detektieren einer Bindung von Autoantikörpern einer Patientenprobe an doppelsträngige Desoxyribonukleinsäure (DNS) unter Verwendung von Crithidia luciliae Zellen mittels Fluoreszenzmikroskopie und mittels digitaler Bildverarbeitung.

Der Nachweis von Autoantikörpern gegen Desoxyribonucleinsäuren (DNS) ist beispielsweise für die Diagnose des SLE (Synonym: Lupus erythematodes disseminatus) von entscheidender Bedeutung. Hierbei müssen grundsätzlich zwei Typen unterschieden werden: Antikörper gegen dsDNS und Antikörper gegen einzelsträngige, denaturierte DNS (Einzelstrang-DNS, ssDNS). Antikörper gegen dsDNS reagieren mit Epitopen, die im Desoxyribosephosphat-Gerüst der DNS liegen. Dagegen binden sich Antikörper gegen ssDNS vorwiegend an Epitope aus dem Bereich der Purin bzw. Pyrimidinbasen. Sie können aber auch Epitope des Desoxyribosephosphat-Gerüsts erkennen. Anti-dsDNS-Antikörper findet man fast ausschließlich beim SLE. Ihre Prävalenz beträgt, je nach Nachweismethode und Krankheitsaktivität, 20 % bis 90 %. Anti-dsDNS-Antikörper werden mitunter auch bei Patienten mit anderen Autoimmunerkrankungen und Infektionen sowie in seltenen Fällen bei klinisch gesunden Personen nachgewiesen. Letztere entwickeln in 85 % der Fälle einen SLE innerhalb von 5 Jahren nach dem Anti-dsDNS-Erstnachweis. Allerdings lässt sich ein SLE nicht ganz ausschließen, wenn keine Antikörper gegen dsDNS vorliegen. Der SLE ist eine systemische Autoimmunerkrankung aus der Gruppe der Kollagenosen. Die Diagnose richtet sich nach den 1997 modifizierten 11 Kriterien des American College of Rheumatology (ACR). Bei Vorhandensein von 4 der 11 Kriterien kann mit 80- bis 90 %-iger Sicherheit die Diagnose eines SLE gestellt werden.

Eine indirekte Immunfluoreszenz ist ein in-vitro-Test für die Bestimmung humaner Antikörper gegen dsDNS. Es können beispielsweise sogenannte BIOCHIPs als Substrate dienen, die mit Crithidia luciliae Ausstrichen beschichtet sind. Diese werden beispielsweise mit verdünnten Patientenproben inkubiert. Bei positiven Reaktionen binden sich spezifische Antikörper an die Antigene. Gebundene Antikörper (IgG) werden in einem zweiten Inkubationsschritt beispielsweise mit Fluorescein-markierten Anti-Human-Antikörpern angefärbt und im Fluoreszenzmikroskop sichtbar gemacht.

Aus dem Stand der Technik ist es daher bekannt, dass ein Substrat bereitgestellt wird, bei welchem mehrere Crithidia luciliae Zellen auf dem Substrat fixiert sind. Eine solche Fixierung kann beispielsweise mittels Ethanol vorgenommen sein.

Das Substrat wird dann mit einer Patientenprobe, vorzugsweise verdünntem Blutserum, inkubiert, wobei die Patientenprobe potentiell die zu detektierenden Autoantikörper aufweist. Nach dem Stand der Technik können dann die Zellen bzw. das Substrat mit einem sogenannten Konjugat inkubiert werden, welches sekundäre Antikörper aufweist, welche mit einem beispielsweise grünen Fluoreszenzfarbstoff markiert sind.

Es kann dann nach Bestrahlen des inkubierten Substrates mit Anregungslicht eine durch den grünen Fluoreszenzfarbstoff emittierte Fluoreszenzstrahlung als ein Mikroskopbild einer Fluoreszenzmikroskopie erfasst werden.

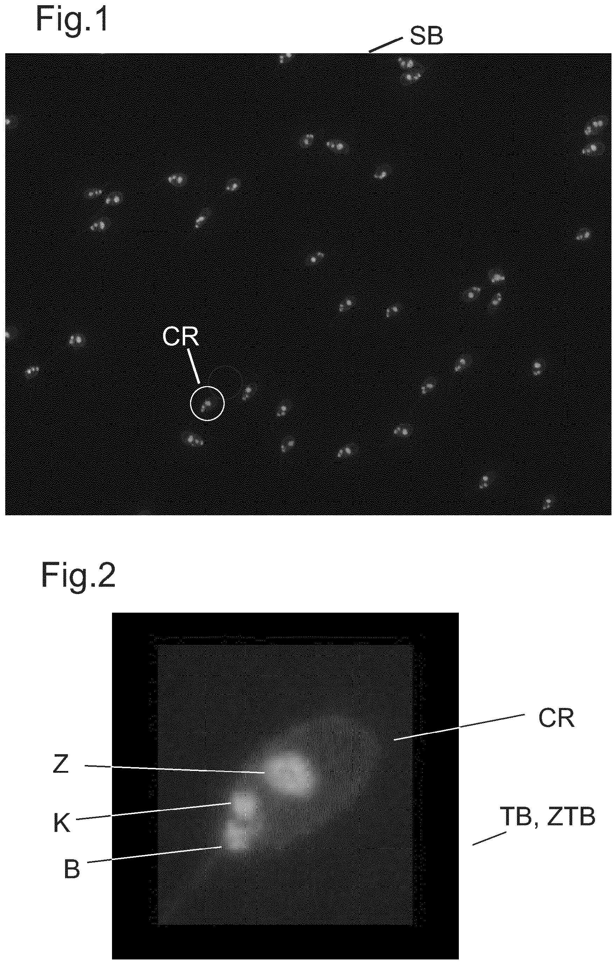

Ein solches Mikroskopbild ist beispielhaft in der Figur 1 dargestellt als das Bild SB.

Eine einzelne Crithidien Zelle CR aus der Figur 1 ist noch einmal in der Figur 2 detaillierter dargestellt. Ein solches Teilbild TB einer Crithidia luciliae Zelle CR zeigt deutlich eine Färbung am Kinetoplasten K, welcher auch als Mitochondrium bezeichnet wird. Weitere Färbungen sind am Zellkern Z als auch am Basalkörper B gegeben.

Für ein zuverlässiges Detektieren einer Bindung von Autoantikörpern aus der Patientenprobe an doppelsträngige DNS ist es entscheidend, eine Färbung von mehreren Kinetoplasten in dem Fluoreszenzbild SB des Substrates zu detektieren. Wie die Figur 2 zeigt, kann eine Bindung bzw. eine Färbung auch für einen Zellkern Z als auch für ein Basalkörper B gegeben sein, so dass mittels Bildverarbeitung der Kinetoplast K zuverlässig hinsichtlich seiner Lage im Bild bestimmt werden muss.

Durch eine Auswertung bezüglich einer jeweiligen Färbung jeweiliger Kinetoplasten jeweiliger Crithidia luciliae Zellen in dem Fluoreszenzbild SB kann dann insgesamt eine im Mittel gesehene Gesamtbindung von Autoantikörpern der Patientenprobe an doppelsträngige DNS ermittelt werden.

Aufgabe ist es also, ein Verfahren unter Verwendung digitaler Bildverarbeitung bereitzustellen, um eine Bindung von Autoantikörpern aus einer Patientenprobe an doppelsträngige DNS zu bestimmen, wobei eine Färbung unterschiedlicher Kinetoplastbereiche unterschiedlicher Crithidia luciliae Zellen innerhalb eines Fluoreszenzbildes sicher bestimmt werden kann.

Das Dokument Gabriella Lakos et al., "Detection of anti-dsDNA antibodies by computeraided automated immunofluorescence analysis", Journal of Immunological Methods, NL, (20160601), vol. 433, doi:10.1016/j.jim.2016.02.019, ISSN 0022-1759, pages 17 - 22, XP055501252 [X], offenbart eine Verwendung eines computerunterstützendes Mikroskops zur Durchführung und zur Analyse von IFT Messungen. Hierbei werden die Zellen mit zwei Fluoreszenzfarbstoffen inkubiert und es wird jeweils ein Bild erstellt. Das eine der Bilder dient zum genauen Identifizieren der Zellen, das andere Bild dient zum Vermessen einer Bindung von Antikörpern an Kinetoplasten.

In dem Dokument Soda P et al., "A decision support system for Crithidia Luciliae image classification", Artificial Intelligence In Medicine, Elsevier, NL, vol. 51, no. 1, ISSN 0933-3657, (20110101), pages 67 - 74, (20100714), XP027587179 [I], wird ebenfalls ein IFT Verfahren für den Nachweis von Antikörpern offenbart. Zunächst werden Zellen mit angefärbten Kinetoplasten in Fluoreszenzaufnahmen identifiziert. Anschließend werden verschiedene Parameter einer Zelle extrahiert und vermessen.

Das Dokument Gerlach S et al., "Evaluation of Crithidia luciliae IFT can be reliably automated with EUROPattern", Clinical Chemistry, American Association For Clinical Chemistry, US, (20180101), vol. 64, no. Supplement 1, ISSN 1530-8561, page S125, XP009513229 [I], offenbart eine Software in Form einer EUROPattern-Suite zur Analyse von IFT Bildern.

Das Dokument Stefan Gerlach et al., "Automated Evaluation of Crithidia luciliae Based Indirect Immunofluorescence Tests: A Novel Application of the EUROPattern-Suite Technology", Journal of Immunology Research, (20150101), vol. 2015, doi:10.1155/2015/742402, ISSN 2314-8861, pages 1 - 8, XP055588770 [I], offenbart ebenfalls eine Software des Namens EUROPattern-Suites zur Analyse von IFT Bildern. Zwei verschiedene Fluoreszenzfarbstoffe zum jeweiligen Anfärben der Zellen und Kinetoplasten kommen zum Einsatz. Zellen werden auf segmentierten Bildern mittels eines der Farbstoffe identifiziert.

Anschließend wird eine Fluoreszenz des Kinetoplasten dem anderen Bild vermessen, um die Bindung von Antikörpern zu bestimmen. Es wird ein Gesamtbindungsmaß basierend auf den vermessenen Kinetoplasten erstellt.

Das Dokument Bayramoglu Neslihan et al, "Human Epithelial Type 2 cell classification with convolutional neural networks", 2015 IEEE 15th International Conference on Bioinformatics and Bioengineering (BIBE), IEEE, (20151102), doi:10.1109/BIBE.2015.7367705, pages 1 - 6, XP032839513 [A], offenbart eine Verwendung eines Convolutional Neural Network zur automatisierten Bildanalyse von Fluoreszenzaufnahmen. Zum Nachweis von Antikörpern wurden HeP-2-Zellen als Substrat mit einer Probe und fluoreszenzmarkierten Sekundärantikörpern markiert und mittels indirekter Fluoreszenzmikroskopie nachgewiesen.

Vorgeschlagen wird daher ein Verfahren zum Detektieren einer Bindung von Autoantikörpern einer Patientenprobe an doppelsträngige DNS unter Verwendung von Crithidia luciliae Zellen mittels Fluoreszenzmikroskopie und mittels digitaler Bildverarbeitung, welches unterschiedliche Schritte aufweist. Es erfolgt zunächst ein Bereitstellen eines Substrates, welches mehrere Crithidia luciliae Zellen aufweist. Das Substrat wird dann mit der Patientenprobe inkubiert, welche potenziell die Autoantikörper aufweist. Anschließend wird das Substrat mit einem ersten Fluoreszenzfarbstoff inkubiert, welcher vorzugsweise eine Färbung in einem Rotkanal erzeugt. Ferner wird das Substrat mit einem Konjugat bzw. mit sekundären Antikörpern inkubiert, welche jeweils mit einem zweiten Fluoreszenzfarbstoff, vorzugsweise einem grünen Fluoreszenzfarbstoff, markiert sind, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wird. Es erfolgt ferner ein Erfassen eines ersten Fluoreszenzbildes des Substrates in einem ersten Farbkanal, vorzugsweise einem roten Farbkanal, welcher zu dem ersten Fluoreszenzfarbstoff korrespondiert. Das Erfassen erfolgt vorzugsweise mittels eines Fluoreszenzmikroskops. Ferner erfolgt ein Erfassen eines zweiten Fluoreszenzbildes des Substrates in einem zweiten Farbkanal, vorzugsweise einem grünen Farbkanal, welcher zu dem zweiten Fluoreszenzfarbstoff korrespondiert. Das Erfassen erfolgt vorzugsweise mittels des Fluoreszenzmikroskops. Es erfolgt dann ferner ein Identifizieren jeweiliger erster Teilbilder in dem ersten Fluoreszenzbild, wobei die ersten Teilbilder jeweils wenigstens eine Crithidia luciliae Zellen repräsentieren. Ferner erfolgt ein Bestimmen jeweiliger zweiter Teilbilder des zweiten Fluoreszenzbildes, welche zu den jeweiligen ersten Teilbildern des ersten Fluoreszenzbildes korrespondieren. Es erfolgt ferner ein jeweiliges Prozessieren wenigstens einer Teilmenge der jeweiligen zweiten Teilbilder mittels eines vortrainierten Convolutional Neural Network (CNN) zur Bestimmung jeweiliger Bindungsmaße. Die jeweiligen Bindungsmaße indizieren einen jeweiligen Grad einer jeweiligen Bindung von Autoantikörpern in einem jeweiligen Kinetoplastbereich einer jeweiligen Crithidia luciliae Zelle eines jeweiligen zweiten Teilbildes. Das Convolutional Neural Network prozessiert ein jeweiliges zweites Teilbild für sich jeweils separat, um für ein jeweiliges zweites Teilbild ein jeweiliges Subbild zu bestimmen, das einen jeweiligen Kinetoplastbereich einer jeweiligen Crithidia luciliae Zelle repräsentiert. Das CNN bestimmt das jeweilige Bindungsmaß auf Basis des jeweiligen Subbildes. Das CNN generiert im Zuge der Bestimmung der jeweiligen individuellen Bindungsmaße für ein jeweiliges zweites Teilbild eine jeweilige finale Feature-Map. Es wird ferner auf Basis der finalen Feature-Map ein jeweiliges positives Konfidenzmaß, bezogen auf ein Vorliegen der Bindung von Autoantikörpern in einem jeweiligen Kinetoplastbereich für ein jeweiliges zweites Teilbild, bestimmt. Ferner wird eine Teilmenge der zweiten Teilbilder ausgewählt auf Basis der jeweiligen positiven Konfidenzmaße der jeweiligen zweiten Teilbilder. Es erfolgt schließlich ein Bestimmen eines Gesamtbindungsmaßes bezogen auf die Bindung von Autoantikörpern der Patientenprobe an doppelsträngige DNS auf Basis jener Bindungsmaße, welche zu den zweiten Teilbildern der Teilmenge gehören.

Die Patientenprobe ist eine flüssige Patientenprobe, vorzugsweise flüssiges Blut oder flüssige Blutbestandteile. Insbesondere ist die flüssige Patientenprobe flüssiges Blutserum. Vorzugsweise ist die Patientenprobe verdünnt mit sogenanntem Waschpuffer, vorzugsweise sogenanntem PBS Tween.

Das Konjugat weist sekundäre Antikörper auf, welche mit dem zweiten Fluoreszenzfarbstoff markiert sind.

Das vorgeschlagene Verfahren ist insbesondere ein Verfahren zur in vitro Bestimmung eines Vorhandenseins von primären Antikörpern in einer flüssigen Patientenprobe.

Das Substrat ist insbesondere eine biologische Probe, welche tierpathogene Hemoflagellaten der Art Crithidia luciliae aufweist. Diese Einzeller besitzen ein Doppelstrang-DNS haltiges Riesenmitochondrium (Kinetoplast), welches im Wesentlichen keines der übrigen in dem Zellkern enthaltende Antigene aufweist. Primäre Autoantikörper aus der Patientenprobe, die mit dem Kinetoplasten reagieren, sind gegen dsDNS gerichtet.

Die Fluoreszenzmikroskopie ist insbesondere eine sogenannte indirekte Immunfluoreszenzmikroskopie (IIFT Mikroskopie).

Das Substrat wird vorzugsweise mit Anregungsstrahlung beleuchtet, um Fluoreszenzstrahlung des ersten und des zweiten Fluoreszenzfarbstoffes anzuregen. Die Anregungsstrahlung ist vorzugsweise ein blaues Licht und weist somit Wellenlängen in einem Blauspektrum auf. Vorzugsweise ist die Anregungsstrahlung von einer gleichen Wellenlänge bzw. einem gleichen Wellenlängenbereich für den ersten und auch für den zweiten Fluoreszenzfarbstoff. Der erste Fluoreszenzfarbstoff ist vorzugsweise ein roter Fluoreszenzfarbstoff, insbesondere sogenanntes Evans Blue (T-1824). Der zweite Fluoreszenzfarbstoff ist vorzugsweise ein grüner Fluoreszenzfarbstoff, insbesondere des Typs Fluorescein isothiocyanate (FITC).

Zur Erläuterung eines oder mehrerer möglicherweise zu erreichender Vorteile des erfindungsgemäßen Verfahrens werden im Folgenden genauere Ausführung gemacht.

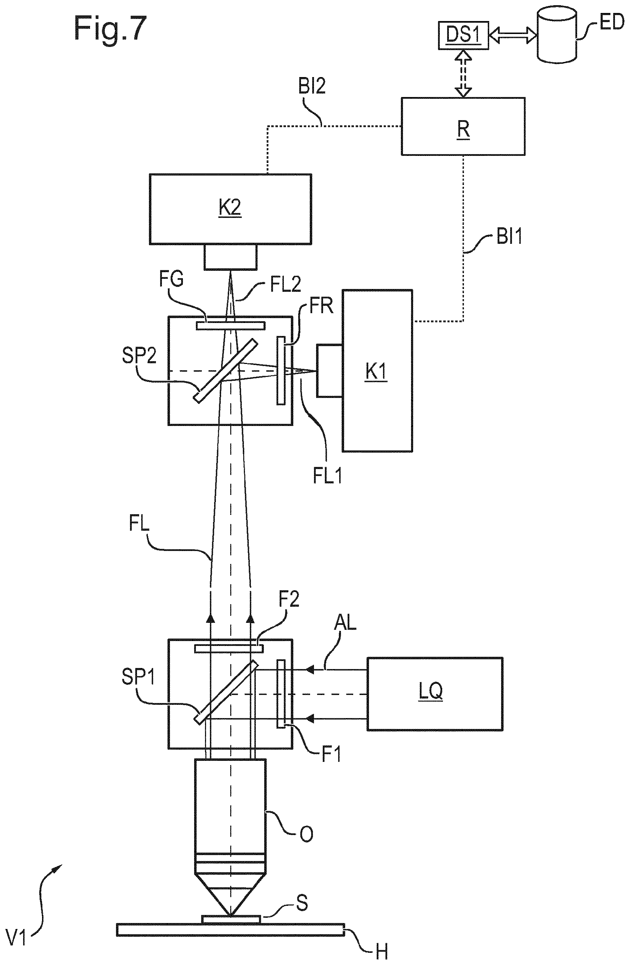

Die Figur 7 zeigt eine Vorrichtung V1, mittels welcher das erfindungsgemäße Verfahren durchgeführt werden kann. Die Vorrichtung V1 kann als ein Fluoreszenzmikroskop bezeichnet werden. Die Vorrichtung V1 weist eine Halterung H für ein Substrat S auf, welches in der zuvor geschriebenen Weise inkubiert wurde. Über eine Optik O wird Anregungslicht AL einer Anregungslichtquelle LQ hin zu Substrat S geleitet. Sich ergebende Fluoreszenzstrahlung FL wird dann in zwei unterschiedlichen Farbkanälen in unterschiedliche Fluoreszenzstrahlungen FL1 und FL2 mittels weiterer optischer Elemente aufgeteilt. Eine Kamera K1 ist eine erste Bilderfassungseinheit und erfasst dann ein erstes Fluoreszenzbild, insbesondere in einem Rotkanal mittels eine Rotfilters FR. Eine weitere Kamera K2 ist eine zweite Bilderfassungseinheit und erfasst dann ein zweites Fluoreszenzbild des Substrates S in einem zweiten Farbkanal, insbesondere einem Grünkanal mittels eines Grünfilters. Die sich ergebenden Fluoreszenzbilder werden als Bildinformationen BI1, BI2 an eine Recheneinheit R bereitgestellt und durch diese verarbeitet. Vorzugsweise ist nur eine einzige Bilderfassungseinheit gegeben, so dass durch temporäres Austauschen der Filter FR, FG die einzige Bilderfassungseinheit die unterschiedlich gefärbten Fluoreszenzbilder der unterschiedlichen Farbkanäle zu unterschiedlichen Zeitpunkten erfasst.

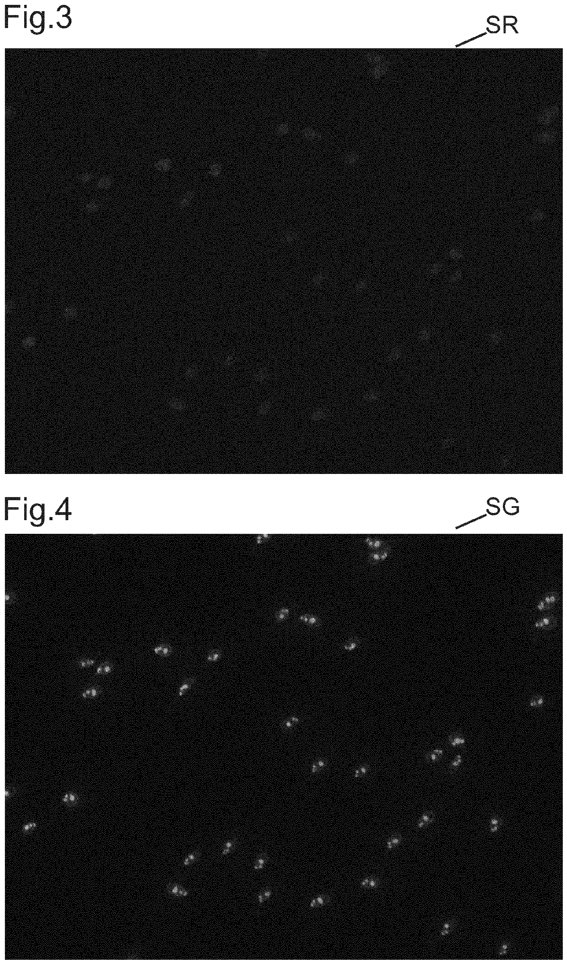

Die Figur 3 zeigt ein beispielhaftes erstes Fluoreszenzbild SR aus einem Rotkanal. Die Figur 4 zeigt ein beispielhaftes zweites Fluoreszenzbild SG in einem Grünkanal.

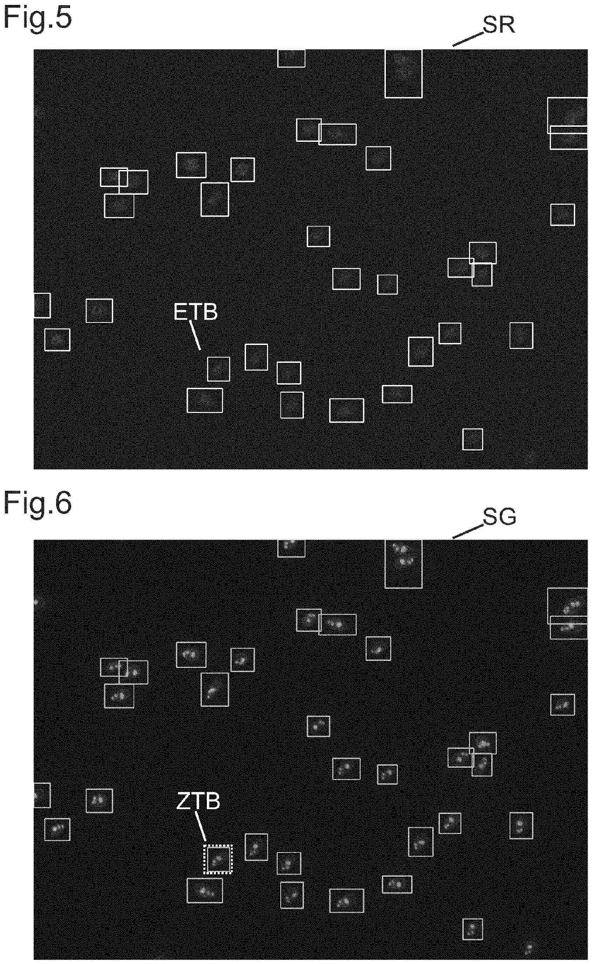

Erfindungsgemäß werden zunächst auf Basis des ersten Fluoreszenzbildes SR aus der Figur 3 erste Teilbilder identifiziert, welche wenigstens eine Crithidia luciliae Zelle aufweisen. Die Figur 5 zeigt hierzu entsprechende durch Rechtecke markierte erste Teilbilder sowie einen bestimmten ersten Teilbildbereich ETB.

Die Figur 6 zeigt zu dem zweiten Fluoreszenzbild SG aus der Figur 4 jeweilige zweite Teilbilder bzw. zweite Teilbildbereiche, welche zu den jeweiligen ersten Teilbildern des ersten Fluoreszenzbildes SR aus der Figur 5 korrespondieren. Insbesondere wird ein bestimmter zweiter Teilbildbereich ZTB in Korrespondenz zu dem bestimmten ersten Teilbildbereich ETB aus der Figur 5 dargestellt. Im Sinne dieser Anmeldung kann ein Teilbildbereich auch als ein Teilbild bezeichnet werden.

Der zweite Teilbildbereich ZTB aus der Figur 6 ist nicht absolut identisch mit dem ersten Teilbildbereich ETB, allerdings korrespondiert er zu ihm, da eine gleiche Crithidia luciliae Zelle durch diese Teilbildbereiche ETB, ZTB erfasst wird. Wurde ein erster Teilbildbereich bzw. ein erstes Teilbild ETB in dem ersten Fluoreszenzbild, wie in der Figur 5 dargestellt, bestimmt, so kann dann das Zentrum dieses ersten Teilbildbereiches ETB verwendet werden und ein entsprechender zweiter Teilbildbereich ZTB einer fest vorgegebenen Größe um dieses Zentrum drum herum gelegt werden, um den zweiten Teilbildbereich ZTB zu definieren. In der Figur 6 ist der zweite Teilbildbereich durch eine gestrichelte Markierung eingetragen, welches die Korrespondenz zwischen dem ersten Teilbildbereich ETB aus der Figur 5 und dem zweiten Teilbildbereich ZTB aus Figur 6 verdeutlicht. Der Teilbildbereich ZTB muss also nicht absolut identisch zu dem Teilbildbereich ETB sein, kann es aber sein.

Ein solches beispielhaftes zweites Teilbild ZTB ist in der Figur 2 dargestellt. Wie es in Figur 2 zu erkennen ist, können in einer Crithidia-luciliae Zelle bis zu drei signifikant gefärbte Bereiche aufgrund einer Bindung von Antikörpern gegeben sein, nämlich im Bereich des Kinetoplasten K, des Basalkörpers B als auch des Zellkerns Z. Daher muss sichergestellt werden, dass in dem Fluoreszenzbild SG des zweiten Farbkanals nicht irgendwelche signifikante Färbungen als Bewertungskriterium verwendet werden, sondern nur Färbungen von Kinetoplastbereichen.

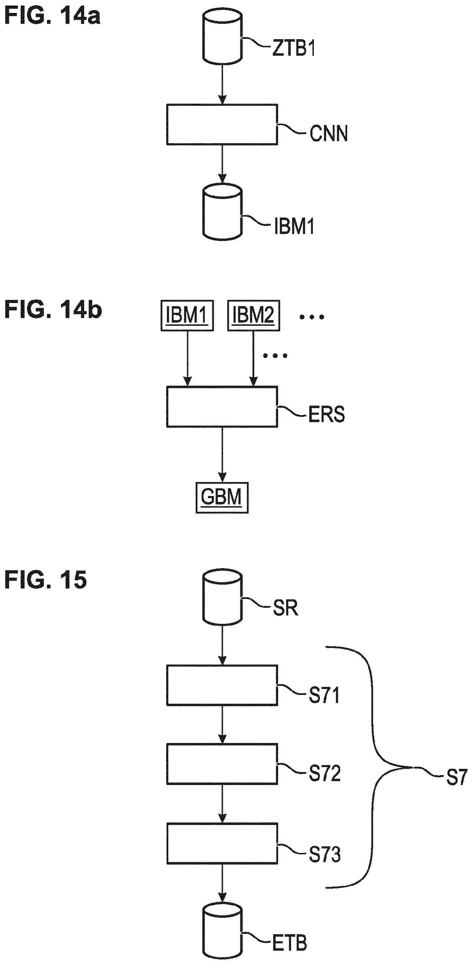

Die Figur 14a zeigt einen weiteren Schritt des erfindungsgemäßen Verfahrens, in welchem für ein bestimmtes zweites Teilbild ZTB1 mittels eines Convolutional Neural Network CNN ein zugehöriges Bindungsmaß IBM1 bestimmt wird. Das Bindungsmaß IBM1 indiziert einen Grad einer Bindung von Autoantikörpern in einem Kinetoplastbereich der in dem zweiten Teilbild dargestellten Crithidia luciliae Zelle.

Die Figur 14b illustriert einen weiteren Schritt des erfindungsgemäßen Verfahrens, in welchem auf Basis unterschiedlicher Bindungsmaße IBM1, IBM2 von unterschiedlichen zweiten Teilbildbereichen dann ein Gesamtbindungsmaß ermittelt wird, welches bezogen ist auf die zu detektierende Bindung von Autoantikörpern der Patientenprobe an doppelsträngige DNS in dem Substrat.

Das Ziel des erfindungsgemäßen Verfahrens ist also ein Bestimmen der Bindung der primären Autoantikörper an die dsDNS in den jeweiligen Kinetoplastbereich der jeweiligen Crithidia luciliae Zellen mittels Bestimmung entsprechender Färbungen der entsprechenden Kinetoplastbereiche durch den zweiten Fluoreszenzfarbstoff in den entsprechenden zweiten Teilbildbereichen.

Es ist festzustellen, dass in dem erfindungsgemäßen Verfahren nicht einfach das gesamte Fluoreszenzbild des zweiten Farbkanals SG aus der Figur 4 einem Convolutional Nuronal Network für eine gesamte Erkennung mehrerer gefärbter Kinetoplastbereiche zugeführt wird, sondern dass die Erfindung explizit von einem solchen ganzheitlichen Klassifikationsansatz für das gesamte zweite Fluoreszenzbild SG mittels eines Convolutional Neural Network abweicht. Erfindungsgemäß erfolgt nämlich zunächst eine Vorprozessierung in der Weise, dass in dem ersten Fluoreszenzbild die ersten Teilbildbereiche identifiziert werden, um dann in dem zweiten Fluoreszenzfarbstoffbild aus der Figur 6 korrespondierende zweite Teilbildbereiche auszuschneiden, wie in der Figur 2 beispielhaft für einen solchen zweiten Teilbereich ZTB illustriert. Wie es in Figur 2 zu erkennen ist, können in einer Crithidia-luciliae Zelle bis zu drei signifikant gefärbte Bereiche aufgrund einer Bindung von Antikörpern gegeben sein, nämlich im Bereich des Kinetoplasten K, des Basalkörpers B als auch des Zellkerns Z. Diese zweiten Teilbildbereiche werden dann zunächst jeweils für sich separat durch das CNN prozessiert, um für ein jeweiliges zweites Teilbild ein jeweiliges Subbild zu bestimmen, das den Kinetoplastbereich repräsentiert, und um dann insbesondere mittels des Subbildes ein jeweiliges Bindungsmaß zu bestimmen, welches einen jeweiligen Grad einer Bindung von Autoantikörpern in einem jeweiligen Kinetoplastbereich einer jeweiligen Crithidia luciliae Zelle eines jeweiligen zweiten Teilbildes indiziert. Mit anderen Worten: Es werden insbesondere diese zweiten Teilbildbereiche zunächst jeweils für sich separat durch das CNN prozessiert, um für ein jeweiliges zweites Teilbild ein jeweiliges Subbild zu identifizieren und um dann auf Basis des jeweiligen Subbildes ein jeweiliges Bindungsmaß für das jeweilige zweite Teilbild zu bestimmen. Ein solches Bindungsmaß indiziert einen jeweiligen Grad einer Bindung von Autoantikörpern in einem jeweiligen Kinetoplastbereich einer jeweiligen Crithidia luciliae Zelle eines jeweiligen zweiten Teilbildes. Auf Basis dieser jeweiligen einzelnen Bindungsmaße wird dann das Gesamtbindungsmaß ermittelt.

Das erfindungsgemäße Verfahren erzielt durch den hier beschriebenen Ansatz eine hohe Genauigkeit bei der Lokalisation der Kinetoplastbereiche beziehungsweise bei einer Detektion einer Verfärbung der Kinetoplastbereiche. Würde das gesamte Fluoreszenzfarbbild des zweiten Farbkanals SG aus der Figur 4 einem Convolutional Neural Network Neural Network zugeführt werden, um eine Verfärbung eines Kinetoplasten bzw. der verschiedenen Kinetoplastbereiche der verschiedenen Crithidia luciliae Zellen zu detektieren, so könnten hier andere Bereiche wie Basalkörperbereiche oder Zellkernbereiche fehlerhaft als jeweils ein Kinetoplastbereich identifiziert werden und eine Bestimmung einer Bindung von primären Autoantikörpern an dsDNS daher verfälscht werden. Ein gesamtes Fluoreszenzbild SG weist ferner zu einem sehr großen Anteil von ca. 90% lediglich Hintergrundbereiche auf, so dass bei einem Prozessieren eines gesamten Fluoreszenzbildes SG durch ein CNN auch die zu dem Hintergrundbereich zugehörigen Bildpixel prozessiert werden müssten, welches das CNN sehr aufwändig machen würde und eine Prozessierung ineffizient. Ferner müsste das CNN aus dem gesamten Fluoreszenzbild SG auch noch solche Unterbereiche identifizieren, welche jeweils einen Kinetoplasten repräsentieren, welches aufgrund möglicher Färbungen von Zellkernbereichen oder Basalkörperbereichen aufwändig ist. Es muss nämlich im Falle einer Färbung eines Basalkörperbereiches und/oder eines Zellkernbereiches nicht unbedingt eine Färbung eines Kinetoplastbereiches vorliegen. Auch aufgrund unterschiedlicher Lagen der Crithidia luciliae Zellen innerhalb des Substrates bzw. des Fluoreszenzfarbbildes des zweiten Farbkanal SG, wie in der Figur 4 darstellt, ergeben sich eben zu viele Freiheitsgrade für eine sichere Klassifikation einzelner Bildsegmente als Kinetoplast durch ein Convolutional Neural Network.

Es erfolgt statt eines Prozessierens eines gesamten zweiten Fluoreszenzbildes durch ein CNN erfindungsgemäß ein separates Prozessieren durch das CNN für ein jeweiliges zweites Teilbild, welches eine jeweilige Crithidia luciliae Zelle aufweist. Das zweite Teilbild wird eben in seiner Lage bezogen auf das gesamte zweite Fluoreszenzbild aufgrund einer Auswertung des ersten Fluoreszenzbildes bestimmt. Hierdurch wird es ermöglicht, dass das CNN explizit auf einzelne zweite Teilbilder trainiert werden kann, also auf jeweilige zweite Teilbilder mit einzelnen Crithidia luciliae Zellen. Das CNN muss dann nur noch in diesem zweiten Teilbild die Lage des Kinetoplasten detektieren. Diese Lage kann eben vorzugsweise als ein sogenanntes Subbild bestimmt werden. Es kann dann bezogen auf den einzelnen Kinetoplasten ein Bindungsmaß für diese Zelle bzw. diesen Kinetoplasten durch das CNN bestimmt werden.

Mit anderen Worten: Erfindungsgemäß wird durch Zuführen jeweils nur bestimmte einzelner zweiter Teilbildbereiche in das Convolutional Neural Network es ermöglicht, dass das Convolutional Neural Network lediglich auf eine Analyse eines solchen zweiten Teilbildes bzw. einer einzelnen Darstellung einer einzelnen Crithidia luciliae Zelle beschränkt wird, um den Kinetoplasten zu identifizieren und das Bindungsmaß bezogen auf die Autoantikörper zu bestimmen. Würde ein gesamtes Fluoreszenzfarbbild wie das Bild SG aus der Figur 4 einem Convolutional Neural Network für eine Klassifikationsaufgabe zur Detektion von unterschiedlichen Kinetoplasten im Zuge einer Trainingsphase zugeführt werden, so wäre dieses Convolutional Neural Network sehr groß in seinem Freiheitsgrad auszugestalten und es wäre sehr aufwendig und ineffizient, ein solches CNN zu trainieren. Dadurch, dass das erfindungsgemäße Convolutional Neural Network jeweils nur einen einzelnen zweiten Teilbildbereich für sich prozessieren muss um dann ein entsprechendes Bindungsmaß zu bestimmen, kann das Convolutional Neural Network auf eine Prozessierung eines eine Crtihidia luciliae Zelle repräsentierenden Bildes, wie in der Figur 2 dargestellt, beschränkt werden.

Vorteilhafte Ausführungsformen der Erfindung sind Gegenstand der abhängigen Ansprüche und werden in der folgenden Beschreibung unter Teilweise Bezugnahme auf die Figuren näher erläutert.

Vorzugsweise weist das Verfahren für ein jeweiliges zweites Teilbild aus der ausgewählten Teilmenge die Schritte auf: Selektieren eines jeweiligen Subbildes des jeweiligen zweiten Teilbildes auf Basis der zu dem jeweiligen zweiten Teilbild korrespondierenden jeweiligen finalen Feature Map, wobei das jeweilige Subbild einen jeweiligen Kinetoplastbereich einer jeweiligen Crithidia luciliae Zelle repräsentiert, sowie ferner Bestimmen des jeweiligen Bindungsmaßes auf Basis des jeweiligen Subbildes. Das Verfahren weist ferner vorzugsweise auf: Bestimmen des Gesamtbindungsmaßes auf Basis der jeweiligen Bindungsmaße.

Vorzugsweise weist das Verfahren für ein jeweiliges zweites Teilbild aus der ausgewählten Teilmenge ferner die Schritte auf: Ermitteln eines jeweiligen Maskierungsoperators auf Basis der jeweiligen finalen Feature Map, ferner Selektieren des jeweiligen Subbildes des jeweiligen zweiten Teilbildes mittels Anwendung des jeweiligen Maskierungsoperators auf das jeweilige zweite Teilbild, ferner Bestimmen des jeweiligen Bindungsmaßes auf Basis des jeweiligen Subbildes. Das Verfahren weist ferner vorzugsweise auf: Bestimmen des Gesamtbindungsmaßes auf Basis der jeweiligen Bindungsmaße.

Vorzugsweise ist das Verfahren so ausgestaltet, dass das Convolutional Neural Network im Zuge einer Prozessierung eines zweiten Teilbildes in einer ersten Prozessierungsebene auf Basis des zweiten Teilbildes mittels eines ersten Convolutional Layer eine erste Menge von resultierenden Feature Maps generiert, ferner in einer zweiten Prozessierungsebene auf Basis der ersten Menge zweidimensionaler Feature Maps mittels eines zweiten Convolutional Layer eine zweite Menge von resultierenden Feature Maps generiert sowie ferner auf Basis der zweiten Menge zweidimensionaler Feature Maps mittels eines dritten Convolutional Layer eine dritte Menge von resultierenden Feature Maps generiert, wobei die zweite Menge eine geringere Anzahl resultierender Feature Maps aufweist als die erste Menge und wobei die dritte Menge eine größere Anzahl resultierender Feature Maps aufweist als die zweite Menge.

Vorzugsweise ist das Verfahren so ausgestaltet, dass das zweite Convolutional Layer und das dritte Convolutional Layer als Teilschritte eines sequentiellen Prozessierungspfades aufeinander abfolgen, wobei in der zweiten Prozessierungsebene ein weiterer Prozessierungspfad parallel zu dem sequentiellen Prozessierungspfad vorhanden ist, in welchem das Convolutional Neural Network auf Basis der ersten Menge zweidimensionaler Feature Maps mittels wenigstens eines vierten Convolutional Layer eine vierte Menge von resultierenden Feature Maps generiert, wobei ferner das Convolutional Neural Network die zu dem zweiten Teilbild korrespondierende finale Feature Map auf Basis der dritten und der vierten Menge von resultierenden Feature Maps generiert und wobei ferner die Anzahl aufeinanderfolgender Convolution Layer in dem parallelen Prozessierungspfad geringer ist als die Anzahl aufeinanderfolgender Convolution Layer in dem sequentiellen Prozessierungspfad.

Vorzugsweise weist das Verfahren die Schritte auf: Erfassen eines ersten vorläufigen Fluoreszenzbildes in dem ersten Farbkanal unter Verwendung eines fest vorgegebenen Erfassungsparameters, Bestimmen eines Helligkeitswertes, welcher eine Helligkeit des ersten vorläufigen Fluoreszenzbildes des ersten Farbkanals indiziert, Modifizieren des Erfassungsparameters in Abhängigkeit des bestimmten Helligkeitswertes, Erfassen eines zweiten vorläufigen Fluoreszenzbildes in dem ersten Farbkanal unter Verwendung des modifizierten Erfassungsparameters sowie ferner Verwenden des zweiten vorläufigen Fluoreszenzbildes des ersten Farbkanals als das erste Fluoreszenzbild des ersten Farbkanals.

Vorzugsweise weist das Verfahren die Schritte auf: Erfassen eines ersten vorläufigen Fluoreszenzbildes in dem ersten Farbkanal unter Verwendung eines fest vorgegebenen Erfassungsparameters, Bestimmen eines Helligkeitswerte, welcher eine Helligkeit des ersten vorläufigen Fluoreszenzbildes des ersten Farbkanals indiziert, Feststellen mittels des Helligkeitswertes, ob eine Helligkeit des ersten vorläufigen Fluoreszenzbildes des ersten Farbkanals einer erwarteten Helligkeit entspricht, in dem Fall, dass die Helligkeit des ersten vorläufigen Fluoreszenzbildes des ersten Farbkanals der erwarteten Helligkeit entspricht, Verwenden des ersten vorläufigen Fluoreszenzbildes des ersten Farbkanals als das erste Fluoreszenzbild des ersten Farbkanals, in dem Fall, dass die Helligkeit des ersten vorläufigen Fluoreszenzbildes nicht der erwarteten Helligkeit entspricht, Modifizieren des Erfassungsparameters in Abhängigkeit des bestimmten Helligkeitswertes, Erfassen eines zweiten vorläufigen Fluoreszenzbildes in dem ersten Farbkanal unter Verwendung des modifizierten Erfassungsparameters, Verwenden des zweiten vorläufigen Fluoreszenzbildes des ersten Farbkanals als das erste Fluoreszenzbild des ersten Farbkanals.

Vorzugsweise weist das Verfahren die Schritte auf: Erfassen eines ersten vorläufigen Fluoreszenzbildes in dem zweiten Farbkanal unter Verwendung eines fest vorgegebenen Erfassungsparameters, Feststellen, ob eine Helligkeit des ersten vorläufigen Fluoreszenzbildes des zweiten Farbkanals eine maximale Helligkeit übersteigt, in dem Fall, dass das erste vorläufige Fluoreszenzbild des zweiten Farbkanals die maximale Helligkeit nicht übersteigt, Verwenden des ersten vorläufigen Fluoreszenzbildes als das zweite Fluoreszenzbild des zweiten Farbkanals, in dem Fall, dass das das erste vorläufige Fluoreszenzbild des zweiten Farbkanals die maximale Helligkeit übersteigt, Erfassen eines zweiten vorläufigen Fluoreszenzbildes in dem zweiten Farbkanal und Verwenden des zweiten vorläufigen Fluoreszenzbildes des zweiten Farbkanals als das zweite Fluoreszenzbild des zweiten Farbkanals.

Vorgeschlagen wird ferner eine erfindungsgemäße Vorrichtung zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung, aufweisend eine Haltevorrichtung für ein Substrat, welches mehrere Crithidia luciliae Zellen aufweist und welches mit einer Patientenprobe aufweisend die Autoantikörper, mit einem ersten Fluoreszenzfarbstoff sowie ferner mit sekundären Antikörpern, welche jeweils mit einem zweiten Fluoreszenzfarbstoff markiert sind, inkubiert wurde, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wird. Die Vorrichtung weist ferner wenigstens eine Bilderfassungseinheit zum Erfassen eines ersten Fluoreszenzbildes des Substrates in einem ersten Farbkanal sowie ferner zum Erfassen eines zweiten Fluoreszenzbildes des Substrates in einem zweiten Farbkanal auf. Die Vorrichtung weist ferner wenigstens eine Recheneinheit auf, welche ausgebildet ist, jeweilige erste Teilbilder in dem ersten Fluoreszenzbild zu identifizieren, welche jeweils wenigstens eine Crithidia Luciliae Zelle repräsentieren, ferner jeweilige zweite Teilbilder des zweiten Fluoreszenzbildes zu bestimmen, welche zu den jeweiligen ersten Teilbildern des ersten Fluoreszenzbildes korrespondieren, ferner wenigstens eine Teilmenge der jeweiligen zweiten Teilbilder jeweils zu prozessieren mittels eines vortrainierten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen zweiten Teilbildes indizieren. Das Convolutional Neural Network prozessiert ein jeweiliges zweites Teilbild für sich jeweils separat, um für ein jeweiliges zweites Teilbild ein jeweiliges Subbild zu bestimmen, das einen jeweiligen Kinetoplastbereich einer jeweiligen Crithidia luciliae Zelle repräsentiert. Das CNN bestimmte das jeweilige Bindungsmaß auf Basis des jeweiligen Subbildes. Das CNN generiert im Zuge der Bestimmung der jeweiligen individuellen Bindungsmaße für ein jeweiliges zweites Teilbild eine jeweilige finale Feature-Map. Es wird ferner auf Basis der finalen Feature-Map ein jeweiliges positives Konfidenzmaß, bezogen auf ein Vorliegen der Bindung von Autoantikörpern in einem jeweiligen Kinetoplastbereich für ein jeweiliges zweites Teilbild, bestimmt. Ferner wird eine Teilmenge der zweiten Teilbilder ausgewählt auf Basis der jeweiligen positiven Konfidenzmaße der jeweiligen zweiten Teilbilder. Es wird ferner ein Gesamtbindungsmaß einer Bindung von Autoantikörpern der Patientenprobe an doppelsträngige Desoxyribonukleinsäure auf Basis der jeweiligen Bindungsmaße bestimmt.

Vorgeschlagen wird ferner eine Recheneinheit, welche ausgebildet ist, im Zuge einer digitalen Bildverarbeitung ein erstes Fluoreszenzbild entgegenzunehmen, welches eine Färbung eines Substrates, welches wiederum mehrere Crithidia luciliae Zellen aufweist, durch einen ersten Fluoreszenzfarbstoff repräsentiert, sowie ein zweites Fluoreszenzbild entgegenzunehmen, welches eine Färbung des Substrates durch einen zweiten Fluoreszenzfarbstoff repräsentiert, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wird, ferner jeweilige erste Teilbilder in dem ersten Fluoreszenzbild zu identifizieren, welche jeweils wenigstens eine Crithidia Luciliae Zelle repräsentieren, ferner jeweilige zweite Teilbilder des zweiten Fluoreszenzbildes zu bestimmen, welche zu den jeweiligen ersten Teilbildern des ersten Fluoreszenzbildes korrespondieren, ferner wenigstens eine Teilmenge der jeweiligen zweiten Teilbilder jeweils zu prozessieren mittels eines vortrainierten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen zweiten Teilbildes indizieren. Das Convolutional Neural Network prozessiert ein jeweiliges zweites Teilbild für sich jeweils separat, um für ein jeweiliges zweites Teilbild ein jeweiliges Subbild zu bestimmen, das einen jeweiligen Kinetoplastbereich einer jeweiligen Crithidia luciliae Zelle repräsentiert. Das CNN bestimmte das jeweilige Bindungsmaß auf Basis des jeweiligen Subbildes. Das CNN generiert im Zuge der Bestimmung der jeweiligen individuellen Bindungsmaße für ein jeweiliges zweites Teilbild eine jeweilige finale Feature-Map. Es wird ferner auf Basis der finalen Feature-Map ein jeweiliges positives Konfidenzmaß, bezogen auf ein Vorliegen der Bindung von Autoantikörpern in einem jeweiligen Kinetoplastbereich für ein jeweiliges zweites Teilbild, bestimmt. Ferner wird eine Teilmenge der zweiten Teilbilder ausgewählt auf Basis der jeweiligen positiven Konfidenzmaße der jeweiligen zweiten Teilbilder. Es wird ferner ein Gesamtbindungsmaß einer Bindung von Autoantikörpern der Patientenprobe an doppelsträngige Desoxyribonukleinsäure auf Basis der jeweiligen Bindungsmaße bestimmt.

Vorgeschlagen wird ferner eine Datennetzwerkvorrichtung aufweisend wenigstens eine Datenschnittstelle zum Entgegennehmen eines ersten Fluoreszenzbildes, welches eine Färbung eines Substrates, welches wiederum mehrere Crithidia luciliae Zellen aufweist, durch einen ersten Fluoreszenzfarbstoff repräsentiert sowie ferner zum Entgegennehmen eines zweiten Fluoreszenzbildes, welches eine Färbung des Substrates durch einen zweiten Fluoreszenzfarbstoff repräsentiert, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wird. Die Datennetzwerkvorrichtung weist ferner eine Recheneinheit auf, welche ausgebildet ist, im Zuge einer digitalen Bildverarbeitung jeweilige erste Teilbilder in dem ersten Fluoreszenzbild zu identifizieren, welche jeweils wenigstens eine Crithidia Luciliae Zelle repräsentieren, ferner jeweilige zweite Teilbilder des zweiten Fluoreszenzbildes zu bestimmen, welche zu den jeweiligen ersten Teilbildern des ersten Fluoreszenzbildes korrespondieren, ferner wenigstens eine Teilmenge der jeweiligen zweiten Teilbilder jeweils zu prozessieren mittels eines vortrainierten Convolutional Neural Network zur Bestimmung jeweiliger Bindungsma-ße, welche einen jeweiligen Grad einer Bindung von Autoantikörpern in einem jeweiligen Kinetoplastbereich einer jeweiligen Crithidia luciliae Zelle eines jeweiligen zweiten Teilbildes indizieren. Das Convolutional Neural Network prozessiert ein jeweiliges zweites Teilbild für sich jeweils separat, um für ein jeweiliges zweites Teilbild ein jeweiliges Subbild zu bestimmen, das einen jeweiligen Kinetoplastbereich einer jeweiligen Crithidia luciliae Zelle repräsentiert. Das CNN bestimmte das jeweilige Bindungsmaß auf Basis des jeweiligen Subbildes. Das CNN generiert im Zuge der Bestimmung der jeweiligen individuellen Bindungsmaße für ein jeweiliges zweites Teilbild eine jeweilige finale Feature-Map. Es wird ferner auf Basis der finalen Feature-Map ein jeweiliges positives Konfidenzmaß, bezogen auf ein Vorliegen der Bindung von Autoantikörpern in einem jeweiligen Kinetoplastbereich für ein jeweiliges zweites Teilbild, bestimmt. Ferner wird eine Teilmenge der zweiten Teilbilder ausgewählt auf Basis der jeweiligen positiven Konfidenzmaße der jeweiligen zweiten Teilbilder. Es wird ferner ein Gesamtbindungsmaß einer Bindung von Autoantikörpern der Patientenprobe an doppelsträngige Desoxyribonukleinsäure auf Basis der jeweiligen Bindungsmaße bestimmt.

Vorgeschlagen wird ferner ein Verfahren zur digitalen Bildverarbeitung aufweisend die Schritte: Entgegennehmen eines ersten Fluoreszenzbildes, welches eine Färbung eines Substrates, welches wiederum mehrere Crithidia luciliae Zellen aufweist, durch einen ersten Fluoreszenzfarbstoff repräsentiert sowie ferner eines zweiten Fluoreszenzbildes, welches eine Färbung des Substrates durch einen zweiten Fluoreszenzfarbstoff repräsentiert, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wird, ferner Identifizieren jeweiliger erster Teilbilder in dem ersten Fluoreszenzbild, welche jeweils wenigstens eine Crithidia Luciliae Zelle repräsentieren, ferner Bestimmen jeweiliger zweiter Teilbilder des zweiten Fluoreszenzbildes, welche zu den jeweiligen ersten Teilbildern des ersten Fluoreszenzbildes korrespondieren, ferner jeweiliges Prozessieren wenigstens einer Teilmenge der jeweiligen zweiten Teilbilder mittels eines vortrainierten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliae Zelle eines jeweiligen zweiten Teilbildes indizieren. Das Convolutional Neural Network prozessiert ein jeweiliges zweites Teilbild für sich jeweils separat, um für ein jeweiliges zweites Teilbild ein jeweiliges Subbild zu bestimmen, das einen jeweiligen Kinetoplastbereich einer jeweiligen Crithidia luciliae Zelle repräsentiert. Das CNN bestimmte das jeweilige Bindungsmaß auf Basis des jeweiligen Subbildes. Das CNN generiert im Zuge der Bestimmung der jeweiligen individuellen Bindungsmaße für ein jeweiliges zweites Teilbild eine jeweilige finale Feature-Map. Es wird ferner auf Basis der finalen Feature-Map ein jeweiliges positives Konfidenzmaß, bezogen auf ein Vorliegen der Bindung von Autoantikörpern in einem jeweiligen Kinetoplastbereich für ein jeweiliges zweites Teilbild, bestimmt. Ferner wird eine Teilmenge der zweiten Teilbilder ausgewählt auf Basis der jeweiligen positiven Konfidenzmaße der jeweiligen zweiten Teilbilder. Es wird ferner Bestimmen eines Gesamtbindungsmaßes einer Bindung von Autoantikörpern der Patientenprobe an doppelsträngige Desoxyribonukleinsäure auf Basis der jeweiligen Bindungsmaße durchgeführt.

Vorgeschlagen wird ferner ein Computerprogrammprodukt, umfassend Befehle, die bei der Ausführung des Programms durch einen Computer diesen veranlassen, das erfindungsgemäße Verfahren zur digitalen Bildverarbeitung durchzuführen

Vorgeschlagen wird ferner ein Datenträgersignal, welches das vorgeschlagene Computerprogrammprodukt überträgt.

Im Folgenden wird die Erfindung anhand spezieller Ausführungsform ohne Beschränkung des allgemeinen Erfindungsgedankens anhand der Figuren näher erläutert. Dabei zeigen:
Figur 1 ein Fluoreszenzbild eines Substrates aufweisend mehrere Crithidia luciliae Zellen
Figur 2 ein Teilbild des Fluoreszenzbildes aus der Figur 1 mit einer Crithidia luciliae Zelle
Figur 3 ein Fluoreszenzbild des Substrates in einem ersten Farbkanal,
Figur 4 ein Fluoreszenzbild des Substrates in einem zweiten Farbkanal,
Figur 5 das Fluoreszenzbild des ersten Farbkanals mit identifizierten jeweiligen ersten Teilbildbereichen,
Figur 6 das Fluoreszenzbild des zweiten Farbkanals mit bestimmten bzw. identifizierten jeweiligen zweiten Teilbildbereichen, welche zu den jeweiligen ersten Teilbildbereichen des ersten Fluoreszenzbildes korrespondieren,
Figur 7 ein Ausführungsbeispiel der erfindungsgemäßen Vorrichtung,
Figur 8 eine modifizierte Fassung des Fluoreszenzbildes des erstens Farbkanal mit Kontrasterhöhung,
Figur 9 ein binärwertiges Bild abgeleitet aus dem kontrasterhöhten Fluoreszenzbild der Figur 8,
Figur 10a ein beispielhaftes zweites Teilbild,
Figur 10b eine erste finale Feature Map,
Figur 10c eine vorzugsweise zu bestimmende zweite finale Feature Map,
Figur 11a eine interpolierte Version der ersten finalen Feature Map aus der Figur 10b,
Figur 11b einen binärwertigen Maskierungsoperator abgeleitet aus der interpolierten Feature Map der Figur 11a,
Figur 11c einen selektierten Subbildbereich eines Subbildes aus dem zweiten Teilbildbereich der Figur 10a,
Figur 12a noch einmal das zweite Teilbild,
Figur 12b eine Darstellung eines indizierten Bildbereiches in dem zweiten Teilbildbereich, welcher zu dem Subbild aus der Figur 11c korrespondiert,
Figur 13 Schritte zur Durchführung des erfindungsgemäßen Verfahrens
Figur 14a ein Grundprinzip zur Ermittlung eines Bindungsmaßes einer Bindung primärer Autoantikörper der Patientenprobe an ein Kinetoplastbereich für einen zweiten Teilbildbereich,
Figur 14b eine Prozessierung mehrerer Bindungsmaße zur Bestimmung eines Gesamtbindungsmaßes,
Figur 15 Teilschritte zur Bestimmung erster Teilbildbereiche in dem ersten Fluoreszenzfarbbild des ersten Farbkanals,
Figur 16a eine beispielhafte Illustration einer bevorzugten Ausführungsform eines Convolutional Neural Network,
Figur 16b Teilschritte einer ersten Art eines Convolutional Layer des Convolutional Neural Network,
Figur 16c Teilschritte einer weiteren Art eines Convolutional Layer des Convolutional Neural Network,
Figur 17 Prozessierungsschritte zur Bestimmung eines Konfidenzmaßes bezogen auf ein Vorliegen einer Bindung von Autoantikörpern an einen Kinetoplastbereich für ein entsprechendes zweites Teilbild auf Basis der zugehörigen Feature Maps,
Figur 18 Schritte zum Auswählen einer Teilmenge der zweiten Teilbilder auf Basis der Konfidenzmaße der jeweiligen zweiten Teilbilder,
Figur 19 Schritte für ein Selektieren eines jeweiligen Subbildes eines jeweiligen zweiten Teilbildes sowie für ein Bestimmen eines jeweiligen Bindungsmaßes auf Basis des jeweiligen Subbildes mittels Anwenden eines binärwertigen Maskierungsoperators auf das jeweilige zweite Teilbild,
Figur 20 eine beispielhafte Darstellung einer erfindungsgemäßen Recheneinheit,
Figur 21 eine beispielhafte Darstellung einer erfindungsgemäßen Datennetzwerkvorrichtung,
Figur 22 eine beispielhafte Darstellung eines erfindungsgemäßen Computerprogrammproduktes als auch eines erfindungsgemäßen Datenträgersignals,
Figur 23 ein Ausführungsbeispiel einer ersten Prozessierungsebene des CNN,
Figur 24 ein Ausführungsbeispiel eines ersten Teils einer zweiten Prozessierungsebene des CNN,
Figur 25 ein Ausführungsbeispiel eines zweiten Teils einer zweiten Prozessierungsebene des CNN,
Figur 26 ein Ausführungsbeispiel einer dritten Prozessierungsebene des CNN,
Figur 27 Pixelwerte eines Subbildes,
Figur 28 jeweilige Konfidenzmaßwerte für jeweilige zweite Teilbilder eines zweiten Fluoreszenzbildes,
Figur 29 Schritte eines Verfahrens für eine Erfassung eines ersten Fluoreszenzbildes gemäß einer bevorzugten ersten Ausführungsform,
Figur 30 Schritte eines Verfahrens für eine Erfassung eines ersten Fluoreszenzbildes gemäß einer bevorzugten zweiten Ausführungsform,
sowie Figur 31 Schritte eines Verfahrens für eine Erfassung eines zweiten Fluoreszenzbildes gemäß einer bevorzugten Ausführungsform.

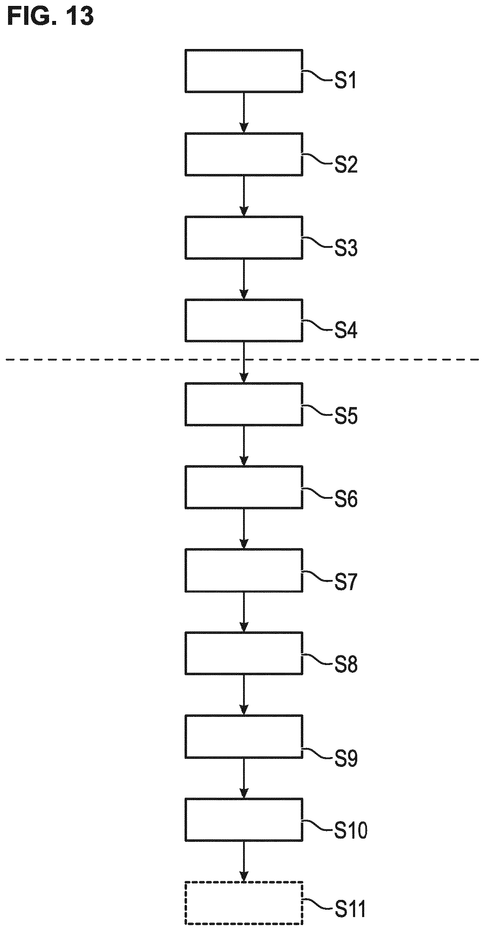

Figur 13 illustriert unterschiedliche Schritte zur Durchführung des erfindungsgemäßen Verfahrens. In einem Schritt S1 wird ein Substrat bereitgestellt, welches mehrere Crithidia luciliae Zellen aufweist. In einem Schritt S2 wird das Substrat mit der Patientenprobe inkubiert. Die Patientenprobe weist potenziell die Autoantikörper auf. In einem Schritt S3 wird das Substrat mit einem ersten Fluoreszenzfarbstoff inkubiert. In einem Schritt S4 wird das Substrat mit sekundären Antikörpern, welche jeweils mit einem zweiten Fluoreszenzfarbstoff markiert sind, inkubiert. In einem Schritt S5 erfolgt ein Erfassen eines ersten Fluoreszenzbildes des Substrates in einem ersten Farbkanal, welcher zu dem ersten Fluoreszenzfarbstoff korrespondiert. In einem Schritt S6 erfolgt ein Erfassen eines zweiten Fluoreszenzbildes des Substrates in einem zweiten Kanal, welcher zu dem zweiten Fluoreszenzfarbstoff korrespondiert. In einem Schritt S6 erfolgt ein Identifizieren jeweiliger erster Teilbilder in dem ersten Fluoreszenzbild, welche jeweils eine Crithidia luciliae Zelle aufweisen. Ein derartiges beispielhaftes erstes Teilbild ETB ist in der Figur 5 dargestellt. In einem darauffolgenden Schritt S8 erfolgt ein Bestimmen jeweiliger zweiter Teilbilder des zweiten Fluoreszenzbildes, welche zu den jeweiligen ersten Teilbildern des ersten Fluoreszenzbildes korrespondieren. Ein solches beispielhaftes zweites Teilbild ZTB ist in der Figur 6 dargestellt, welches zu dem ersten Teilbild ETB aus der Figur 5 korrespondiert. In einem Schritt S9 erfolgt ein jeweiliges Prozessieren wenigstens einer Teilmenge der jeweiligen zweiten Teilbildern mittels eines vortrainierten Convolutional Neural Network zur Bestimmung jeweiliger Bindungsmaße, welche einen jeweiligen Grad einer Bindung von Autoantikörpern in einem jeweiligen Kinetoplastbereich einer jeweiligen Crithidia luciliaeZelle eines jeweiligen zweiten Teilbildes indizieren. In einem Schritt S10 erfolgt ein Bestimmen eines Gesamtbindungsmaßes bezogen auf die Bindung von Autoantikörpern der Patientenprobe an doppelsträngige DNS in dem Substrat auf Basis der jeweiligen Bindungsmaße der jeweiligen zweiten Teilbilder. In einem Schritt S11 erfolgt vorzugsweise ein Bereitstellen des Gesamtbindungsmaßes, alternativ oder zusätzlich erfolgt ein Ausgeben und/oder Anzeigen des Gesamtbindungsmaßes.

Die Figur 14a zeigt ein beispielhaftes, insbesondere partielles, Prozessieren eines bestimmten zweiten Teilbildbereiches ZTB1 durch ein Convolutional Neural Network zur Bestimmung eines jeweiligen, individuellen Bindungsmaßes IBM1. Das Bindungsmaß IBM1 indiziert einen individuellen Grad einer Bindung von Autoantikörpern in einem individuellen Kinetoplastbereich einer individuellen Crithidia luciliae Zelle des individuellen zweiten Teilbildbereichs ZTB1.

Die Figur 14b illustriert ein Bestimmen des Gesamtbindungsmaßes GBM durch einen Ermittlungsschritt ERS, welcher dem Schritt S10 aus der Figur 13 entspricht, um das Gesamtbindungsmaß GBM auf Basis jeweiliger Bindungsmaße IBM1, IBM2 jeweiliger zweiter Teilbilder zu bestimmen.

Die Figur 7 illustriert ein Ausführungsbeispiel der erfindungsgemäßen Vorrichtung V1. Die Vorrichtung V1 weist eine Haltevorrichtung H für das Substrat S auf. Anregungslicht AL einer Anregungslichtquelle LQ wird über ein optisches Filter F1 vorgefiltert und dann mittels eines dichroitischen Spiegels SP1 durch eine Optik O hin zum Substrat geleitet. Entstehende Fluoreszenzstrahlung bzw. entstehendes Fluoreszenzlicht FL tritt dann vom Substrat her zurück durch das Objektiv O durch den dichroitischer Spiegels SP1 und durch ein Abschlussfilter F2 hindurch. Das optische Filter F2 filtert eine Wellenlänge der Anregungsstrahlung bzw. des Anregungslichtes AL heraus. Das Fluoreszenzlicht FL wird dann wenigstens einer Bilderfassungseinheit in Form einer Kamera K1 sowie vorzugsweise einer weiteren Kamera K2 zugeführt. Über einen dichroitischen Spiegel SP2 wird das Fluoreszenzlicht FL aufgeteilt. Über einen optischen Filter FR wird Fluoreszenzlicht FR1 eines ersten Farbkanals, vorzugsweise eines roten Farbkanals, herausgefiltert und der Bilderfassungseinheit K1 zugeführt. Die Bildererfassungseinheit K1 erfasst ein erstes Fluoreszenzbild des Substrates S in dem ersten Farbkanal. Über einen optischen Filter FG wird Fluoreszenzstrahlung FL2 des zweiten Farbkanals, vorzugsweise des grünen Farbkanals, herausgefiltert und der Bilderfassungseinheit K2 zugeführt, welche ein zweites Fluoreszenzbild des Substrates S im zweiten Farbkanal erfasst. Über eine Datenschnittstelle DS1 kann die Vorrichtung V1 Ergebnisdaten ED bereitstellen, welche das Gesamtbindungsmaß indizieren.

Eine Recheneinheit R ist ausgebildet, das erste Fluoreszenzbild in Form digitaler Daten BI1 entgegenzunehmen. Ferner ist die Recheneinheit R ausgebildet, das zweite Fluoreszenzbild in Form digitaler Daten BI2 entgegenzunehmen. Die Recheneinheit R ist ferner ausgebildet, die Schritte S7 bis S10 des erfindungsgemäßen Verfahrens durchzuführen.

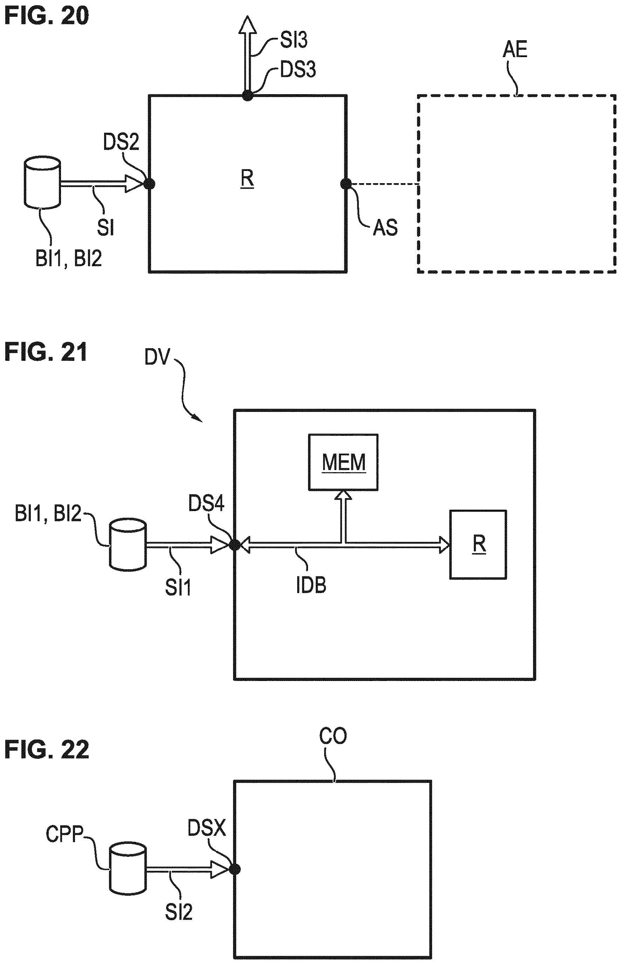

Eine erfindungsgemäße Recheneinheit R kann auch wie in der Figur 20 dargestellt realisiert sein. Hierbei nimmt die Recheneinheit R das erste Fluoreszenzbild BI1 und das zweite Fluoreszenzbild BI2 über wenigstens eine Datenschnittstelle DS2 in Form wenigstens eines Datensignals SI entgegen. Nach Durchführung der entsprechenden Schritte S7 bis S10 des erfindungsgemäßen Verfahrens aus der Figur 13 hat die Recheneinheit R das Gesamtbindungsmaß der Bindung von Autoantikörpern der Patientenprobe an doppelsträngige DNS bestimmt. Vorzugsweise weist die Recheneinheit R eine Ausgabeschnittstelle AS zu einer Anzeigeeinheit AE auf, über welche das Gesamtbindungsmaß ausgegeben bzw. angezeigt werden kann. Vorzugsweise weist die Recheneinheit R eine weitere Datenschnittstelle DS3 hin zu einem Datennetzwerk auf, über welche die Recheneinheit das Gesamtbindungsma-ßes über ein Datensignal SI3 bereitstellt. Die Datenschnittstellen DS2, DS3 der Recheneinheit R können auch eine gemeinsame Datenschnittstelle sein. Die Datenschnittstellen DS2, DS3 sind vorzugsweise Netzwerkdatenschnittstellen.

Die Recheneinheit R kann auch Teil einer erfindungsgemäßen Datennetzwerkvorrichtung DV sein, wie in der Figur 21 illustriert. Die Datennetzwerkvorrichtung DV weist wenigstens eine Datenschnittstelle DS4 zum Entgegennehmen des ersten Fluoreszenzbildes BI1 und des zweiten Fluoreszenzbildes BI2 mittels wenigstens eines Datensignals SI1 auf. Die Datennetzwerkvorrichtung DV weist die Recheneinheit R auf, welche vorzugsweise über einen internen Datenbus IDB mit einer Speichereinheit MEM und der Datenschnittstelle DS4 verbunden ist. Die Recheneinheit R ist in jener Weise ausgebildet, wie zuvor in Bezug auf die Figur 20 beschrieben. Die Datennetzwerkvorrichtung DV kann ein einzelner Computer sein oder aber eine sogenannte Cloud-Lösung. Die Datennetzwerkvorrichtung DV führt also mittels der Recheneinheit R ein erfindungsgemäßes Verfahren zur digitalen Bildverarbeitung durch, bei welchem die Fluoreszenzbilder BI1, BI2 entgegengenommen werden und bei welchem die Recheneinheit R die Schritte S7 bis S10 aus der Figur 13 durchführt.

Die Figur 22 illustriert ein erfindungsgemäßes Computerprogrammprodukt CPP, welches Befehle umfasst, die bei der Ausführung des Programms durch einen Computer CO diesen veranlassen, ein erfindungsgemäßes Verfahren der digitalen Bildverarbeitung durchzuführen.

Das Computerprogrammprodukt CPP kann in Form eines Datenträgersignals SI2 bereitgestellt werden und durch einen Computer CO mittels einer an dem Computer CO befindlichen Datenschnittstelle DSX entgegengenommen werden. Das Datenträgersignal SI2 überträgt also das Computerprogrammprodukt CPP.

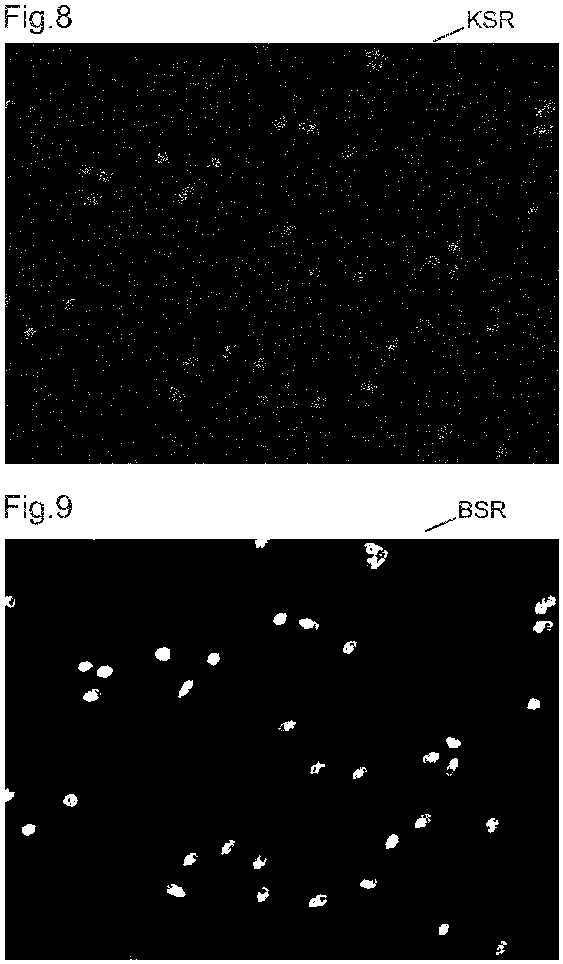

Die Figur 15 illustriert Teilschritte S71, S72, S73 als Unterschritte des Schrittes S7 zur Identifikation erster Teilbildbereiche ETB, welche in der Figur 5 dargestellt sind. Das Fluoreszenzbild SR aus der Figur 3 wird in einem Teilschritt S71 mittels des sogenannten CLAHE-Verfahrens (Contrast Limited Adaptive Histogram Equalization) in seinem Kontrast modifiziert, sodass sich das modifizierte Fluoreszenzbild KSR aus der Figur 8 ergibt. Mittels des Schwellenwertverfahrens nach OTSU wird dann in dem Schritt S72 auf Basis des Bildes KSR aus der Figur 8 ein Binärbild BSR aus der Figur 9 gewonnen. Dieses Binärbild aus der Figur 9 wird dann mittels des Verfahrens "Satoshi Suzuki and others. Topological structural analysis of digitized binary images by border following. Computer Vision, Graphics, and Image Processing, 30(1):32-46, 1985" nachbearbeitet, um relevante Konturen in dem Bild BSR aufzufinden. Ferner werden dann in dem Schritt S73 sogenannte bounding boxes mittels der Funktion *boundingRect* aus der Sektion "Structural analysis and shape descriptors" aus der Datenbank OpenCV (https://opencv.org) nachbearbeitet. Die bounding boxes haben hierbei eine Größe von 150×150 Pixel. Für das CLAHE-Verfahren können Rechteckbereiche der Größe 64×64 Pixel verwendet werden. Hierdurch ergeben sich dann bounding boxes als erste Teilbildbereiche ETB, wie in der Figur 5 dargestellt.

Vorzugsweise kann das Fluoreszenzbild SR aus der Figur 3 in seiner Qualität bewertet werden, indem nach Ermittlung der Konturen in dem Bild BSR aus der Figur 9 wenigstens zehn identifizierte Zellbereiche bzw. zweite Teilbilder eine bestimmte Morphologie als auch eine gewisse Mindestgröße aufweisen müssen. Nur in dem Fall, dass wenigstens zehn Zellen diese Kriterien beiden erfüllen, erfolgt dann eine weitere Prozessierung der Fluoreszenzbilder ohne Fehlerausgabe in dem Verfahren. Erfüllen weniger als zehn identifizierte Zellbereiche bzw. zweite Teilbilder die beiden Kriterien nicht, so wird das Verfahren vorzugsweise abgebrochen und eine Fehlermeldung ausgegeben.

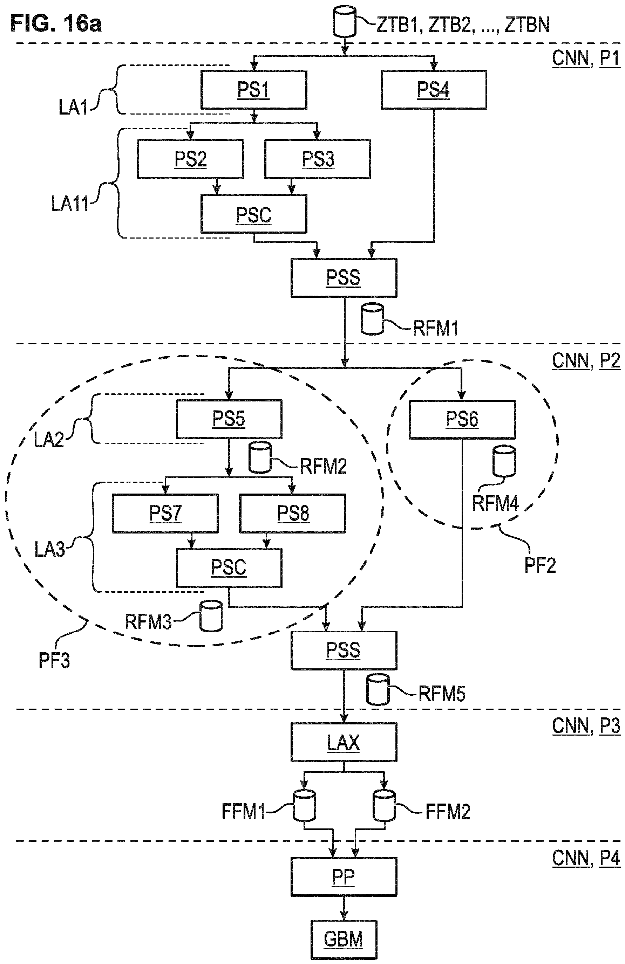

Die Figur 16a illustriert unterschiedliche Prozessierungsebenen P1, P2, P3, P4 des Convolutional Neural Network CNN zur Bestimmung des Gesamtbindungsmaßes GBM auf Basis mehrerer zweiter Teilbildbereiche ZTB1, ..., ZTBN wie zuvor ausgeführt. Das CNN prozessiert hierbei jeweilige zweite Teilbildbereiche ZTB1, ZTB2, ..., ZTBN für sich, um dann für einen jeweiligen zweiten Teilbildbereich ZTB1, ZTB2, ..., ZTBN ein jeweiliges individuelles Bindungsmaß IBM1, IBM2, ..., IBMN zu generieren, wie zuvor unter Bezug auf die Figur 14a verdeutlicht.

Die Figur 16a zeigt hierbei, dass im Zuge dieser Bestimmung der jeweiligen individuellen Bindungsmaße für jedes einzelne zweite Teilbild ZTB1, ... dann eine jeweilige finale Feature Map FFM1 als auch vorzugsweise eine weitere finale Feature Map FFM2 generiert wird. Das CNN kann so ausgestaltet werden, dass lediglich eine einzige finale Feature Map FFM1 eines einzigen Kanals vorgesehen ist und generiert wird.

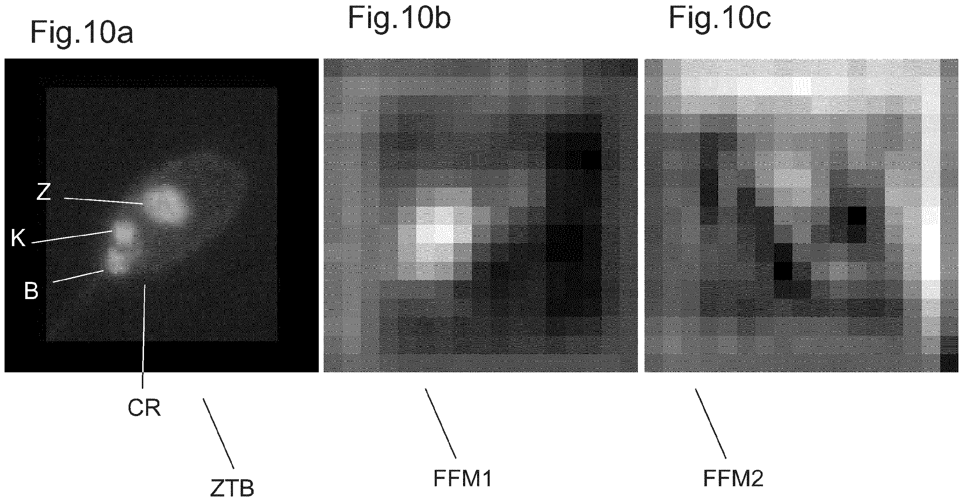

Das Ergebnis der Prozessierungsebene P3 aus der Figur 16a sind also für ein zweites Teilbild ZTB die finale Feature Map FFM1 als auch vorzugsweise die finale Feature Map FFM2. Die erste finale Feature Map FFM1 ist für das zweite Teilbild ZTB aus der Figur 10a in der Figur 10b dargestellt. Die zweite finale Feature Map FFM2 für das zweite Teilbild ZTB ist in der Figur 10c dargestellt.

Das CNN löst die Aufgabe einer sogenannten "Single-Label Classification", also ob der Kinetoplast in dem zweiten Teilbild eine Färbung aufweist oder nicht. Die finale Feature Map FFM1 repräsentiert eine Aktivierung in einem ersten Klassifikationskanal bezogen auf eine positive Entscheidung der "Single Label Classification", also dass der Kinetoplastbereich gefärbt ist. Die vorzugsweise vorzusehende finale Feature Map FFM2 repräsentiert die dazu korrespondierende Aktivierung bezogen auf eine negative Entscheidung, also dass der Kinetoplast nicht wesentlich gefärbt ist.

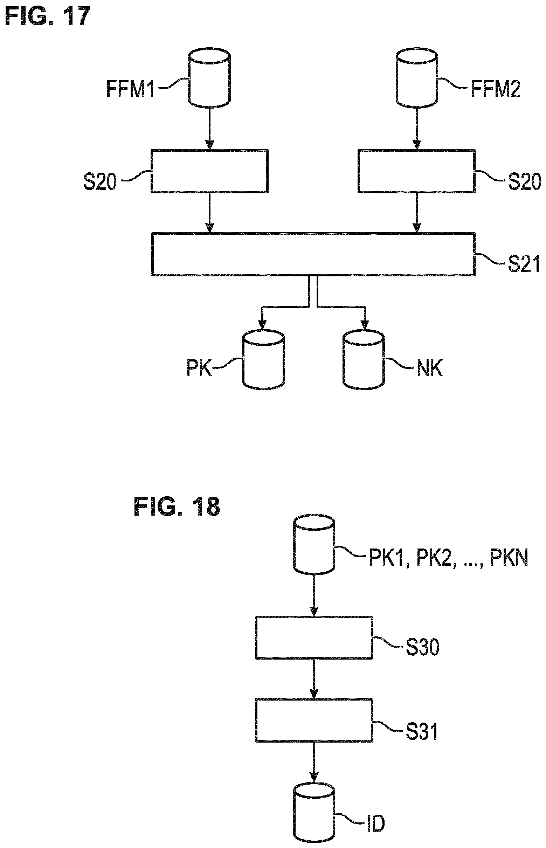

Gemäß der Figur 17 wird auf Basis der ersten finalen Feature Map FFM1 als auch vorzugsweise der zweiten finalen Feature Map FFM2 dann ein positives Konfidenzmaß PK bestimmt bezogen auf eine Färbung des Kinetoplastbereiches bzw. auf ein Vorliegen einer Bindung von Autoantikörpern in dem entsprechenden Kinetoplastbereich K des zweiten Teilbildes ZTB. Vorzugsweise wird auf Basis der ersten finalen Feature Map FFM1 als auch vorzugsweise der zweiten finalen Feature Map FFM2 ein negatives Konfidenzmaß NK bestimmt bezogen auf eine Färbung des Kinetoplastbereiches bzw. auf ein Vorliegen einer Bindung von Autoantikörpern in dem entsprechenden Kinetoplastbereich K des zweiten Teilbildes ZTB.

Vorzugsweise kann auf Basis lediglich der ersten finalen Feature Map ein Konfidenzmaß bezogen ein Vorliegen einer Bindung von Autoantikörpern in einem jeweiligen Kinetoplastbereich für das jeweilige zweite Teilbild ZTB bestimmt werden, ohne die zweite finale Feature Map FFM2 verwenden zu müssen. Es kann dann beispielsweise in einem Schritt S20 die Feature Map FFM1 einem sogenannten Max-Pooling zugeführt werden, welches für die finale Feature Map FFM1 den maximalen Pixelwert als einen einzelnen Skalarwert ermittelt. Vorzugsweise kann dieser Skalarwert als das Konfidenzmaß verwendet werden. Vorzugsweise kann auf Basis dieses Skalarwertes dann beispielsweise mittels einer sogenannten Sigmoid-Funktion ein Wert als das Konfidenzmaß ermittelt werden. Vorzugsweise kann auf Basis dieses Skalarwertes dann beispielsweise mittels einer sogenannten Rectified Linear Unit Activation Funktion ein Wert als das Konfidenzmaß ermittelt werden.

Vorzugsweise werden beide jeweiligen Feature Maps FFM1, FFM2 wird in jeweiligen Schritten S20 jeweils einem sogenannten Max-Pooling zugeführt, welches für eine jeweilige finale Feature Map jeweils den maximalen Pixelwert als einen jeweiligen einzelnen Skalarwert ermitteln. Auf Basis dieser Skalarwerte kann dann in einer sogenannten Softmax-Funktion in einem Schritt S21 eine positive Wahrscheinlichkeit PK als das Konfidenzmaß bezogen auf das Vorliegen der Bindung von Autoantikörpern in dem Kinetoplastbereich bzw. bezogen auf eine Färbung des Kinetoplastbereiches bestimmt werden. Die negative Wahrscheinlichkeit NK kann ebenfalls durch die Softmax-Funktion bestimmt werden. Positive Wahrscheinlichkeit PK und negative Wahrscheinlichkeit NK bilden vorzugsweise addiert eine Summe vom Wert 1. Auf diese Weist kann also für ein jeweiliges zweites Teilbild ZTB durch Ermittlung der ersten finalen Feature Map FFM1 und vorzugsweise auch der zweiten finalen Feature Map FFM2 dann gemäß der Figur 17 ein jeweiliges Konfidenzmaße bezogen auf das Vorliegen der Bindung von Autoantikörpern in den Kinetoplastbereich des zweiten Teilbildes bestimmt werden.

Zu der Softmax-Funktion alternative Funktionen sind beispielsweise die Sigmoid-Funktion, die Rectified Linear Unit Activation Funktion oder die Leaky Rectified Linear Unit Activation Funktion.

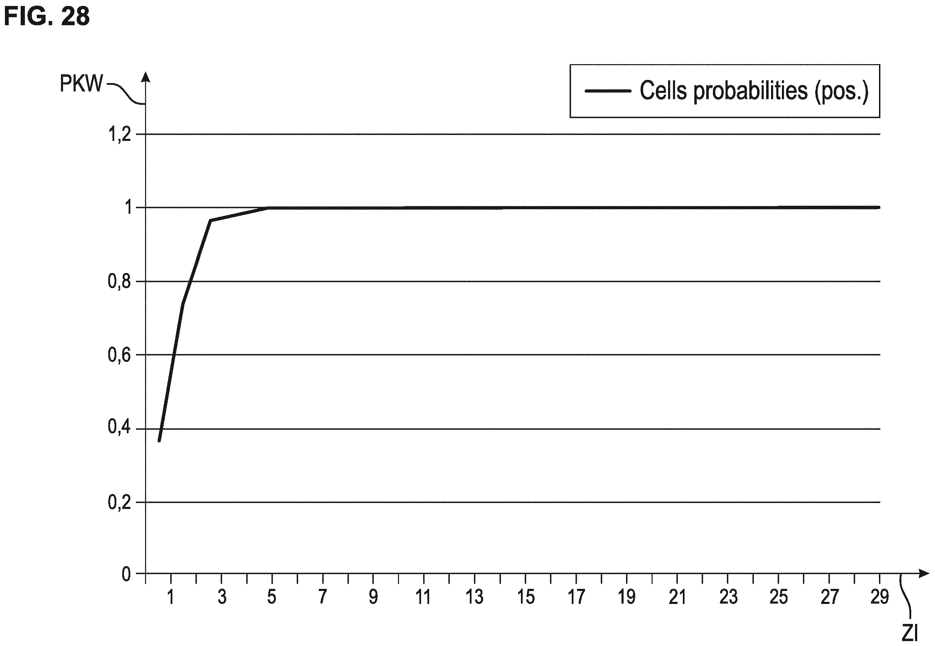

Die Figur 18 zeigt Schritte für ein Auswählen einer Teilmenge der zweiten Teilbilder aus dem zweiten Fluoreszenzbild auf Basis der für die zweiten Teilbilder jeweiligen positiven Konfidenzmaße. Die positiven Konfidenzmaße PK1, PK2, PK3,....., PKN mit Index 1, ..., N von N unterschiedlichen zweiten Teilbildern werden in einem Schritt S30 hinsichtlich ihrer Wertigkeit aufsteigend sortiert. Beispielhaft zeigt die Figur 28 für 29 verschiedene Crithidia luciliae Zellen bzw. für 29 verschiedene zweite Teilbilder aus einem zweiten Fluoreszenzbild die jeweils zugehörigen positiven Konfidenzmaße PK, welche bezüglich ihrer Werte PKW in aufsteigender Wertigkeit über einem entsprechenden Sortierindex 1, ..., 29 aufgetragen sind. Es werden dann in dem Schritt S31 jene Konfidenzmaße und ihre jeweils dazugehörigen zweiten Teilbilder ausgewählt, deren zugehörige Konfidenzmaßwerte die 50 % der höchsten Konfidenzmaßwerte PKW darstellen. Diese Untermenge der dazugehörigen zweiten Teilbilder wird dann durch Ausgabe der entsprechenden Teilbild-Indizes mittels eines Datensatzes ID ausgegeben bzw. indiziert. Anhand der Indizes aus dem Datensatz ID können dann die entsprechenden zweiten Teilbilder und ihre zugehörigen Bindungsmaße für eine Bestimmung des Gesamtbindungsmaßes ausgewählt werden.

Das Gesamtbindungsmaß wird dann auf Basis jener Bindungsmaße bestimmt, welche zu den ausgewählten zweiten Teilbildern gehören. Eine solche Ermittlung des Gesamtbindungsmaßes findet insbesondere in einem Schritt des Post-Processing PP innerhalb der vierten Prozessierungsebene P4 des Convolutional Neural Network statt, wie in Figur 16a dargestellt.

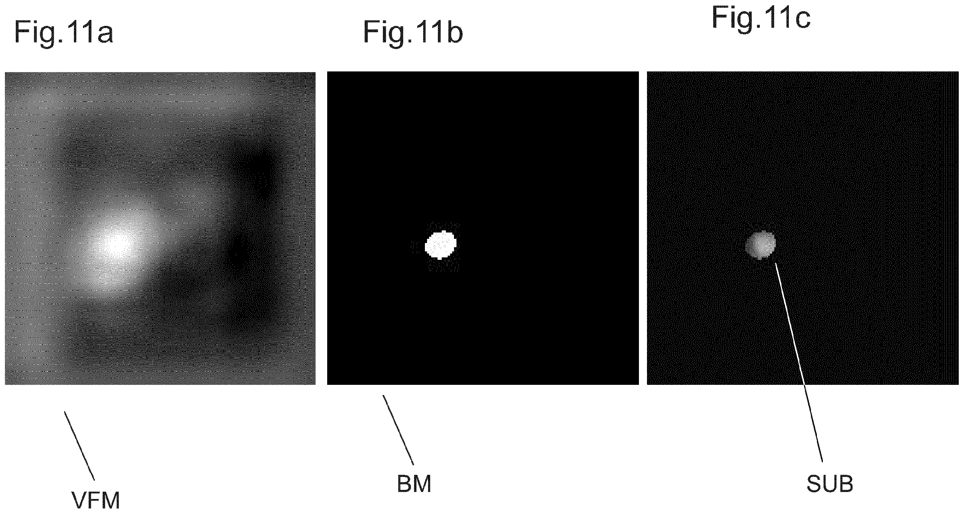

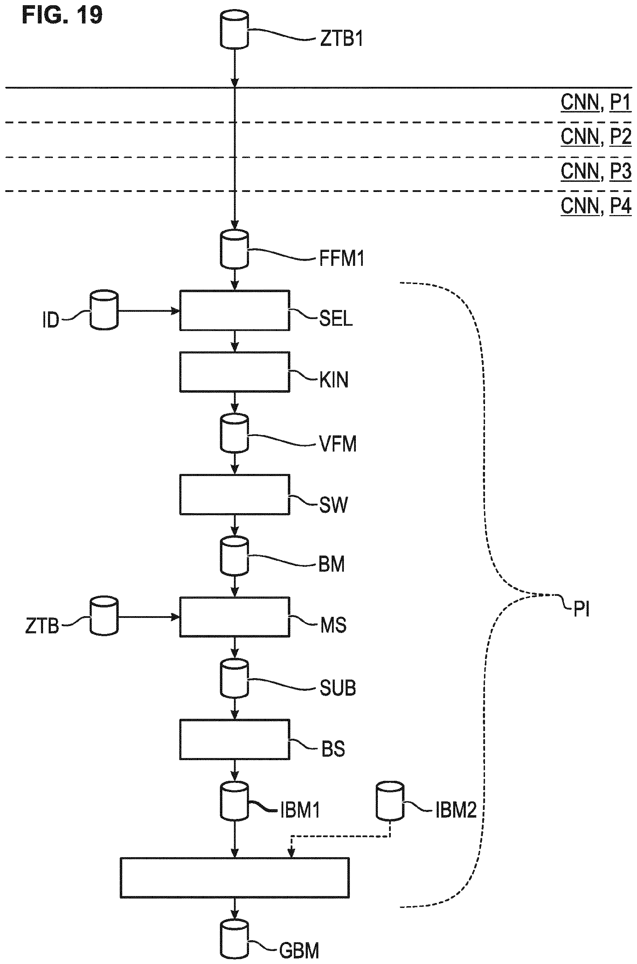

Die Figur 19 zeigt noch einmal eine beispielhafte Prozessierung eines einzelnen zweiten Teilbildes ZTB1 durch das Convolutional Neural Network CNN. Zunächst wird das zweite Teilbild ZTB1 durch die ersten drei Prozessierungsebenen P1 bis P3 des Convolutional Neural Network prozessiert, sodass die finale Feature Map FFM1 und vorzugsweise auch die zweite finale Feature Map bereitgestellt werden. In einem Auswahlschritt SEL erfolgt dann auf Basis der unter Bezug auf die Figuren 17 und 18 erläuterten Indexdaten ID das potentielle Auswählen des zweiten Teilbildes ZTB1 in die Teilmenge der zweiten Teilbilder. Ist das Teilbild ZTB1 in diese Teilmenge ausgewählt worden, so erfolgt dann das weitere Prozessieren als Post-Processing PP in der vierten Prozessierungsebene P4. Hier erfolgt nach Bestimmen der positiven Konfidenzmaße und nach dem Auswählen der Teilmenge zweiter Teilbilder dann ein jeweiliges Post-Processing der jeweiligen ersten Feature Maps der ausgewählten jeweiligen zweiten Teilbilder. Beispielhaft ist hier also für ein individuelles zweites Teilbild ZTB1 ein Post-Processing PP zur Bestimmung eines individuellen Bindungsmaßes IBM1 gezeigt. Hierbei erfolgt für das zweite Teilbild ZTB1 ein Selektieren eines jeweiligen Subbildes auf Basis der finalen Feature Map FFM1. Das Subbild präsentiert einen jeweiligen Kinetoplastbereich der entsprechenden Crithidia luciliae Zelle in dem zweiten Teilbild. Ein solches zweites Teilbild ZTB ist beispielhaft in Figur 10a dargestellt. Die zugehörige erste Feature Map FFM1 aus der Figur 10b wird dann in einem Schritt KIN mittels kubischer Interpolation in ihrer Größe und Auflösung auf das zweite Teilbild ZTB aus der Figur 10a angepasst, sodass sich die vergrößerte Feature Map VFM aus der Figur 11a ergibt.

In einem Schwellenwertschritt SB wird dann eine binärwertigen Maske BM ermittelt, welche in der Figur 11 dargestellt ist. Hierbei wird vorzugsweise als Schwellenwert jener Wert angewendet, welcher die Hälfte der maximal möglichen Grauwertintensität einer Feature Map darstellt. Liegt also ein Grauwert einer Feature Map VFM zwischen den Werten 0 und 1, so wird als Schwellenwert der Wert 0,5 angewendet.

In einem Schritt MS wird dann der Maskierungsoperator BM aus der Figur 11b auf das zweite Teilbild ZTB angewendet, sodass sich das entsprechende Subbild SUB aus der Figur 11c ergibt. Das vorgeschlagene Convolutional Neural Network CNN ist also in der Lage, für jedes jeweilige zweite Teilbild ZTB eine finale Feature Map FFM1 bereitzustellen, welche mittels ihrer Werte einen Subbildbereich für das zweite Teilbild indiziert, der dem Kinetoplasten entspricht. Das Convolutional Neural Network ist also in der Lage ein entsprechendes Subbild aus dem zweiten Teilbild herauszuschneiden bzw. zu selektieren, welches dann für die Bestimmung eines Bindungsmaßes einer Bindung von Autoantikörpern an doppelsträngige DNS an den Kinetoplasten herangezogen werden kann.

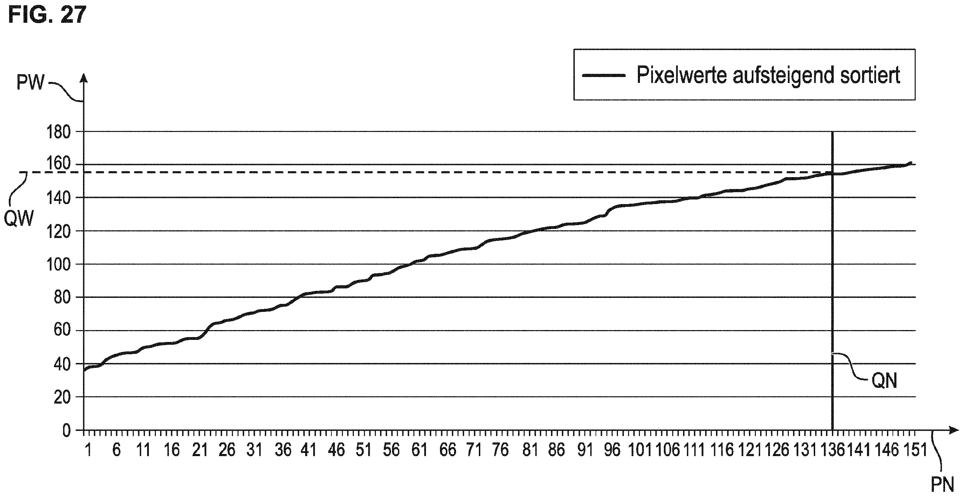

Gemäß der Figur 19 wird dann in einem Bestimmungsschritt BS das Bindungsmaße IBM1 für das zweite Teilbild ZTB1 bestimmt. Dies erfolgt, indem die Pixelwerte aus dem Subbild SUB betrachtet werden und jener Wert gewählt wird, welcher das 90% Quantil für die Pixelwerte aus dem Subbild definiert. Figur 27 zeigt hierzu für die Pixel des Subbildes SUB aus der Figur 11c die entsprechenden Pixelwerte PW in aufsteigender Größe mit entsprechendem Sortierungsindex PN. Der Wert QW für den Index QN ist jener, für welchen 90% der Pixelwerte aus dem Subbild SUB kleiner als der Wert QW sind.

In einem danach folgenden Schritt wird dann auf Basis der mehreren individuellen Bindungsmaße IBM1, IBM2 ... der individuellen zweiten Teilbilder aus der ausgewählten Teilmenge das Gesamtbindungsmaß GBM bestimmt.

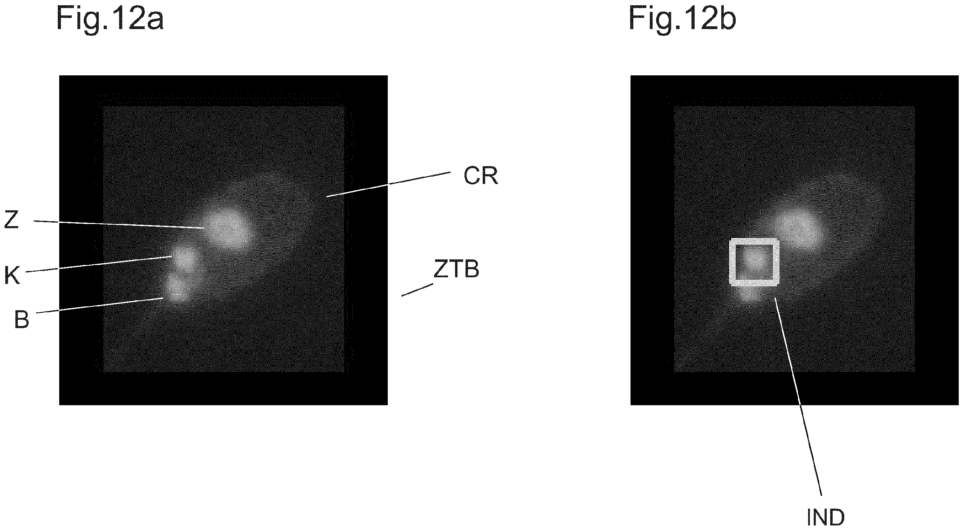

Figur 12b zeigt für das zweite Teilbild aus der Figur 12a einen indizierten Bereich IND, der den Bereich des Subbildes SUB aus der Figur 11c näherungsweise indiziert. Es kann klar erkannt werden, dass die Auswahl des Subbildbereiches durch das Convolutional Neural Network hier erfolgreich war, um den Kinetoplasten zu identifizieren.

Die Figur 16a zeigt eine beispielhafte Ausführungsform des Convolutional Neural Network CNN mit mehreren Prozessierungsebenen P1, P2, P3, P4.

In einer ersten Prozessierungsebene P1 generiert das CNN auf Basis eines zweiten Teilbildes ZTB mittels wenigstens eines ersten Convolutional Layer LA1 und mittels Anwendung mehrerer zweidimensionaler Faltungskernel eine erste Menge von zweidimensionalen resultierenden Feature Maps RFM1. Diese Feature Maps RFM1 müssen nicht direkt aus dem Convolutional Layer LA1 hervorgehen, sondern können mittels weiterer Prozessierungsschritte PS2, PS3, PSC generiert werden.

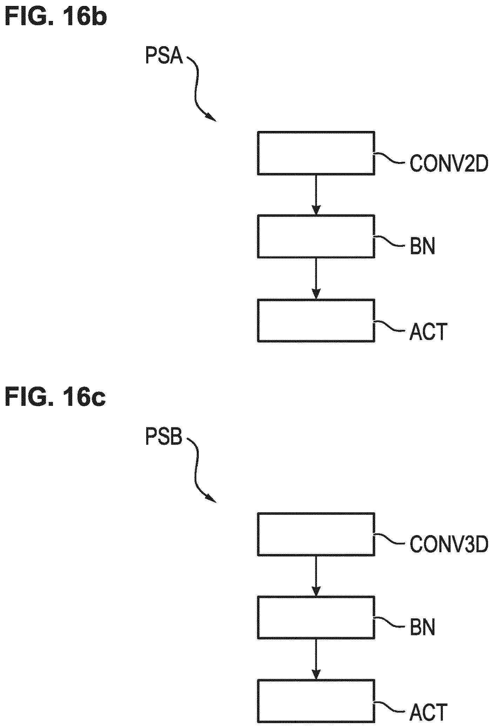

In dem Convolutional Layer LA1 erfolgt eine Prozessierung in einem Schritt PS1 mit einer Abfolge unterschiedlicher Teilschritte. Der Schritt PS1 ist vom Typ eines Schrittes PSA, welcher im Detail in der Figur 16b dargestellt ist. Zunächst erfolgt eine zweidimensionale Faltung CONV2D des eingehenden zweiten Teilbildes mit mehreren Faltungskernel mittels jeweiliger zweidimensionaler Faltungen. Eine anschließende Batch-Normalization erfolgt in einem Schritt BN. Es folgt dann eine sogenannte Activation in einem Schritt ACT.

Im Sinne dieser Anmeldung weist ein Convolutional Layer ein Layer zur Faltung einer oder mehrerer Feature Maps mit einem oder mehreren Faltungskernel auf. Solch ein Layer zur Faltung kann vorzugsweise dann innerhalb des Convolutional Layer von einem Layer einer Batch-Normalization und/ oder einem Layer einer Activation gefolgt sein.

In einer zweiten Prozessierungsebene P2 aus der Figur 16a erfolgt dann auf Basis der ersten Menge von Feature Maps RFM1 mittels wenigstens eines zweiten Convolutional Layer LA2 und mittels Anwendung mehrerer dreidimensionaler Faltungskernel eine Generierung einer zweiten Menge von zweidimensionalen resultierenden Feature Maps RFM2.

Auf Basis der zweiten Menge RFM2 erfolgt dann mittels wenigstens eines dritten Convolutional Layer LA3 und mittels Anwendung mehrerer dreidimensionaler Faltungskernel eine Generierung einer dritten Menge von zweidimensionalen resultierenden Feature Maps RFM3 dargestellt. Diese dritte Menge RFM3 geht direkt oder indirekt in die weitere Prozessierung der dritten Ebene P3 ein. In der dritten Ebene erfolgt eine Bestimmung der ersten finalen Feature Map FFM1 sowie vorzugsweise der zweiten finalen Feature Map FFM2 auf Basis der dritten Menge RFM3 mittels weiterer Convolutional Layer LAX.

Die zweite Menge RFM2 weist eine geringere Anzahl an Feature Maps auf als die erste Menge RFM1. Ferner weist die dritte Menge RFM3 eine größere Anzahl resultierender Feature Map auf als die zweite Menge RFM2. Ein Faltungskernel kann auch als ein Faltungskern oder als ein Faltungsoperator bezeichnet werden.

Durch die Reduktion der Anzahl der Feature Maps in dem zweiten Layer Convolutional LA2 erfolgt ein sogenanntes Squeezing. Die Feature Maps der ersten Menge RFM1 bzw. deren Merkmale werden mittels der Faltungskernel in einen Unterraum projiziert, da die dreidimensionalen Faltungskernel auf Merkmalskorrelation zwischen den Feature Maps reagieren. Es werden also nur die dominantesten Merkmale aus den Feature Maps der ersten Menge RFM1 beibehalten und in Feature Maps der zweiten Menge RFM2 hineinprojiziert. Weniger dominant und weniger aussagekräftige Merkmale werden also hierdurch herausgefiltert.

Durch die Erhöhung der Anzahl von Feature Maps durch das dritte Convolutional Layer LA3 von der zweiten Menge RFM2 hin zu der dritten Menge RFM3 werden die zuvor reduzierten Merkmale bzw. Informationen auf unterschiedliche Merkmalsräume und unterschiedliche Feature Maps verteilt, wobei die Merkmale unterschiedlich kombiniert werden können aufgrund der Freiheitsgrade der in dem Dritten Convolutional Layer LA3 verwendeten dreidimensionalen Faltungskernel. Dieses entspricht einem sogenannten Expandieren bzw. einem "Expand".

Auf das erste Convolutional Layer LA1 kann in der ersten Prozessierungsebene P1 ein weiteres Convolutional Layer LA11 eins folgen. Dieses Layer LA11 verwendet die in dem Layer LA1 erstellten Feature Maps. Vorzugsweise weist das Layer LA11 parallel zueinander angeordnete Prozessionsschritte PS2, PS3 auf. Diese Prozessionsschritte PS2, PS3 sind jeweils von der Art des Prozessionsschrittes PSB aus der Figur 16 C. In einem Teilschritt CONV3D des Schrittes PSB erfolgt eine dreidimensionale Faltung der Feature Maps mit jeweiligen dreidimensionalen Faltungskerneln. Es folgt dann in einem weiteren Teilschritt BN eine sogenannte Batch-Normalization. Es folgt ferner in einem Schritt ACT eine sogenannte Activation.

Die sich aus den Schritten PS2 sowie PS3 des Layer LA11 ergebenden Feature Maps werden dann in einem Konkatenierungs-Schritt PSC miteinander konkateniert; mit anderen Worten: die Feature Maps werden aneinandergereiht.

Vorzugsweise erfolgt in der ersten Prozessionsstufe P1 ferner eine Faltung des zweiten Teilbildes ZTB in einem Schritt PS4 mit zweidimensionalen Faltungskernel. Der Schritt PS4 ist von der Art des Teilschrittes CONV2D aus der Figur 16b.

Es können vorzugsweise die sich aus dem Layer LA11 und die aus dem Schritt PS4 ergebenden Feature Maps in der Weise miteinander verknüpft werden, dass die Einträge der Feature Maps jeweils elementweise miteinander addiert werden. Hierdurch ergibt sich also keine Veränderung der Dimensionalität der Feature Maps sondern die einzelnen Elemente Feature Maps aus dem Layer LA11 werden mit den einzelnen Elementen der Feature Maps aus dem Schritt PS4 elementweise addiert.

Der Schritt PS5 aus dem zweiten Convolutional Layer LA2 ist von der Art Schrittes PSB aus der Figur 16c.

Vorzugsweise werden die Feature Maps aus dem Convolutional Layer LA2 in dem dritten Convolutional Layer LA3 in der Weise prozessiert, dass in entsprechenden Schritten PS7 sowie PS8 als auch in dem Schritt PSC die Feature Maps in einer analogen Weise verarbeitet werden wie jene aus dem Convolutional Layer 11, wobei eine Anzahl verwendeter Faltungskernel und eine Dimensionalität der Faltungskernel voneinander abweichen können. Die Schritte PS7 sowie PS8 sind von der Art des Schrittes PSB aus der Figur 16c. Durch ein elementweises Addieren der jeweiligen Feature Maps aus den jeweiligen Schritten PS7 und PS8 wird dann durch einen Schritt PSC eine dritte Menge an Feature Maps RFM3 generiert.

In der zweiten Prozessierungsebene P2 folgen das zweite Convolutional Layer LA2 und das dritte Convolutional Layer LA3 als Teilschritte eines sequenziellen Prozessierungspfades PF1 aufeinander ab. In der zweiten Prozessierungsebene P2 ist ferner parallel zu dem sequenziellen Prozessierungspfad PF1 ein weiterer Prozessierungspfad PF2 vorhanden, in welchem das CNN auf Basis der ersten Menge RFM2 mittels wenigstens eines vierten Convolutional Layer LA4 und mittels Anwendung mehrerer dreidimensionaler Faltungskernel eine vierte Menge RFM4 von zweidimensionalen resultierenden Feature Maps generiert.

Dies erfolgt durch einen Schritt PS6, welcher von der Art des Teilschrittes CONV3D aus der Figur 16c ist.

Eine durch den Schritt PSS in der Prozessierungsebene P2 bestimmte Menge an Feature Maps RFM5 wird dann wiederum mittels eines Schrittes PSS aus der dritten Menge RFM3 von Feature Maps und der vierten Menge RFM4 von Feature Maps generiert. Diese Menge von Feature Maps RFM5 kann dann in einer dritten Prozessierungsebene P3 verwendet werden, um mittels weiterer Schritte LAX, welche später noch detailliert erläutert werden, die erste finale Feature Map FFM1 und vorzugsweise die zweite finale Feature Map FFM2 zu generieren.

In einer weiteren Prozessierungsebene PS4 erfolgt dann das sogenannte Post-Processing, wie in der Figur 19 im Detail erläutert.

Das CNN generiert also die zu dem zweiten Teilbild ZTB korrespondierende finale Feature Map FFM1 auf Basis der dritten Menge RFM3 von Feature Maps und auf Basis der vierten Menge RFM4 von Feature Maps. Die Anzahl der aufeinanderfolgenden Convolutional Layer LA4 in dem parallelen Prozessierungspfad PF2 ist hierbei geringer als die Anzahl der aufeinander folgenden Convolutional Layer LA2, LA3 in dem sequenziellen Prozessierungspfad PF1. Der parallele Prozessierungspfad PF2 weist also weniger Convolutional Layer auf als der sequenzielle Pfad PF1. Hierduch wird es im Zuge eines Trainings des Convolutional Neural Network ermöglicht, dass bei einer Neuberechnung einzelner Gewichte der Faltungskernel im Zuge einer Backpropagation das Problem des sogenannten "Vanishing Gradient" vermieden bzw. reduziert wird.

Wie zuvor in Bezug auf die Figur 16a dargelegt, kann das CNN aus vier Prozessierungsebenen bestehen.

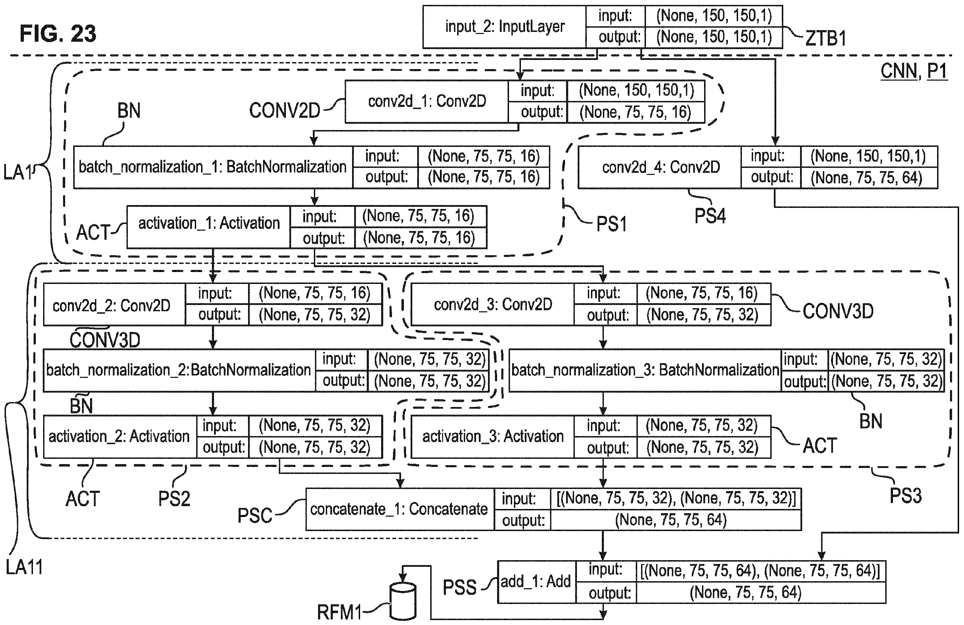

Figur 23 zeigt hierzu eine detaillierte Ausführungsform der ersten Prozessierungsebene P1, welche zu der ersten Prozessierungsebene P1 aus der Figur 16a korrespondiert.

Für jeden einzelnen Schritt ist im Detail angegeben, von welcher Dimensionsalität eine Eingangsgröße in Form eines zweiten Teilbildes oder einer Menge von Feature Maps ist.

Hierbei kann für jeden einzelnen Schritt in der oberen Zeile "Input" in darauffolgenden Klammern durch den zweiten und dritten Eintrag die Dimensionalität der Eingangsgröße bzw. Eingangsgrößen entnommen werden. Beispielsweise sind die zweiten Teilbilddaten ZTB1 von einer Dimensionalität von 150 × 150 Pixel. Es gibt für die Daten ZTB1 nur eine einzige Eingangsgröße, welches in dem vierten bzw. letzten Eintrag in den Klammern durch das Element "1" indiziert wird. Die Bilddaten ZTB1 sind in ihrem Wertebereich vorzugsweise auf einen Wertebereich von 0 bis 1 normiert.

In dem Schritt PS wird beispielsweise diese Eingangsgröße ZTB1 dann in der Weise mit Faltungskernel prozessiert, dass sich Feature Maps von einer Dimensionalität von 75 × 75 Pixeln ergeben. Der letzte Eintrag in der unteren Zeile "Output" indiziert hierbei die Anzahl der generierten Feature Maps der resultierenden Menge von Feature Maps. Hierdurch kann ein Fachmann also für jeden Prozessierungsschritt aus den hier angegebenen Parametern klar ableiten, wieviele Faltungskernel auf eingehende Daten ZTB1 oder eingehende Feature Maps angewendet werden müssen, um auf eine bestimmte Anzahl ausgehender Feature Maps zu kommen. In dem Beispiel Schrittes PS4 sind dies 64 Faltungskernel. Ferner kann ein Fachmann aufgrund der angegebenen Dimensionalität der eingehenden Feature Maps und der angegebene Dimensionalität der ausgehenden Feature Maps ableiten, inwiefern ein sogenanntes "Striding", also ein Versatz während des Faltens einer Feature Map mit einem Faltungskernel, um eine bestimmte Anzahl von Pixel vorgenommen werden muss. In dem Beispiel des Schrittes PS4 ist dies ein Striding des Wertes 2.

Dem Fachmann werden als durch die Angaben aus der Figur 23 klare Anweisungen gegeben, um die Prozessierungsebene P1 des CNN auszugestalten.

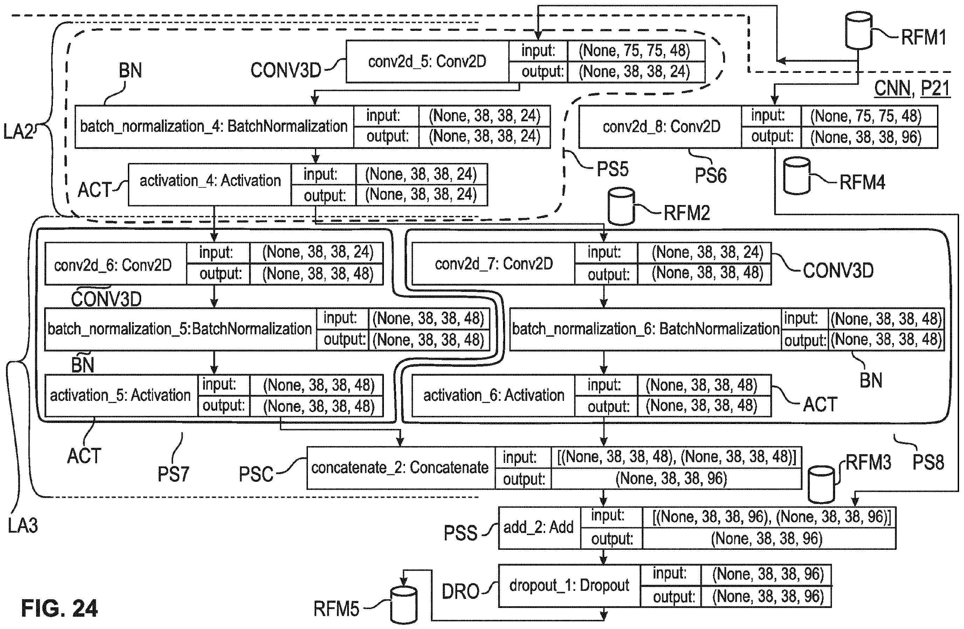

Die Figur 24 zeigt einen ersten Teil P21 der Prozessierungsebene P2 aus der Figur 16a. Hierbei entspricht die Struktur der Teil-Prozessierungsebene P21 im Wesentlichen der Prozessierungsebene P2 aus der Figur 16a. Zusätzlich ist hier noch ein so genannter Schritt eines "Dropout" für die Traingsphase vorgesehen, in welchem einzelne Einträge der Feature Maps während des Trainings, nicht aber während der tatsächlichen Klassifikation in der Testphase, in ihren Pixelwerten auf den Wert "0" (null) gesetzt werden. Der Drop-Faktor ist hierbei vorzugsweise ein Wert von 50 %, sodass die Hälfte der Pixelwerte wird auf null gesetzt werden. Die Pixelwerte werden zufällig gewählt, indem ihre Indizes mittels einer Zufallsfunktion ausgewählt werden.

Es ergibt sich dann in der Teil-Prozessierungsebene P21 die Menge RFM5 an Feature Maps, wie zuvor in der Figur 16a in der Prozessierungsebene P2 gezeigt.

Vorzugsweise kann das CNN eine weitere Teil-Prozessierungsebene P22 aufweisen, welche zuvor in der Figur 16a nicht dargestellt wurde. Hierbei wird die Menge an Feature Maps RFM5 weiterverarbeitet, um eine modifizierte Menge an Feature Maps RFM51 zu generieren.

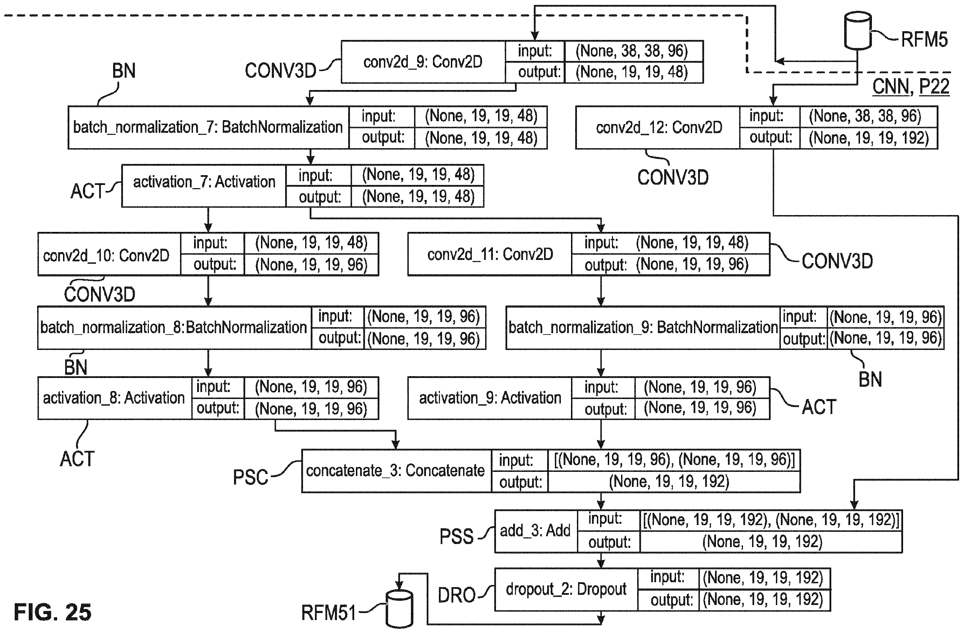

Auch hier sind in Figur 25 genaue Angaben für einen Fachmann enthalten, in welcher Weise diese modifizierte Menge an Features Maps RFM51 zu generieren ist. Auch hier erfolgt ein sogenanntes Dropout DRO während der Trainingsphase aber nicht der Testphase.

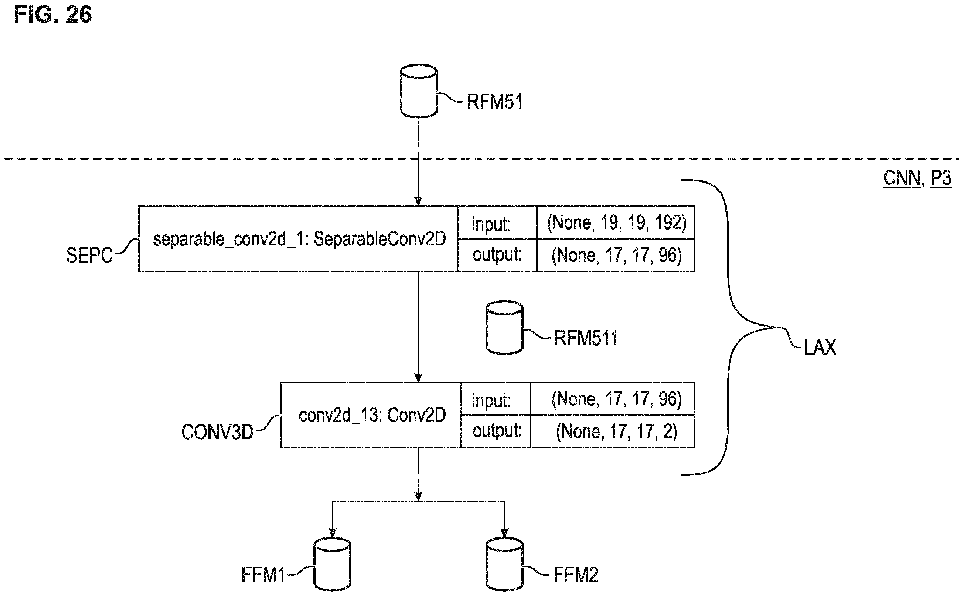

Die Figur 26 zeigt eine Ausführungsform der dritten Prozessierungsebene P3 zur Generierung der ersten finalen Feature Map FFM1 als auch vorzugsweise der zweiten finalen Feature Map FFM2. Die Feature Map FFM1 als auch vorzugsweise die Feature Map FFM2 wernde indirekt auf Basis der dritten Menge an Feature Maps RFM3 und der vierten Menge RFM4 an Feature Maps generiert. Hierbei weist der zuvor in Figur 16a dargestellte Prozessierungsschritt LAX Teilschritte auf. In einem ersten Teilschritt SEPC erfolgt eine sogenannte "depthwise convolution" der eingehenden Feature Maps RFM51, bei welcher jede einzelne zweidimensionale Feature Map aus der Menge RFM51 jeweils für sich mit einem zweidimensionalen Faltungskernel gefaltet wird, woraus sich eine Menge an zweidimensionalen Feature Maps RFM511 ergibt. Es erfolgt dann direkt im Anschluss in einem weiteren Teilschritt CONV3D eine Faltung der sich aus dem Schritt SEPC ergebenden mehreren zweidimensionalen Feature Maps der Menge RFM511 mit einem dreidimensionalen Faltungskernel der Dimensionalität 1x1xG , wobei G die Anzahl der Feature Maps der Menge ist, so dass hieraus die erste finale Feature Map FFM1 bestimmt wird. Vorzugsweise erfolgt in dem weiteren Teilschritt CONV3D ferner auch eine weitere Faltung der sich aus dem Schritt SEPC ergebenden mehreren zweidimensionalen Feature Maps der Menge RFM511 mit einem weiteren dreidimensionalen Faltungskernel der Dimensionalität 1x1xG , wobei G die Anzahl der Feature Maps der Menge ist, so dass hieraus die erste finale Feature Map FFM2 bestimmt wird.

An die dritte Prozessierungsebene P3 aus der Figur 26 schließt sich dann das sogenannte Post-Processing PP an, wie in der Figur 16a in der Prozessierungsebene P4 angedeutet und wie in der Figur 19 im Detail erläutert.

Für eine Implementation eines oder mehrerer Ausführungsbeispiele des hier vorgeschlagenen Convolutional Neural Network kann ein Fachmann auf eine sogenannte Open Source Deep-Learning Bibliothek namens "Keras" zurückgegriffen werden. Detaillierte Informationen findet der Fachmann unter https://keras.io . Die Ausführungsform des vorgeschlagenen CNN mit den Prozessierungsebenen P1, P21, P22 und P3 aus den Figuren 23 bis 26 sowie der Prozessierungsebene P4, wie im Detail in Figur 19 illustriert, wurde für Versuchszwecke mittels der sogenannte Open Source Deep-Learning Bibliothek "Keras" erstellt. Es wurden zwei Datensätze an Fluoreszenzbildern verwendet. Der erste Datensatz an Fluoreszenzbildern war ein solcher, bei welchem es bekannt war, dass die zur Inkubation verwendeten Patientenproben Autoantikörper aufwiesen und somit in den Fluoreszenzbildern die Kinetoplastbereiche eine relevante Färbung aufwiesen. Der zweite Datensatz an Fluoreszenzbildern war ein solcher, bei welchem es bekannt war, dass die zur Inkubation verwendeten Patientenproben keine Autoantikörper aufwiesen und somit in den Fluoreszenzbildern die Kinetoplastbereiche keine relevante Färbung aufwiesen.

Mittels einer Vorprozessierung des ersten Datensatzes von Fluoreszenzbildern in der hier detailliert beschriebenen Weise wurden dann aus den entsprechenden ersten Fluoreszenzbildern insgesamt ca. 23.000 erste Teilbilder und somit auch ca. 23.000 korrespondierende zweite Teilbilder der zweiten Fluoreszenzbilder bestimmt, für welche eben eine Bindung von Autoantikörpern aus einer Patientenprobe an dsDNS im Kinetoplastbereich gegeben war, so dass der Kinetoplastbereich eine signifikante Färbung aufwies und daher eine positive Klassifikationsentscheidung gefällt werden muss.

Mittels einer Vorprozessierung des zweiten Datensatzes von Fluoreszenzbildern in der hier detailliert beschriebenen Weise wurden dann aus den entsprechenden ersten Fluoreszenzbildern insgesamt ca. 23.000 erste Teilbilder und somit auch ca. 23.000 korrespondierende zweite Teilbilder der zweiten Fluoreszenzbilder bestimmt, für welche eben keine Bindung von Autoantikörpern aus einer Patientenprobe an dsDNS im Kinetoplastbereich gegeben war, so dass der Kinetoplastbereich keine signifikante Färbung aufwies und daher eine negative Klassifikationsentscheidung gefällt werden muss.

Von den ca. 23.000 als positiv zu beurteilenden zweiten Teilbildern wurden ca. 18.000 zweite Teilbilder für eine Trainingsphase des CNN verwendet. Die weiteren ca. 5.000 positiven zweiten Teilbilder wurden für eine Testphase verwendet. Von den 23.000 als negativ zu beurteilenden zweiten Teilbildern wurden ca. 18.000 für eine Trainingsphase des CNN verwendet. Die weiteren ca. 5.000 negativen zweiten Teilbilder wurden für eine Testphase verwendet.

Das CNN wurde in 100 Epochen unter Verwendung einer Learning-Rate von 1e-3 trainiert.

Bezogen auf eine Entscheidung, ob ein zweites Teilbild aus den 10.000 zweiten Teilbildern in der Testphase mittels des CNN anhand der Konfidenzmaße PK und NK korrekt als positiv oder negativ beurteilt wurde, ergab sich eine Sensitivität von 95,7% und eine Spezifität von 98,1%.

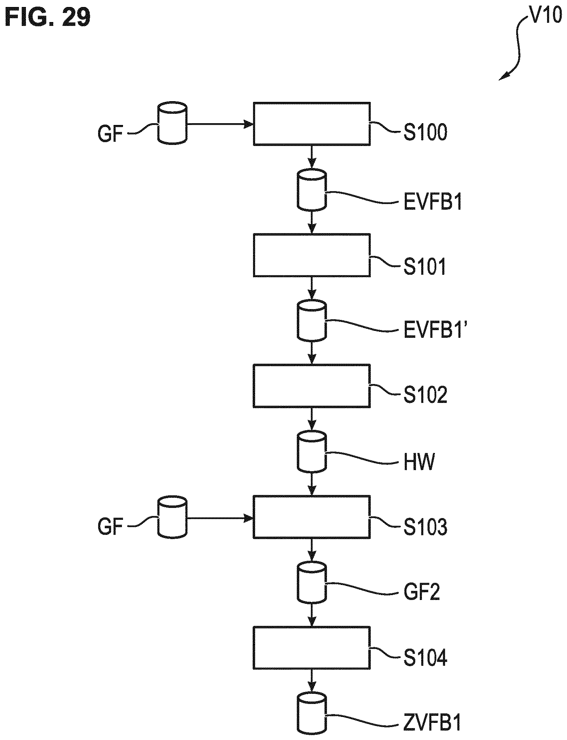

Die Figur 29 zeigt Verfahrensschritte V10, mittels welcher das erste Fluoreszenzbild des ersten Farbkanals mittels wenigstens eines ersten vorläufigen Fluoreszenzbildes bestimmt wird.

Unter Verwendung eines fest vorgegebenen Erfassungsparameters GF, welcher vorzugsweise ein Gain-Parameter für eine Skalierung erfasster Grauwerte ist, wird ein erstes vorläufiges Fluoreszenzbild EVFB1 in dem ersten Farbkanal erfasst. Es wird dann in einem Schritt S101 ein Histogramm über die Pixelwerte des ersten vorläufigen Fluoreszenzbildes EVFB1 erstellt. Mittels des Histogramms wird dann ein Schwellenwert als ein Grauwert bestimmt, welcher auf die Pixelwerte des ersten vorläufigen Fluoreszenzbildes EVFB1 angewendet wird. Hierbei kann beispielsweise zunächst der Peak-Wert des Histogramms bestimmt werden und dann der zu dem Peak-Wert zugehörige Grauwert bestimmt werden. Dieser Grauwert kann dann vorzugsweise um einen fest vorgegebenen Wert von fünf Pixelwerten erhöht werden, um so den Schwellenwert zu bestimmen. Pixelwerte des Bildes EVFB1, welche unterhalb dieses Schwellenwertes liegen, werden dann als Hintergrund klassifiziert und gehen nicht in ein modifiziertes erstes vorläufiges Fluoreszenzbild EVFB1' ein. Das modifizierte erste vorläufige Fluoreszenzbild EVFB1' repräsentiert dann jene Pixelwerte, welche den zu vorbestimmten Schwellenwert übersteigen.

Auf Basis dieser Pixelwerte des Bildes EVFB1' wird dann in dem Schritt der Mittelwert über diese Pixelwerte bildet, um einen Helligkeitswert HW zu bestimmen.

In einem Schritt S103 wird dann der Erfassungsparameter GF in Abhängigkeit des Helligkeitswertes HW modifiziert, um einen modifizierten Erfassungsparameter GF2 zu bestimmen. Vorzugsweise wird hierbei ein fest vorgegebener Parameterwert, beispielsweise ein Zielgrauwert des Wertes 55 für einen Quantisierungsbereich von 0 bis 255, durch den Helligkeitswert HW geteilt und dann mit dem zuvor fest vorgegebenen Erfassungsparameter GF multipliziert gemäß GF2= (55/HW) × GF . Hierdurch erfolgt also eine Skalierung des fest vorgegebenen Erfassungsparameters GF durch den Helligkeitswert HW zur Bestimmung eines modifizierten Erfassungsparameters GF 2. Dieser Erfassungsparameter ist vorzugsweise eben ein modifizierter Gain-Parameter GF2.

Unter Verwendung des modifizierten Erfassungsparameters GF2 wird dann in einem Schritt S104 ein zweites vorläufiges Fluoreszenzbild ZVFB1 erfasst und dann als das erste Fluoreszenzbild des ersten Farbkanals SR verwendet, siehe Figur 3.

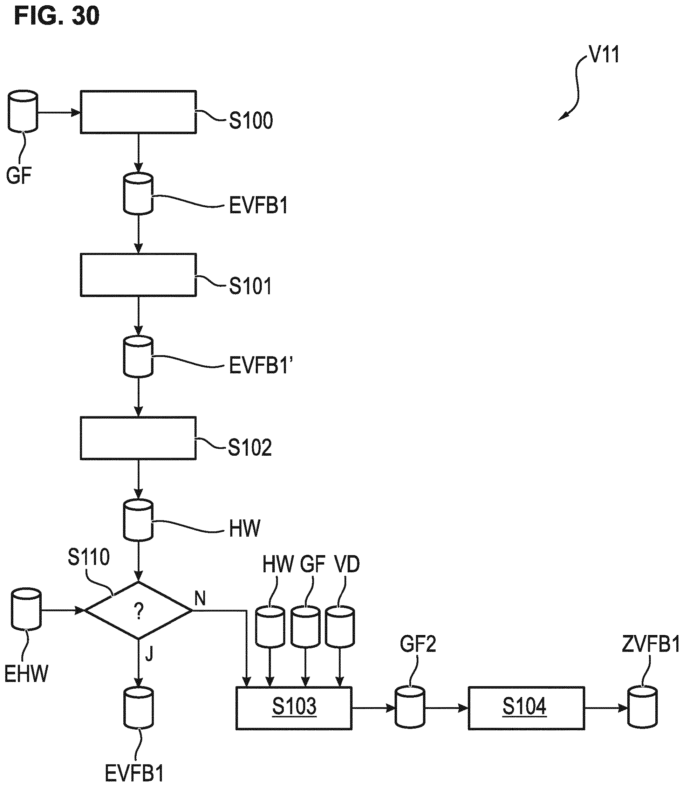

Die Figur 30 zeigt eine Variante V11 des Verfahrens V10 aus der Figur 29. Das Verfahren V11 aus der Figur 30 weist einen zusätzlichen Schritt S110 auf, in welchem überprüft wird, ob der Helligkeitswert des ersten vorläufigen Fluoreszenzbildes EVFB1 einem erwarteten Helligkeitswert EHW entspricht. Beispielsweise kann für einen beispielhaften Quantisierungsbereich von 0 bis 255 der Grauwerte dann der Helligkeitswert HW einem erwarteten Helligkeitswert EHW entsprechen, wenn er in ein Intervall von 45 bis 60 fällt. In diesem Fall wird dann aus dem Schritt S110 hin zu einer Ausgabe des ersten vorläufigen Fluoreszenzbildes EVFB1 verzweigt, sodass dieses Bild EVFB1 als das erste Fluoreszenzbild SR des ersten Farbkanals verwendet wird, siehe Figur 3.

Entspricht der Helligkeitswert HW nicht dem erwarteten Helligkeitswert, so wird in einem Schritt S103 ein modifizierter Erfassungsparameter GF2 in Abhängigkeit des zuvor bestimmten Helligkeitswertes HW bestimmt. Vorzugsweise kann in dem Fall, dass der Helligkeitswert HW in einen Wertebereich von 30 bis 45 fällt, der modifizierte Erfassungsparameter GF2 als der Erfassungsparameter GF skaliert um einen Faktor 1,75 bestimmt gemäß GF2 = 1.75 x GF . Vorzugsweise kann der Zweiterfassungsparameter GF2 in dem Fall, dass der Helligkeitswert HW in einen Wertebereich von 60 bis 70 fällt, der modifizierte Erfassungsparameter GF2 als der Erfassungsparameter GF skaliert um einen Faktor 0,625 bestimmt gemäß GF2 = 0,625 x GF.

Entsprechende Randwerte von Wertebereichen und entsprechende Skalierungsfaktoren können als Vorgabedaten VD bereitgestellt werden.

Es wird dann in dem Schritt S104 ein zweites vorläufiges Fluoreszenzbild ZVFB1 erfasst unter Verwendung des modifizierten Erfassungsparameters GF2. Das zweite vorläufige Fluoreszenzbild ZVFB1 wird dann als das erste Fluoreszenzbild SR des ersten Farbkanals verwendet, siehe Figur 3.

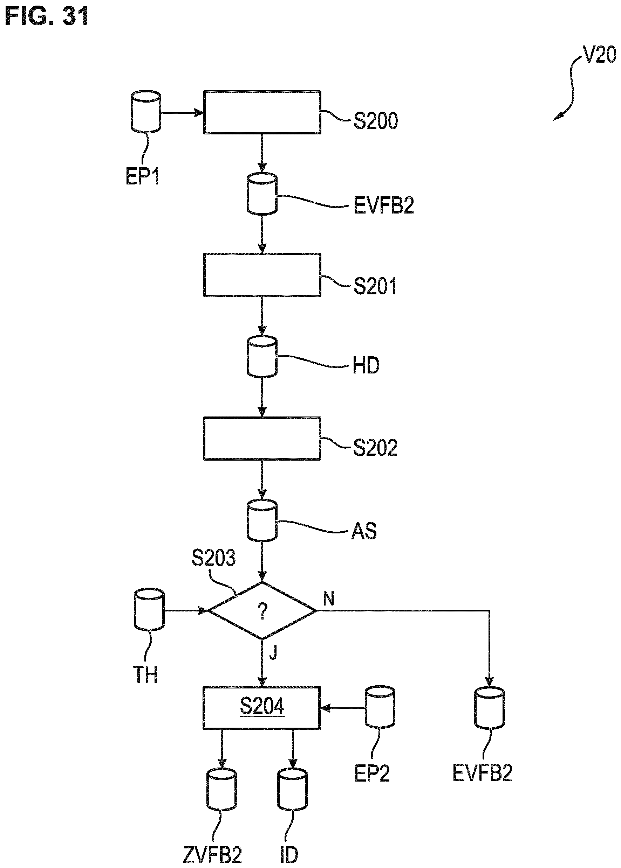

Die Figur 31 zeigt ein Verfahren V20, in welchem vorzugsweise durchzuführende Schritte zur Erfassung des zweiten Fluoreszenzbildes des zweiten Farbkanals durchgeführt werden.

In einem Schritt S200 wird mittels wenigstens eines fest vorgegebenen Erfassungsparameters EP1, welches vorzugsweise ein Gain-Parameter ist, ein erstes vorläufiges Fluoreszenzbild EVFB2 in dem zweiten Farbkanal erfasst.

In einem Schritt S201 wird dann ein Histogramm über die Pixelwerte des Bildes EVFB2 gebildet, sodass das Histogrammdaten HD ermittelt und bereitgestellt werden.

In einem Schritt S202 wird dann festgestellt, wie hoch die Anzahl von Pixeln ist, welche hinsichtlich einer Helligkeit eine bestimmte Sättigung überschreiten. Für einen beispielhaften Quantisierungsbereich der Pixelwerte von 0 bis 255 wird beispielsweise festgestellt, wie viele Pixel einen Pixelwert bzw. einen Grauwert von 255 aufweisen. Diese Anzahl der Pixel, welche in der Helligkeits-Sättigung sind, werden als Daten AS bereitgestellt.

Es wird dann in einem Schritt S203 überprüft, ob die Anzahl der Pixel AS, welche in einem Sättigungsbereich sind, einen vorgegebenen Schwellenwert TH überschreitet. Liegt keine Schwellenwertüberschreitung vor (siehe Verzweigung "N"), so wird das erste vorläufige Fluoreszenzbild des zweiten Farbkanals EVFB2 als das zweite Fluoreszenzbild des zweiten Farbkanals SG verwendet, siehe Figur 4. Überschreitet die Anzahl der Pixel, welche in der Sättigung sind, den Schwellenwert TH (siehe Verzweigung "J"), so wird in einem Schritt S204 ein zweites vorläufiges Fluoreszenzbild ZVFB2 in dem zweiten Farbkanal unter Verwendung wenigstens eines zweiten fest vorgegebenen Erfassungsparameters EP2 erfasst. Der fest vorgegebene Erfassungsparameter EP2 unterscheidet sich von dem zuvor fest vorgegebenen ersten Erfassungsparameter EP1. Vorzugsweise ist der Erfassungsparameter EP2 ein Gain-Parameter und ist geringer als der erste Erfassungsparameter EP1, sodass das Bild ZVFB2 weniger stark belichtet ist als das Bild EVFB2. Es wird dann das erfasste zweite vorläufige Fluoreszenzbild EVFB2 als das zweite Fluoreszenzbild SG des zweiten Farbkanals verwendet, siehe Figur 4. Vorzugsweise werden ferner Indikatordaten bereitgestellt, welche indizieren, dass die Helligkeit des ersten vorläufigen Fluoreszenzbildes EVFB2 eine maximale Helligkeit überstiegen hat und es sich bei dem zweiten Fluoreszenzbild SG um ein mit reduzierter Helligkeit erfasstes zweites vorläufiges Fluoreszenzbild ZVFB2 des zweiten Farbkanals handelt.

Es kann dann das zweite vorläufige Fluoreszenzbild EVFB2 des zweiten Farbkanals in üblicher Weise als das zweite Fluoreszenzbild SG des zweiten Farbkanals in dem vorgeschlagenen Verfahren verwendet werden. Hierbei ermittelt vorzugsweise das CNN mittels eines Konfidenzmaßes PK, ob für eine Mindestanzahl an zweiten Teilbildbereichen, vorzugsweise wenigstens zehn zweite Teilbildbereiche, eine Färbung von Kinetoplastbereichen vorhanden ist. Ist dies der Fall, so gibt das CNN als Gesamtbindungsmaß die maximale Helligkeit bzw. den maximalen Helligkeitswert, vorzugsweise 255, aus. Stellt das CNN fest, dass die Kinetoplastbereiche nicht wirklich gefärbt sind, so wird das zweite Fluoreszenzbild SG des zweiten Farbkanals als insgesamt negativ bewertet.

Obwohl manche Aspekte im Zusammenhang mit einer Vorrichtung beschrieben wurden, versteht es sich, dass diese Aspekte auch eine Beschreibung der entsprechenden Verfahren darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Recheneinheit R oder die Datennetzwerkvorrichtung in Hardware und/oder in Software umsetzen. Eine Umsetzung einer hier genannten Recheneinheit R kann hier als wenigstens eine Recheneinheit erfolgen oder aber durch mehrere Recheneinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Recheneinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird.

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

## Patentansprüche

1. Verfahren zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung, aufweisend
- Bereitstellen eines Substrates (S), welches mehrere Crithidia luciliae Zellen (CR) aufweist,
- Inkubieren des Substrates (S) mit der Patientenprobe, welche potentiell die Autoantikörper aufweist,
- Inkubieren des Substrates (S) mit einem ersten Fluoreszenzfarbstoff,
- Inkubieren des Substrates (S) mit sekundären Antikörpern, welche jeweils mit einem zweiten Fluoreszenzfarbstoff markiert sind, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wird,
- Erfassen eines ersten Fluoreszenzbildes (SR) des Substrates (S) in einem ersten Farbkanal, welcher zu dem ersten Fluoreszenzfarbstoff korrespondiert,
- Erfassen eines zweiten Fluoreszenzbildes (SG) des Substrates (S) in einem zweiten Farbkanal, welcher zu dem zweiten Fluoreszenzfarbstoff korrespondiert,
- Identifizieren jeweiliger erster Teilbilder (ETB) in dem ersten Fluoreszenzbild (SR), welche jeweils eine Crithidia Luciliae Zelle (CR) repräsentieren,
- Bestimmen jeweiliger zweiter Teilbilder (ZTB) des zweiten Fluoreszenzbildes (SG), welche zu den jeweiligen ersten Teilbildern (ETB) des ersten Fluoreszenzbildes (SR) korrespondieren,
- jeweiliges Prozessieren wenigstens einer Teilmenge der jeweiligen zweiten Teilbilder (ZTB) mittels eines vortrainierten Convolutional Neural Network (CNN) zur Bestimmung jeweiliger Bindungsmaße (IBM1, IBM2), welche einen jeweiligen Grad einer Bindung von Autoantikörpern in einem jeweiligen Kinetoplastbereich (K) einer jeweiligen Crithidia luciliae Zelle (CR) eines jeweiligen zweiten Teilbildes (ZTB) indizieren,
wobei das CNN ein jeweiliges zweites Teilbild (ZTB) für sich jeweils separat prozessiert um für ein jeweiliges zweites Teilbild (ZTB) ein jeweiliges Subbild zu bestimmen, das einen jeweiligen Kinetoplastbereich einer jeweiligen Crithidia luciliae Zelle (CR) repräsentiert,
und wobei das CNN das jeweilige Bindungsmaß (IBM 1) auf Basis des jeweiligen Subbildes (SUB) bestimmt,
**gekennzeichnet dadurch, dass** das CNN im Zuge der Bestimmung der jeweiligen individuellen Bindungsmaße für ein jeweiliges zweites Teilbild eine jeweilige finale Feature-Map (FFM1) generiert,
dass ferner auf Basis der finalen Feature-Map ein jeweiliges positives Konfidenzmaß bezogen auf ein Vorliegen der Bindung von Autoantikörpern in einem jeweiligen Kinetoplastbereich für ein jeweiliges zweites Teilbild bestimmt wird,
dass ferner eine Teilmenge der zweiten Teilbilder ausgewählt wird auf Basis der jeweiligen positiven Konfidenzmaße der jeweiligen zweiten Teilbilder,
und dass ferner ein Gesamtbindungsmaß (GBM) bezogen auf die Bindung von Autoantikörpern der Patientenprobe an doppelsträngige Desoxyribonukleinsäure auf Basis jener Bindungsmaße bestimmt wird, welche zu den ausgewählten zweiten Teilbildern der Teilmenge gehören.

2. Verfahren nach Anspruch 1,
aufweisend, für ein jeweiliges zweites Teilbild (ZTB) aus der ausgewählten Teilmenge,
- Selektieren eines jeweiligen Subbildes (SUB) des jeweiligen zweiten Teilbildes (ZTB) auf Basis einer zu dem jeweiligen zweiten Teilbild (ZTB) korrespondierenden jeweiligen finalen Feature Map (FFM1), wobei das jeweilige Subbild (SUB) einen jeweiligen Kinetoplastbereich (K) einer jeweiligen Crithidia luciliae Zelle (CR) repräsentiert,
- Bestimmen des jeweiligen Bindungsmaßes (IBM1) auf Basis des jeweiligen Subbildes (SUB),
sowie ferner aufweisend
- Bestimmen des Gesamtbindungsmaßes (GBM) auf Basis der jeweiligen Bindungsmaße (IBM1, IBM2).

3. Verfahren nach Anspruch 2,
aufweisend für ein jeweiliges zweites Teilbild (ZTB) aus der ausgewählten Teilmenge,
- Ermitteln eines jeweiligen Maskierungsoperators (BM) auf Basis der jeweiligen finalen Feature Map (FFM1),
- Selektieren des jeweiligen Subbildes (SUB) des jeweiligen zweiten Teilbildes (ZTB) mittels Anwendung des jeweiligen Maskierungsoperators (BM) auf das jeweilige zweite Teilbild (ZTB),
- Bestimmen des jeweiligen Bindungsmaßes (IBM1) auf Basis des jeweiligen Subbildes (SUB),
sowie aufweisend
- Bestimmen des Gesamtbindungsmaßes (GBM) auf Basis der jeweiligen Bindungsmaße (IBM1, IBM2).

4. Verfahren nach Anspruch 1,
wobei das Convolutional Neural Network (CNN) im Zuge einer Prozessierung eines zweiten Teilbildes (ZTB)
- in einer ersten Prozessierungsebene (P1) auf Basis des zweiten Teilbildes (ZTB) mittels wenigstens eines ersten Convolutional Layer (LA1) und mittels Anwendung mehrerer zweidimensionaler Faltungskernel eine erste Menge von resultierenden Feature Maps (RFM1) generiert,
- in einer zweiten Prozessierungsebene (P2)
∘ auf Basis der ersten Menge zweidimensionaler Feature Maps (RFM1) mittels wenigstens eines zweiten Convolutional Layer (LA2) und mittels Anwendung mehrerer dreidimensionaler Faltungskernel eine zweite Menge von resultierenden Feature Maps (RFM2) generiert,
∘ sowie ferner auf Basis der zweiten Menge zweidimensionaler Feature Maps (RFM2) mittels wenigstens eines dritten Convolutional Layer (LA3) und mittels Anwendung mehrerer dreidimensionaler Faltungskernel eine dritte Menge von resultierenden Feature Maps (RFM3) generiert,
wobei die zweite Menge (RFM2) eine geringere Anzahl resultierender Feature Maps aufweist als die erste Menge (RFM1) und wobei die dritte Menge (RFM3) eine größere Anzahl resultierender Feature Maps aufweist als die zweite Menge (RFM2).

5. Verfahren nach Anspruch 4,
wobei in der zweiten Prozessierungsebene (P2) das zweite Convolutional Layer (LA2) und das dritte Convolutional Layer (LA3) als Teilschritte eines sequentiellen Prozessierungspfades (PF1) aufeinander abfolgen,
wobei in der zweiten Prozessierungsebene (P2) ein weiterer Prozessierungspfad (PF2) parallel zu dem sequentiellen Prozessierungspfad (PF1) vorhanden ist, in welchem das Convolutional Neural Network (CNN) auf Basis der ersten Menge (RFM1) zweidimensionaler Feature Maps mittels wenigstens eines vierten Convolutional Layer (LA4) eine vierte Menge (RFM4) von resultierenden Feature Maps generiert, wobei das Convolutional Neural Network (CNN) die zu dem zweiten Teilbild (ZTB) korrespondierende finale Feature Map (FFM1) auf Basis der dritten (RFM3) und der vierten Menge (RFM4) von resultierenden Feature Maps generiert,
und wobei die Anzahl aufeinanderfolgender Convolution Layer in dem parallelen Prozessierungspfad (PF2) geringer ist als die Anzahl aufeinanderfolgender Convolution Layer in dem sequentiellen Prozessierungspfad (PF1).

6. Verfahren nach Anspruch 1,
aufweisend
- Erfassen eines ersten vorläufigen Fluoreszenzbildes (EVB1) in dem ersten Farbkanal unter Verwendung eines fest vorgegebenen Erfassungsparameters (GF),
- Bestimmen eines Helligkeitswertes (HW), welcher eine Helligkeit des ersten vorläufigen Fluoreszenzbildes des ersten Farbkanals (EVFB1) indiziert,
- Modifizieren des Erfassungsparameters in Abhängigkeit des bestimmten Helligkeitswertes (HW),
- Erfassen eines zweiten vorläufigen Fluoreszenzbildes (ZVFB1) in dem ersten Farbkanal unter Verwendung des modifizierten Erfassungsparameters (GF2),
- Verwenden des zweiten vorläufigen Fluoreszenzbildes des ersten Farbkanals (ZVFB1) als das erste Fluoreszenzbild (SR) des ersten Farbkanals.

7. Verfahren nach Anspruch 6,
aufweisend
- Erfassen eines ersten vorläufigen Fluoreszenzbildes (EVB1) in dem ersten Farbkanal unter Verwendung eines fest vorgegebenen Erfassungsparameters (GF),
- Bestimmen eines Helligkeitswerte (HW), welcher eine Helligkeit des ersten vorläufigen Fluoreszenzbildes des ersten Farbkanals (EVFB1) indiziert,
- Feststellen mittels des Helligkeitswertes (HW), ob eine Helligkeit des ersten vorläufigen Fluoreszenzbildes des ersten Farbkanals (EVFB1) einer erwarteten Helligkeit entspricht,
- in dem Fall, dass die Helligkeit des ersten vorläufigen Fluoreszenzbildes (EVFB1) des ersten Farbkanals der erwarteten Helligkeit entspricht, Verwenden des ersten vorläufigen Fluoreszenzbildes des ersten Farbkanals (EVFB1) als das erste Fluoreszenzbild (SR) des ersten Farbkanals,
- in dem Fall, dass die Helligkeit des ersten vorläufigen Fluoreszenzbildes des ersten Farbkanals (EVFB1) nicht der erwarteten Helligkeit entspricht,
∘ Modifizieren des Erfassungsparameters in Abhängigkeit des bestimmten Helligkeitswertes (HW),
∘ Erfassen eines zweiten vorläufigen Fluoreszenzbildes in dem ersten Farbkanal (ZVFB1) unter Verwendung des modifizierten Erfassungsparameters (GF2),
∘ Verwenden des zweiten vorläufigen Fluoreszenzbildes des ersten Farbkanals (ZFB1) als das erste Fluoreszenzbild (SR) des ersten Farbkanals.

8. Verfahren nach Anspruch 1,
aufweisend
- Erfassen eines ersten vorläufigen Fluoreszenzbildes (EVFB2) in dem zweiten Farbkanal unter Verwendung eines fest vorgegebenen Erfassungsparameters (EP1),
- Feststellen, ob eine Helligkeit des ersten vorläufigen Fluoreszenzbildes (EVFB2) des zweiten Farbkanals eine maximale Helligkeit übersteigt,
- in dem Fall, dass das erste vorläufige Fluoreszenzbild des zweiten Farbkanals (EVB2) die maximale Helligkeit nicht übersteigt, Verwenden des ersten vorläufigen Fluoreszenzbildes (EVFB2) als das zweite Fluoreszenzbild (SG) des zweiten Farbkanals,
- in dem Fall, dass das das erste vorläufige Fluoreszenzbild des zweiten Farbkanals (EVB2) die maximale Helligkeit übersteigt, Erfassen eines zweiten vorläufigen Fluoreszenzbildes in dem zweiten Farbkanal (ZVFB2) und Verwenden des zweiten vorläufigen Fluoreszenzbildes des zweiten Farbkanals (ZVFB2) als das zweite Fluoreszenzbild des zweiten Farbkanals (SG).

9. Vorrichtung (V1) zum Detektieren einer Bindung von Autoantikörpern einer Patientenprobe an doppelsträngige Desoxyribonukleinsäure unter Verwendung von Crithidia luciliae Zellen mittels Fluoreszenzmikroskopie und mittels digitaler Bildverarbeitung, aufweisend
- eine Haltevorrichtung (H) für ein Substrat (S), welches mehrere Crithidia luciliae Zellen (CR) aufweist und welches mit einer Patientenprobe aufweisend die Autoantikörper, mit einem ersten Fluoreszenzfarbstoff sowie ferner mit sekundären Antikörpern, welche jeweils mit einem zweiten Fluoreszenzfarbstoff markiert sind, inkubiert wurde, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wurde,
- wenigstens eine Bilderfassungseinheit (K1, K2) zum Erfassen eines ersten Fluoreszenzbildes (SR) des Substrates in einem ersten Farbkanal sowie ferner zum Erfassen eines zweiten Fluoreszenzbildes (SG) des Substrates (S) in einem zweiten Farbkanal,
sowie ferner aufweisend wenigstens eine Recheneinheit (R), welche ausgebildet ist,
- jeweilige erste Teilbilder (ZTB) in dem ersten Fluoreszenzbild (SR) zu identifizieren, welche jeweils wenigstens eine Crithidia Luciliae Zelle (CR) repräsentieren,
- jeweilige zweite Teilbilder (ZTB) des zweiten Fluoreszenzbildes (SG) zu bestimmen, welche zu den jeweiligen ersten Teilbildern (ETB) des ersten Fluoreszenzbildes (SR) korrespondieren,
wobei die Recheneinheit ferner ausgebildet ist,
- wenigstens eine Teilmenge der jeweiligen zweiten Teilbilder (ZTB) jeweils zu prozessieren mittels eines vortrainierten Convolutional Neural Network (CNN) zur Bestimmung jeweiliger Bindungsmaße (IBM1, IBM2), welche einen jeweiligen Grad einer Bindung von Autoantikörpern in einem jeweiligen Kinetoplastbereich (K) einer jeweiligen Crithidia luciliae Zelle (CR) eines jeweiligen zweiten Teilbildes (ZTB) indizieren
wobei das CNN ein jeweiliges zweites Teilbild (ZTB) für sich jeweils separat prozessiert um für ein jeweiliges zweites Teilbild (ZTB) ein jeweiliges Subbild zu bestimmen, das einen jeweiligen Kinetoplastbereich einer jeweiligen Crithidia luciliae Zelle (CR) repräsentiert,
und wobei das CNN das jeweilige Bindungsmaß (IBM1) auf Basis des jeweiligen Subbildes (SUB) bestimmt,
wobei die Recheneinheit **dadurch gekennzeichnet ist, dass** das CNN im Zuge der Bestimmung der jeweiligen individuellen Bindungsmaße für ein jeweiliges zweites Teilbild eine jeweilige finale Feature-Map (FFM1) generiert,
dass ferner die Recheneinheit auf Basis der finalen Feature-Map ein jeweiliges positives Konfidenzmaß bezogen auf ein Vorliegen einer Bindung von Autoantikörpern in einem jeweiligen Kinetoplastbereichen für ein jeweiliges zweites Teilbild bestimmt, dass ferner die Recheneinheit eine Teilmenge der zweiten Teilbilder auswählt auf Basis der jeweiligen positiven Konfidenzmaße der jeweiligen zweiten Teilbilder, und dass ferner die Recheneinheit ein Gesamtbindungsmaß (GBM) bezogen auf die Bindung von Autoantikörpern der Patientenprobe an doppelsträngige Desoxyribonukleinsäure auf Basis jener Bindungsmaße bestimmt, welche zu den ausgewählten zweiten Teilbildern der Teilmenge gehören.

10. Recheneinheit (R), welche ausgebildet ist im Zuge einer digitalen Bildverarbeitung
- ein erstes Fluoreszenzbild (SG) entgegenzunehmen, welches eine Färbung eines Substrates, welches wiederum mehrere Crithidia luciliae Zellen (CR) aufweist, durch einen ersten Fluoreszenzfarbstoff repräsentiert, sowie ein zweites Fluoreszenzbild (SG) entgegenzunehmen, welches eine Färbung des Substrates durch einen zweiten Fluoreszenzfarbstoff repräsentiert, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wurde,
- jeweilige erste Teilbilder (ETB) in dem ersten Fluoreszenzbild (SR) zu identifizieren, welche jeweils wenigstens eine Crithidia Luciliae Zelle (CR) repräsentieren,
- jeweilige zweite Teilbilder (ZTB) des zweiten Fluoreszenzbildes (SG) zu bestimmen, welche zu den jeweiligen ersten Teilbildern (ETB) des ersten Fluoreszenzbildes (SR) korrespondieren,
wobei die Recheneinheit ferner ausgebildet ist,
- wenigstens eine Teilmenge der jeweiligen zweiten Teilbilder (ZTB) jeweils zu prozessieren mittels eines vortrainierten Convolutional Neural Network (CNN) zur Bestimmung jeweiliger Bindungsmaße (IBM1, IBM2), welche einen jeweiligen Grad einer Bindung von Autoantikörpern in einem jeweiligen Kinetoplastbereich (K) einer jeweiligen Crithidia luciliae Zelle (CR) eines jeweiligen zweiten Teilbildes (ZTB) indizieren
wobei das CNN ein jeweiliges zweites Teilbild (ZTB) für sich jeweils separat prozessiert um für ein jeweiliges zweites Teilbild (ZTB) ein jeweiliges Subbild zu bestimmen, das einen jeweiligen Kinetoplastbereich einer jeweiligen Crithidia luciliae Zelle (CR) repräsentiert,
und wobei das CNN das jeweilige Bindungsmaß (IBM1) auf Basis des jeweiligen Subbildes (SUB) bestimmt,
wobei die Recheneinheit **dadurch gekennzeichnet ist, dass** das CNN im Zuge der Bestimmung der jeweiligen individuellen Bindungsmaße für ein jeweiliges zweites Teilbild eine jeweilige finale Feature-Map (FFM1) generiert,
dass ferner die Recheneinheit auf Basis der finalen Feature-Map ein jeweiliges positives Konfidenzmaß bezogen auf ein Vorliegen einer Bindung von Autoantikörpern in einem jeweiligen Kinetoplastbereich für ein jeweiliges zweites Teilbild bestimmt, dass ferner die Recheneinheit eine Teilmenge der zweiten Teilbilder auswählt auf Basis der jeweiligen positiven Konfidenzmaße der jeweiligen zweiten Teilbilder,
und dass ferner die Recheneinheit ein Gesamtbindungsmaß (GBM) bezogen auf die Bindung von Autoantikörpern der Patientenprobe an doppelsträngige Desoxyribonukleinsäure auf Basis jener Bindungsmaße bestimmt, welche zu den ausgewählten zweiten Teilbildern der Teilmenge gehören.

11. Datennetzwerkvorrichtung (DV), aufweisend
wenigstens eine Datenschnittstelle (DS4) zum Entgegennehmen eines ersten Fluoreszenzbildes (BI1, SR), welches eine Färbung eines Substrates, welches wiederum mehrere Crithidia luciliae Zellen aufweist, durch einen ersten Fluoreszenzfarbstoff repräsentiert sowie ferner eines zweiten Fluoreszenzbildes (BI2, SG), welches eine Färbung des Substrates durch einen zweiten Fluoreszenzfarbstoff repräsentiert, wobei der zweite Fluoreszenzfarbstoff eine Färbung in jenen Bereichen bewirkt, in welchen die Autoantikörper der Patientenprobe an die dsDNS in dem jeweiligen Kinetoplastbereich der jeweiligen Crithidia luciliae gebunden wurde,
sowie ferner wenigstens eine Recheneinheit (R), welche ausgebildet ist im Zuge einer digitalen Bildverarbeitung
- jeweilige erste Teilbilder (ETB) in dem ersten Fluoreszenzbild (SR) zu identifizieren, welche jeweils wenigstens eine Crithidia Luciliae Zelle (CR) aufweisen,
- jeweilige zweite Teilbilder (ZTB) des zweiten Fluoreszenzbildes (SG) zu bestimmen, welche zu den jeweiligen ersten Teilbildern (ETB) des ersten Fluoreszenzbildes (SR) korrespondieren,
- wobei die Recheneinheit ferner ausgebildet ist,
- wenigstens eine Teilmenge der jeweiligen zweiten Teilbilder (ZTB) jeweils zu prozessieren mittels eines vortrainierten Convolutional Neural Network (CNN) zur Bestimmung jeweiliger Bindungsmaße (IBM1, IBM2), welche einen jeweiligen Grad einer Bindung von Autoantikörpern in einem jeweiligen Kinetoplastbereich (K) einer jeweiligen Crithidia luciliae Zelle (CR) eines jeweiligen zweiten Teilbildes (ZTB) indizieren,
- wobei das CNN ein jeweiliges zweites Teilbild (ZTB) für sich jeweils separat prozessiert um für ein jeweiliges zweites Teilbild (ZTB) ein jeweiliges Subbild zu bestimmen, das einen jeweiligen Kinetoplastbereich einer jeweiligen Crithidia luciliae Zelle (CR) repräsentiert,
- und wobei das CNN das jeweilige Bindungsmaß (IBM1) auf Basis des jeweiligen Subbildes (SUB) bestimmt,
wobei die Recheneinheit **dadurch gekennzeichnet ist, dass** das CNN im Zuge der Bestimmung der jeweiligen individuellen Bindungsmaße für ein jeweiliges zweites Teilbild eine jeweilige finale Feature-Map (FFM1) generiert,
dass ferner die Recheneinheit auf Basis der finalen Feature-Map ein jeweiliges positives Konfidenzmaß bezogen auf ein Vorliegen einer Bindung von Autoantikörpern in einem jeweiligen Kinetoplastbereich für ein jeweiliges zweites Teilbild bestimmt,
dass ferner die Recheneinheit eine Teilmenge der zweiten Teilbilder auswählt auf Basis der jeweiligen positiven Konfidenzmaße der jeweiligen zweiten Teilbilder, und dass ferner die Recheneinheit ein Gesamtbindungsmaß (GBM) bezogen auf die Bindung von Autoantikörpern der Patientenprobe an doppelsträngige Desoxyribonukleinsäure auf Basis jener Bindungsmaße bestimmt, welche zu den ausgewählten zweiten Teilbildern der Teilmenge gehören.

12. Verfahren zur digitalen Bildverarbeitung, aufweisend
- Entgegennehmen eines ersten Fluoreszenzbildes (SR), welches eine Färbung eines Substrates (S), welches wiederum mehrere Crithidia luciliae Zellen (CR) aufweist, durch einen ersten Fluoreszenzfarbstoff repräsentiert sowie ferner eines zweiten Fluoreszenzbildes (SG), welches eine Färbung des Substrates (S) durch einen zweiten Fluoreszenzfarbstoff repräsentiert, wobei der zweite Fluoreszenzfarbstoff eine Färbung in jenen Bereichen bewirkt, in welchen die Autoantikörper der Patientenprobe an die dsDNS in den jeweiligen Kinetoplastbereich der jeweiligen Crithidia luciliae gebunden wurde,
- Identifizieren jeweiliger erster Teilbilder (ETB) in dem ersten Fluoreszenzbild (SR), welche jeweils eine Crithidia Luciliae Zelle (CR) repräsentiert,
- Bestimmen jeweiliger zweiter Teilbilder (ZTB) des zweiten Fluoreszenzbildes (SG), welche zu den jeweiligen ersten Teilbildern (ETB) des ersten Fluoreszenzbildes (SR) korrespondieren,
- ferner aufweisend
- jeweiliges Prozessieren wenigstens einer Teilmenge der jeweiligen zweiten Teilbilder (ZTB) mittels eines vortrainierten Convolutional Neural Network (CNN) zur Bestimmung jeweiliger Bindungsmaße (IBM 1, IBM2), welche einen jeweiligen Grad einer Bindung von Autoantikörpern in einem jeweiligen Kinetoplastbereich (K) einer jeweiligen Crithidia luciliae Zelle (CR) eines jeweiligen zweiten Teilbildes (ZTB) indizieren,
- wobei das CNN ein jeweiliges zweites Teilbild (ZTB) für sich jeweils separat prozessiert um für ein jeweiliges zweites Teilbild (ZTB) ein jeweiliges Subbild zu bestimmen, das einen jeweiligen Kinetoplastbereich einer jeweiligen Crithidia luciliae Zelle (CR) repräsentiert,
- und wobei das CNN das jeweilige Bindungsmaß (IBM1) auf Basis des jeweiligen Subbildes (SUB) bestimmt,
- sowie ferner Bestimmen eines Gesamtbindungsmaß (GBM) einer Bindung von Autoantikörpern der Patientenprobe an doppelsträngige Desoxyribonukleinsäure auf Basis der jeweiligen Bindungsmaße (IBM1, IBM2).
**gekennzeichnet dadurch, dass** das CNN im Zuge der Bestimmung der jeweiligen individuellen Bindungsmaße für ein jeweiliges zweites Teilbild eine jeweilige finale Feature-Map (FFM1) generiert,
dass ferner auf Basis der finalen Feature-Map ein jeweiliges positives Konfidenzmaß bezogen auf ein Vorliegen einer Bindung von Autoantikörpern in einem jeweiligen Kinetoplastbereich für ein jeweiliges zweites Teilbild bestimmt wird, dass ferner eine Teilmenge der zweiten Teilbilder ausgewählt wird auf Basis der jeweiligen positiven Konfidenzmaße der jeweiligen zweiten Teilbilder,
und dass ferner ein Gesamtbindungsmaß (GBM) bezogen auf die Bindung von Autoantikörpern der Patientenprobe an doppelsträngige Desoxyribonukleinsäure auf Basis jener Bindungsmaße bestimmt wird, welche zu den ausgewählten zweiten Teilbildern der Teilmenge gehören.

13. Computerprogrammprodukt (CPP),
umfassend Befehle, die bei der Ausführung des Programms durch einen Computer diesen veranlassen, das Verfahren zur digitalen Bildverarbeitung nach Anspruch 12 durchzuführen.

## Claims

1. Method for detecting a binding of autoantibodies from a patient sample to double-stranded deoxyribonucleic acid using Crithidia luciliae cells by means of fluorescence microscopy and by means of digital image processing,
comprising
- provision of a substrate (S) which has multiple Crithidia luciliae cells (CR),
- incubation of the substrate (S) with the patient sample which potentially has the autoantibodies,
- incubation of the substrate (S) with a first fluorescent dye,
- incubation of the substrate (S) with secondary antibodies which have each been labelled with a second fluorescent dye, wherein the second fluorescent dye causes a staining in those regions in which the autoantibodies from the patient sample are bound to the dsDNS in the respective kinetoplast region of the respective Crithidia luciliae,
- acquisition of a first fluorescence image (SR) of the substrate (S) in a first colour channel which corresponds to the first fluorescent dye,
- acquisition of a second fluorescence image (SG) of the substrate (S) in a second colour channel which corresponds to the second fluorescent dye,
- identification of respective first sub-images (ETB) in the first fluorescence image (SR) that each represent a respective Crithidia luciliae cell (CR),
- determination of respective second sub-images (ZTB) of the second fluorescence image (SG) that correspond to the respective first sub-images (ETB) of the first fluorescence image (SR),
- respective processing of at least one subset of the respective second sub-images (ZTB) by means of a pretrained convolutional neural network (CNN) for determining respective binding measures (IBM1, IBM2) which indicate a respective extent of a binding of autoantibodies in a respective kinetoplast region (K) of a respective Crithidia luciliae cell (CR) of a respective second sub-image (ZTB),
wherein the CNN processes a respective second sub-image (ZTB) each separately in order to determine for a respective second sub-image (ZTB) a respective subordinate image representing a kinetoplast region of a respective Chritidia luciliae cell (CR),
and wherein the CNN determines the respective binding measure (IBM1) on the basis of the respective subordinate image (SUB),
**characterized in that** the CNN generates, in the course of the determination of the respective individual binding measures, a respective final feature map (FFM1) for a respective second sub-image,
that, furthermore, on the basis of the final feature map, a respective positive confidence measure with regard to a presence of the binding of autoantibodies in a respective kinetoplast region is determined for a respective second sub-image, that, furthermore, a subset of the second sub-images is selected on the basis of the respective positive confidence measures of the respective second sub-images, and that, furthermore, an overall binding measure (GBM) with regard to the binding of autoantibodies from the patient sample to double-stranded deoxyribonucleic acid is determined on the basis of those binding measures which belong to the selected second sub-images of the subset.

2. Method according to Claim 1,
comprising, for a respective second sub-image (ZTB) from the selected subset,
- selection of a respective subordinate image (SUB) of the respective second sub-image (ZTB) on the basis of a respective final feature map (FFM1) corresponding to the respective second sub-image (ZTB), the respective subordinate image (SUB) representing a respective kinetoplast region (K) of a respective Crithidia luciliae cell (CR),
- determination of the respective binding measure (IBM1) on the basis of the respective subordinate image (SUB),
and comprising furthermore
- determination of the overall binding measure (GBM) on the basis of the respective binding measures (IBM1, IBM2).

3. Method according to Claim 2,
comprising, for a respective second sub-image (ZTB) from the selected subset,
- ascertainment of a respective masking operator (BM) on the basis of the respective final feature map (FFM1),
- selection of the respective subordinate image (SUB) of the respective second sub-image (ZTB) by means of application of the respective masking operator (BM) to the respective second sub-image (ZTB),
- determination of the respective binding measure (IBM1) on the basis of the respective subordinate image (SUB),
and comprising
- determination of the overall binding measure (GBM) on the basis of the respective binding measures (IBM1, IBM2).

4. Method according to Claim 1,
wherein, in the course of a processing of a second sub-image (ZTB), the convolutional neural network (CNN),
- in a first processing level (P1), generates a first set of resultant feature maps (RFM1) on the basis of the second sub-image (ZTB) by means of at least one first convolutional layer (LA1) and by means of application of multiple two-dimensional convolution kernels,
- in a second processing level (P2),
∘ generates a second set of resultant feature maps (RFM2) on the basis of the first set of two-dimensional feature maps (RFM1) by means of at least one second convolutional layer (LA2) and by means of application of multiple three-dimensional convolution kernels,
∘ and furthermore generates a third set of resultant feature maps (RFM3) on the basis of the second set of two-dimensional feature maps (RFM2) by means of at least one third convolutional layer (LA3) and by means of application of multiple three-dimensional convolution kernels,
wherein the second set (RFM2) has a smaller number of resultant feature maps than the first set (RFM1) and wherein the third set (RFM3) has a larger number of resultant feature maps than the second set (RFM2).

5. Method according to Claim 4,
wherein, in the second processing level (P2), the second convolutional layer (LA2) and the third convolutional layer (LA3) are in a sequence as sub-steps of a sequential processing path (PF1),
wherein, in the second processing level (P2), there is in parallel to the sequential processing path (PF1) a further processing path (PF2) in which the convolutional neural network (CNN) generates a fourth set (RFM4) of resultant feature maps on the basis of the first set (RFM1) of two-dimensional feature maps by means of at least one fourth convolutional layer (LA4),
wherein the convolutional neural network (CNN) generates on the basis of the third (RFM3) and the fourth set (RFM4) of resultant feature maps the final feature map (FFM1) corresponding to the second sub-image (ZTB),
and wherein the number of successive convolution layers in the parallel processing path (PF2) is smaller than the number of successive convolution layers in the sequential processing path (PF1).

6. Method according to Claim 1,
comprising
- acquisition of a first preliminary fluorescence image (EVB1) in the first colour channel using a predefined acquisition parameter (GF),
- determination of a brightness value (HW) indicating a brightness of the first preliminary fluorescence image of the first colour channel (EVFB1),
- modification of the acquisition parameter depending on the determined brightness value (HW),
- acquisition of a second preliminary fluorescence image (ZVFB1) in the first colour channel using the modified acquisition parameter (GF2),
- use of the second preliminary fluorescence image of the first colour channel (ZVFB1) as the first fluorescence image (SR) of the first colour channel.

7. Method according to Claim 6,
comprising
- acquisition of a first preliminary fluorescence image (EVB1) in the first colour channel using a predefined acquisition parameter (GF),
- determination of a brightness value (HW) indicating a brightness of the first preliminary fluorescence image of the first colour channel (EVFB1),
- establishment by means of the brightness value (HW) as to whether a brightness of the first preliminary fluorescence image of the first colour channel (EVFB1) corresponds to an expected brightness,
- in the event of the brightness of the first preliminary fluorescence image (EVFB1) of the first colour channel corresponding to the expected brightness, use of the first preliminary fluorescence image of the first colour channel (EVFB1) as the first fluorescence image (SR) of the first colour channel,
- in the event of the brightness of the first preliminary fluorescence image of the first colour channel (EVFB1) not corresponding to the expected brightness,
∘ modification of the acquisition parameter depending on the determined brightness value (HW),
∘ acquisition of a second preliminary fluorescence image in the first colour channel (ZVFB1) using the modified acquisition parameter (GF2),
∘ use of the second preliminary fluorescence image of the first colour channel (ZFB1) as the first fluorescence image (SR) of the first colour channel.

8. Method according to Claim 1,
comprising
- acquisition of a first preliminary fluorescence image (EVFB2) in the second colour channel using a predefined acquisition parameter (EP1),
- establishment of whether a brightness of the first preliminary fluorescence image (EVFB2) of the second colour channel exceeds a maximum brightness,
- in the event of the first preliminary fluorescence image of the second colour channel (EVFB2) not exceeding the maximum brightness, use of the first preliminary fluorescence image (EVFB2) as the second fluorescence image (SG) of the second colour channel,
- in the event of the first preliminary fluorescence image of the second colour channel (EVFB2) exceeding the maximum brightness, acquisition of a second preliminary fluorescence image in the second colour channel (ZVFB2) and use of the second preliminary fluorescence image of the second colour channel (ZVFB2) as the second fluorescence image of the second colour channel (SG).

9. Device (V1) for detecting a binding of autoantibodies from a patient sample to double-stranded deoxyribonucleic acid using Crithidia luciliae cells by means of fluorescence microscopy and by means of digital image processing, comprising
- a mounting device (H) for a substrate (S) which has multiple Crithidia luciliae cells (CR) and which has been incubated with a patient sample having the autoantibodies, with a first fluorescent dye and, furthermore, with secondary antibodies which have each been labelled with a second fluorescent dye, wherein the second fluorescent dye causes a staining in those regions in which the autoantibodies from the patient sample are bound to the dsDNS in the respective kinetoplast region of the respective Crithidia luciliae,
- at least one image acquisition unit (K1, K2) for acquiring a first fluorescence image (SR) of the substrate in a first colour channel and, furthermore, for acquiring a second fluorescence image (SG) of the substrate (S) in a second colour channel,
and comprising furthermore at least one computing unit (R) which is designed
- to identify respective first sub-images (ZTB) in the first fluorescence image (SR) that each represent at least one Crithidia luciliae cell (CR),
- to determine respective second sub-images (ZTB) of the second fluorescence image (SG) that correspond to the respective first sub-images (ETB) of the first fluorescence image (SR),
wherein the computing unit is furthermore designed
- to respectively process at least one subset of the respective second sub-images (ZTB) by means of a pretrained convolutional neural network (CNN) for determining respective binding measures (IBM1, IBM2) which indicate a respective extent of a binding of autoantibodies in a respective kinetoplast region (K) of a respective Crithidia luciliae cell (CR) of a respective second sub-image (ZTB)
wherein the CNN processes a respective second sub-image (ZTB) each separately in order to determine for a respective second sub-image (ZTB) a respective subordinate image representing the kinetoplast region of a respective Chritidia luciliae cell (CR), and wherein the CNN determines the respective binding measure (IBM1) on the basis of the respective subordinate image (SUB),
wherein the computing unit is **characterized in that** the CNN, in the course of the determination of the respective individual binding measures, generates a respective final feature map (FFM1) for a respective second sub-image,
that, furthermore, the computing unit determines on the basis of the final feature map a respective positive confidence measure with regard to a presence of a binding of autoantibodies in a respective kinetoplast region for a respective second sub-image, that, furthermore, the computing unit selects a subset of the second sub-images on the basis of the respective positive confidence measures of the respective second sub-images,
and that, furthermore, the computing unit determines an overall binding measure (GBM) with regard to the binding of autoantibodies from the patient sample to double-stranded deoxyribonucleic acid on the basis of those binding measures which belong to the selected second sub-images of the subset.

10. Computing unit (R) which is designed, in the course of a digital image processing,
- to receive a first fluorescence image (SG) which represents a staining of a substrate, which in turn has multiple Crithidia luciliae cells (CR), by a first fluorescent dye and to receive a second fluorescence image (SG) which represents a staining of the substrate by a second fluorescent dye, wherein the second fluorescent dye causes a staining in those regions in which the autoantibodies from the patient sample are bound to the dsDNS in the respective kinetoplast region of the respective Crithidia luciliae,
- to identify respective first sub-images (ETB) in the first fluorescence image (SR) that each represent at least one Crithidia luciliae cell (CR),
- to determine respective second sub-images (ZTB) of the second fluorescence image (SG) that correspond to the respective first sub-images (ETB) of the first fluorescence image (SR),
wherein the computing unit is furthermore designed
- to respectively process at least one subset of the respective second sub-images (ZTB) by means of a pretrained convolutional neural network (CNN) for determining respective binding measures (IBM1, IBM2) which indicate a respective extent of a binding of autoantibodies in a respective kinetoplast region (K) of a respective Crithidia luciliae cell (CR) of a respective second sub-image (ZTB)
wherein the CNN processes a respective second sub-image (ZTB) each separately in order to determine for a respective second sub-image (ZTB) a respective subordinate image representing the kinetoplast region of a respective Chritidia luciliae cell (CR), and wherein the CNN determines the respective binding measure (IBM1) on the basis of the respective subordinate image (SUB),
wherein the computing unit is **characterized in that** the CNN, in the course of the determination of the respective individual binding measures, generates a respective final feature map (FFM1) for a respective second sub-image,
that, furthermore, the computing unit determines on the basis of the final feature map a respective positive confidence measure with regard to a presence of a binding of autoantibodies in a respective kinetoplast region for a respective second sub-image, that, furthermore, the computing unit selects a subset of the second sub-images on the basis of the respective positive confidence measures of the respective second sub-images,
and that, furthermore, the computing unit determines an overall binding measure (GBM) with regard to the binding of autoantibodies from the patient sample to double-stranded deoxyribonucleic acid on the basis of those binding measures which belong to the selected second sub-images of the subset.

11. Data network device (DV) comprising
at least one data interface (DS4) for receiving a first fluorescence image (BI1, SR) which represents a staining of a substrate, which in turn has multiple Crithidia luciliae cells, by a first fluorescent dye and, furthermore, of a second fluorescence image (BI2, SG) which represents a staining of the substrate by a second fluorescent dye, wherein the second fluorescent dye causes a staining in those regions in which the autoantibodies from the patient sample are bound to the dsDNS in the respective kinetoplast region of the respective Crithidia luciliae,
and furthermore at least one computing unit (R) which is designed, in the course of a digital image processing,
- to identify respective first sub-images (ETB) in the first fluorescence image (SR) that each have at least one Crithidia luciliae cell (CR),
- to determine respective second sub-images (ZTB) of the second fluorescence image (SG) that correspond to the respective first sub-images (ETB) of the first fluorescence image (SR),
- wherein the computing unit is furthermore designed
- to respectively process at least one subset of the respective second sub-images (ZTB) by means of a pretrained convolutional neural network (CNN) for determining respective binding measures (IBM1, IBM2) which indicate a respective extent of a binding of autoantibodies in a respective kinetoplast region (K) of a respective Crithidia luciliae cell (CR) of a respective second sub-image (ZTB)
- wherein the CNN processes a respective second sub-image (ZTB) each separately in order to determine for a respective second sub-image (ZTB) a respective subordinate image representing the kinetoplast region of a respective Chritidia luciliae cell (CR),
- and wherein the CNN determines the respective binding measure (IBM1) on the basis of the respective subordinate image (SUB)
wherein the computing unit is **characterized in that** the CNN, in the course of the determination of the respective individual binding measures, generates a respective final feature map (FFM1) for a respective second sub-image, that, furthermore, the computing unit determines on the basis of the final feature map a respective positive confidence measure with regard to a presence of a binding of autoantibodies in a respective kinetoplast region for a respective second sub-image,
that, furthermore, the computing unit selects a subset of the second sub-images on the basis of the respective positive confidence measures of the respective second sub-images,
and that, furthermore, the computing unit determines an overall binding measure (GBM) with regard to the binding of autoantibodies from the patient sample to double-stranded deoxyribonucleic acid on the basis of those binding measures which belong to the selected second sub-images of the subset.

12. Method for digital image processing, comprising
- receiving of a first fluorescence image (SR) which represents a staining of a substrate (S), which in turn has multiple Crithidia luciliae cells (CR), by a first fluorescent dye and, furthermore, of a second fluorescent image (SG) which represents a staining of the substrate (S) by a second fluorescent dye, wherein the second fluorescent dye causes a staining in those regions in which the autoantibodies from the patient sample are bound to the dsDNS in the respective kinetoplast region of the respective Crithidia luciliae
- identification of respective first sub-images (ETB) in the first fluorescence image (SR) that each represent a Crithidia luciliae cell (CR),
- determination of respective second sub-images (ZTB) of the second fluorescence image (SG) that correspond to the respective first sub-images (ETB) of the first fluorescence image (SR),
- further comprising
- respective processing of at least one subset of the respective second sub-images (ZTB) by means of a pretrained convolutional neural network (CNN) for determining respective binding measures (IBM1, IBM2) which indicate a respective extent of a binding of autoantibodies in a respective kinetoplast region (K) of a respective Crithidia luciliae cell (CR) of a respective second sub-image (ZTB),
- wherein the CNN processes a respective second sub-image (ZTB) each separately in order to determine for a respective second sub-image (ZTB) a respective subordinate image representing the kinetoplast region of a respective Chritidia luciliae cell (CR),
- and wherein the CNN determines the respective binding measure (IBM1) on the basis of the respective subordinate image (SUB)
- and, furthermore, determination of an overall binding measure (GBM) of a binding of autoantibodies from the patient sample to double-stranded deoxyribonucleic acid on the basis of the respective binding measures (IBM1, IBM2),
**characterized in that** the CNN, in the course of the determination of the respective individual binding measures, generates a respective final feature map (FFM1) for a respective second sub-image,
that, furthermore, on the basis of the final feature map a respective positive confidence measure with regard to a presence of a binding of autoantibodies in a respective kinetoplast region is determined for a respective second sub-image, that, furthermore, a subset of the second sub-images is selected on the basis of the respective positive confidence measures of the respective second sub-images,
and that, furthermore, an overall binding measure (GBM) with regard to the binding of autoantibodies from the patient sample to double-stranded deoxyribonucleic acid is determined on the basis of those binding measures which belong to the selected second sub-images of the subset.

13. Computer program product (CPP)
comprising commands which, upon the execution of the program by a computer, prompt said computer to carry out the method for digital image processing according to Claim 12.

## Revendications

1. Procédé destiné à détecter une liaison d'auto-anticorps d'un échantillon de patient à de l'acide désoxyribonucléique double brin à l'aide de cellules de Crithidia luciliae par microscopie de fluorescence et traitement d'image numérique,
présentant
- la fourniture d'un substrat (S), qui présente plusieurs cellules de Crithidia luciliae (CR),
- l'incubation du substrat (S) avec l'échantillon de patient qui présente potentiellement les auto-anticorps,
- l'incubation du substrat (S) avec un premier colorant fluorescent,
- l'incubation du substrat (S) avec des anticorps secondaires, qui sont respectivement marqués par un deuxième colorant fluorescent, le deuxième colorant fluorescent provoquant une coloration dans les zones dans lesquelles les auto-anticorps de l'échantillon de patient sont liés à l'ADNdb dans la zone de kinétoplaste respective de Crithidia luciliae respective,
- la détection d'une première image de fluorescence (SR) du substrat (S) dans un premier canal de couleur qui correspond au premier colorant fluorescent,
- la détection d'une deuxième image de fluorescence (SG) du substrat (S) dans un deuxième canal de couleur qui correspond au deuxième colorant fluorescent,
- l'identification de premières images partielles (ETB) respectives dans la première image de fluorescence (SR), qui représentent respectivement une cellule de Crithidia Luciliae (CR),
- la détermination de deuxièmes images partielles (ZTB) respectives de la deuxième image de fluorescence (SG), qui correspondent aux premières images partielles (ETB) respectives de la première image de fluorescence (SR),
- le traitement respectif d'au moins une quantité partielle des deuxièmes images partielles (ZTB) respectives au moyen d'un réseau neuronal convolutif (CNN) préentraîné pour déterminer un taux de liaison (IBM1, IBM2) respectif, qui indique un degré respectif d'une liaison d'auto-anticorps dans une zone de kinétoplaste (K) respective d'une cellule de Crithidia luciliae (CR) respective d'une deuxième image partielle (ZTB) respective,
le CNN traitant respectivement séparément, en soi, une deuxième image partielle (ZTB) respective pour déterminer, pour une deuxième image partielle (ZTB) respective, une sous-image respective qui représente une zone de kinétoplaste respective d'une cellule de Crithidia luciliae (CR) respective
et le CNN déterminant le taux de liaison (IBM1) respectif sur la base de la sous-image (SUB) respective,
**caractérisé en ce que** le CNN génère, au cours de la détermination du taux de liaison individuel respectif, pour une deuxième image partielle respective, une Feature-Map (FFM1) respective,
**en ce qu'**en outre, sur la base de la Feature-Map finale, un indice de confiance positif respectif concernant la présence de la liaison d'auto-anticorps dans une zone de kinétoplaste respective pour une deuxième image partielle respective est déterminé, **en ce qu'**en outre une quantité partielle des deuxièmes images partielles est choisie sur la base de l'indice de confiance positif respectif des deuxièmes images partielles respectives
et **en ce qu'**en outre un taux de liaison total (GBM) concernant la liaison d'auto-anticorps de l'échantillon de patient à l'acide désoxyribonucléique double brin est déterminé sur la base des différents taux de liaison, qui sont associés aux deuxièmes images partielles choisies de la quantité partielle.

2. Procédé selon la revendication 1,
présentant, pour une deuxième image partielle (ZTB) respective de la quantité partielle choisie,
- la sélection d'une sous-image (SUB) respective de la deuxième image partielle (ZTB) respective sur la base d'une Feature-Map finale (FFM1) respective correspondant à la deuxième image partielle (ZTB) respective, la sous-image (SUB) respective représentant une zone de kinétoplaste (K) respective d'une cellule de Crithidia luciliae (CR) respective,
- la détermination du taux de liaison (IBM1) respectif sur la base de la sous-image (SUB) respective
et présentant en outre
- la détermination du taux de liaison total (GBM) sur la base du taux de liaison (IBM1, IBM2) respectif.

3. Procédé selon la revendication 2,
présentant, pour une deuxième image partielle (ZTB) respective de la quantité partielle choisie,
- la détermination d'un opérateur de masquage (BM) respectif sur la base de la Feature-Map finale (FFM1) respective,
- la sélection de la sous-image (SUB) respective de la deuxième image partielle (ZTB) respective par application de l'opérateur de masquage (BM) respectif sur la deuxième image partielle (ZTB) respective,
- la détermination du taux de liaison (IBM1) respectif sur la base de la sous-image (SUB) respective
et présentant
- la détermination du taux de liaison total (GBM) sur la base du taux de liaison (IBM1, IBM2) respectif.

4. Procédé selon la revendication 1,
le réseau neuronal convolutif (CNN) générant, au cours d'un traitement d'une deuxième image partielle (ZTB)
- dans un premier plan de traitement (P1) sur la base de la deuxième image partielle (ZTB) au moyen d'au moins une première couche de convolution (LA1) et par application de plusieurs noyaux de convolution bidimensionnels, une première quantité de Feature-Maps (RFM1) résultantes,
- dans un deuxième plan de traitement (P2)
∘ sur la base de la première quantité de Feature-Maps bidimensionnelles (RFM1) au moyen d'au moins une deuxième couche de convolution (LA2) et par application de plusieurs noyaux de convolution tridimensionnels, une deuxième quantité de Feature-Maps (RFM2) résultantes,
∘ ainsi qu'en outre, sur la base de la deuxième quantité de Feature-Maps bidimensionnelles (RFM2) au moyen d'au moins une troisième couche de convolution (LA3) et par application de plusieurs noyaux de convolution tridimensionnels, une troisième quantité de Feature-Maps (RFM3) résultantes,
la deuxième quantité (RFM2) présentant un nombre de Feature-Maps résultantes inférieur à celui de la première quantité (RFM1) et la troisième quantité (RFM3) présentant un nombre de Feature-Maps résultantes supérieur à celui de la deuxième quantité (RFM2).

5. Procédé selon la revendication 4,
la deuxième couche de convolution (LA2) et la troisième couche de convolution (LA3) dans le deuxième plan de traitement (P2) se succédant en tant qu'étapes partielles d'une voie de traitement séquentielle (PF1),
une autre voie de traitement (PF2) parallèle à la voie de traitement séquentielle (PF1) étant présente dans le deuxième plan de traitement (P2), dans laquelle autre voie parallèle le réseau neuronal convolutif (CNN) génère, sur base de la première quantité (RFM1) de Feature-Maps bidimensionnelles au moyen d'au moins une quatrième couche de convolution (LA4) une quatrième quantité (RFM4) de Feature-Maps résultantes,
le réseau neuronal convolutif (CNN) générant la Feature-Map finale (FFM1) correspondant à la deuxième image partielle (ZTB) sur la base de la troisième (RFM3) et de la quatrième quantité (RFM4) de Feature-Maps résultantes et le nombre de couches de convolution successives dans la voie de traitement parallèle (PF2) étant inférieur au nombre de couches de convolution successives dans la voie de traitement séquentielle (PF1).

6. Procédé selon la revendication 1,
présentant
- la détection d'une première image de fluorescence temporaire (EVB1) dans le premier canal de couleur à l'aide d'un paramètre de détection (GF) prédéterminé,
- la détermination d'une valeur de luminosité (HW), qui indique une luminosité de la première image de fluorescence temporaire du premier canal de couleur (EVFB1),
- la modification du paramètre de détection en fonction de la valeur de luminosité (HW) déterminée,
- la détection d'une deuxième image de fluorescence temporaire (ZVFB1) dans le premier canal de couleur à l'aide du paramètre de détection (GF2) modifié,
- l'utilisation de la deuxième image de fluorescence temporaire du premier canal de couleur (ZVFB1) en tant que première image de fluorescence (SR) du premier canal de couleur.

7. Procédé selon la revendication 6,
présentant
- la détection d'une première image de fluorescence temporaire (EVB1) dans le premier canal de couleur à l'aide d'un paramètre de détection (GF) prédéterminé,
- la détermination d'une valeur de luminosité (HW), qui indique une luminosité de la première image de fluorescence temporaire du premier canal de couleur (EVFB1),
- au moyen de la valeur de luminosité (HW), la constatation du fait qu'une luminosité de la première image de fluorescence temporaire du premier canal de couleur (EVFB1) correspond ou non à une luminosité attendue,
- dans le cas où la luminosité de la première image de fluorescence temporaire (EVFB1) du premier canal de couleur correspond à la luminosité attendue, l'utilisation de la première image de fluorescence temporaire du premier canal de couleur (EVFB1) en tant que première image de fluorescence (SR) du premier canal de couleur,
- dans le cas où la luminosité de la première image de fluorescence temporaire du premier canal de couleur (EVFB1) ne correspond pas à la luminosité attendue,
∘ la modification du paramètre de détection en fonction de la valeur de luminosité (HW) déterminée,
∘ la détection d'une deuxième image de fluorescence temporaire dans le premier canal de couleur (ZVFB1) à l'aide du paramètre de détection (GF2) modifié,
∘ l'utilisation de la deuxième image de fluorescence temporaire du premier canal de couleur (ZFB1) en tant que première image de fluorescence (SR) du premier canal de couleur.

8. Procédé selon la revendication 1,
comprenant
- la détection d'une première image de fluorescence temporaire (EVFB2) dans le deuxième canal de couleur à l'aide d'un paramètre de détection (EP1) prédéterminé,
- la constatation du fait qu'une luminosité de la première image de fluorescence temporaire (EVFB2) du deuxième canal de couleur est supérieure ou non à une luminosité maximale,
- dans le cas où la luminosité de la première image de fluorescence temporaire du deuxième canal de couleur (EVB2) n'est pas supérieure à la luminosité maximale, l'utilisation de la première image de fluorescence temporaire (EVFB2) en tant que deuxième image de fluorescence (SG) du deuxième canal de couleur,
- dans le cas où la luminosité de la première image de fluorescence temporaire du deuxième canal de couleur (EVB2) est supérieure à la luminosité maximale, la détection d'une deuxième image de fluorescence temporaire dans le deuxième canal de couleur (ZVFB2) et l'utilisation de la deuxième image de fluorescence temporaire du deuxième canal de couleur (ZVFB2) en tant que deuxième image de fluorescence du deuxième canal de couleur (SG).

9. Dispositif (V1) destiné à détecter une liaison d'auto-anticorps d'un échantillon de patient à de l'acide désoxyribonucléique double brin à l'aide de cellules de Crithidia luciliae par microscopie de fluorescence et traitement d'image numérique, présentant
- un dispositif support (H) pour un substrat (S) qui présente plusieurs cellules de Crithidia luciliae (CR) et qui a été incubé avec un échantillon de patient présentant les auto-anticorps, avec un premier colorant fluorescent ainsi qu'en outre avec des anticorps secondaires qui sont marqués respectivement par un deuxième colorant fluorescent, le deuxième colorant fluorescent provoquant une coloration dans les zones dans lesquelles les anticorps de l'échantillon de patient ont été liés à l'ADNdb dans la zone de kinétoplaste respective de Crithidia luciliae respective,
- au moins une unité de détection d'image (K1, K2) pour détecter une première image de fluorescence (SR) du substrat dans un premier canal de couleur ainsi qu'en outre pour détecter une deuxième image de fluorescence (SG) du substrat (S) dans un deuxième canal de couleur,
et présentant en outre au moins une unité de calcul (R) qui est conçue pour
- identifier de premières images partielles (ZTB) respectives dans la première image de fluorescence (SR), qui représentent respectivement au moins une cellule de Crithidia Luciliae (CR),
- déterminer des deuxièmes images partielles (ZTB) respectives de la deuxième image de fluorescence (SG), qui correspondent aux premières images partielles (ETB) respectives de la première image de fluorescence (SR),
l'unité de calcul étant en outre conçue pour
- traiter respectivement au moins une quantité partielle des deuxièmes images partielles (ZTB) respectives au moyen d'un réseau neuronal convolutif (CNN) préentraîné pour déterminer un taux de liaison (IBM1, IBM2) respectif, qui indique un degré respectif d'une liaison d'auto-anticorps dans une zone de kinétoplaste (K) respective d'une cellule de Crithidia luciliae (CR) respective d'une deuxième image partielle (ZTB) respective,
le CNN traitant respectivement séparément, en soi, une deuxième image partielle (ZTB) respective pour déterminer, pour une deuxième image partielle (ZTB) respective, une sous-image respective qui représente une zone de kinétoplaste respective d'une cellule de Crithidia luciliae (CR) respective,
et le CNN déterminant le taux de liaison (IBM1) respectif sur la base de la sous-image (SUB) respective,
l'unité de calcul étant **caractérisée en ce que** le CNN génère, au cours de la détermination du taux de liaison individuel respectif, pour une deuxième image partielle respective, une Feature-Map (FFM1) respective,
**en ce qu'**en outre l'unité de calcul détermine, sur la base de la Feature-Map finale, un indice de confiance positif respectif concernant la présence d'une liaison d'auto-anticorps dans une zone de kinétoplaste respective pour une deuxième image partielle respective,
**en ce qu'**en outre l'unité de calcul choisit une quantité partielle des deuxièmes images partielles sur la base de l'indice de confiance positif respectif des deuxièmes images partielles respectives
et **en ce qu'**en outre l'unité de calcul détermine un taux de liaison total (GBM) concernant la liaison d'auto-anticorps de l'échantillon de patient à l'acide désoxyribonucléique double brin sur la base des différents taux de liaison, qui sont associés aux deuxièmes images partielles choisies de la quantité partielle.

10. Unité de calcul (R) qui est conçue pour, au cours d'un traitement d'image numérique,
- recevoir une première image de fluorescence (SG) qui représente une coloration d'un substrat, qui présente à son tour plusieurs cellules de Crithidia luciliae (CR), par un premier colorant fluorescent ainsi que pour recevoir une deuxième image de fluorescence (SG) qui représente une coloration du substrat par un deuxième colorant fluorescent, le deuxième colorant fluorescent provoquant une coloration dans les zones dans lesquelles les anticorps de l'échantillon de patient ont été liés à l'ADNdb dans la zone de kinétoplaste respective de Crithidia luciliae respective,
- identifier des premières images partielles (ETB) respectives dans la première image de fluorescence (SR), qui représentent respectivement au moins une cellule de Crithidia Luciliae (CR),
- déterminer des deuxièmes images partielles (ZTB) respectives de la deuxième image de fluorescence (SG), qui correspondent aux premières images partielles (ETB) respectives de la première image de fluorescence (SR),
l'unité de calcul étant outre conçue pour
- traiter respectivement au moins une quantité partielle des deuxièmes images partielles (ZTB) respectives au moyen d'un réseau neuronal convolutif (CNN) préentraîné pour déterminer un taux de liaison (IBM1, IBM2) respectif, qui indique un degré respectif d'une liaison d'auto-anticorps dans une zone de kinétoplaste (K) respective d'une cellule de Crithidia luciliae (CR) respective d'une deuxième image partielle (ZTB) respective,
le CNN traitant respectivement séparément, en soi, une deuxième image partielle (ZTB) respective pour déterminer, pour une deuxième image partielle (ZTB) respective, une sous-image respective qui représente une zone de kinétoplaste respective d'une cellule de Crithidia luciliae (CR) respective,
et le CNN déterminant le taux de liaison (IBM1) respectif sur la base de la sous-image (SUB) respective,
l'unité de calcul étant **caractérisée en ce que** le CNN génère, au cours de la détermination du taux de liaison individuel respectif, pour une deuxième image partielle respective, une Feature-Map (FFM1) respective,
**en ce qu'**en outre l'unité de calcul détermine, sur la base de la Feature-Map finale, un indice de confiance positif respectif concernant la présence d'une liaison d'auto-anticorps dans une zone de kinétoplaste respective pour une deuxième image partielle respective,
**en ce qu'**en outre l'unité de calcul choisit une quantité partielle des deuxièmes images partielles sur la base de l'indice de confiance positif respectif des deuxièmes images partielles respectives
et **en ce qu'**en outre l'unité de calcul détermine un taux de liaison total (GBM) concernant la liaison d'auto-anticorps de l'échantillon de patient à l'acide désoxyribonucléique double brin sur la base des différents taux de liaison, qui sont associés aux deuxièmes images partielles choisies de la quantité partielle.

11. Dispositif de réseau de données (DV) présentant
au moins une interface de données (DS4) destinée à recevoir une première image de fluorescence (BI1, SR) qui représente une coloration d'un substrat, qui présente à son tour plusieurs cellules de Crithidia luciliae, par un premier colorant fluorescent ainsi que pour recevoir une deuxième image de fluorescence (BI2, SG) qui représente une coloration du substrat par un deuxième colorant fluorescent, le deuxième colorant fluorescent provoquant une coloration dans les zones dans lesquelles les anticorps de l'échantillon de patient ont été liés à l'ADNdb dans la zone de kinétoplaste respective de Crithidia luciliae respective,
ainsi qu'en outre au moins une unité de calcul (R) qui est conçue pour, au cours d'un traitement d'image numérique,
- identifier des premières images partielles (ETB) respectives dans la première image de fluorescence (SR), qui présentent respectivement au moins une cellule de Crithidia Luciliae (CR),
- déterminer des deuxièmes images partielles (ZTB) respectives de la deuxième image de fluorescence (SG), qui correspondent aux premières images partielles (ETB) respectives de la première image de fluorescence (SR),
- l'unité de calcul étant outre conçue pour
- traiter respectivement au moins une quantité partielle des deuxièmes images partielles (ZTB) respectives au moyen d'un réseau neuronal convolutif (CNN) préentraîné pour déterminer un taux de liaison (IBM1, IBM2) respectif, qui indique un degré respectif d'une liaison d'auto-anticorps dans une zone de kinétoplaste (K) respective d'une cellule de Crithidia luciliae (CR) respective d'une deuxième image partielle (ZTB) respective,
- le CNN traitant respectivement séparément, en soi, une deuxième image partielle (ZTB) respective pour déterminer, pour une deuxième image partielle (ZTB) respective, une sous-image respective qui représente une zone de kinétoplaste respective d'une cellule de Crithidia luciliae (CR) respective et
- le CNN déterminant le taux de liaison (IBM1) respectif sur la base de la sous-image (SUB) respective,
l'unité de calcul étant **caractérisée en ce que** le CNN génère, au cours de la détermination du taux de liaison individuel respectif, pour une deuxième image partielle respective, une Feature-Map (FFM1) respective,
**en ce qu'**en outre l'unité de calcul détermine, sur la base de la Feature-Map finale, un indice de confiance positif respectif concernant la présence d'une liaison d'auto-anticorps dans une zone de kinétoplaste respective pour une deuxième image partielle respective,
**en ce qu'**en outre l'unité de calcul choisit une quantité partielle des deuxièmes images partielles sur la base de l'indice de confiance positif respectif des deuxièmes images partielles respectives
et **en ce qu'**en outre l'unité de calcul détermine un taux de liaison total (GBM) concernant la liaison d'auto-anticorps de l'échantillon de patient à l'acide désoxyribonucléique double brin sur la base des différents taux de liaison, qui sont associés aux deuxièmes images partielles choisies de la quantité partielle.

12. Procédé pour le traitement d'image numérique, présentant
- la réception d'une première image de fluorescence (SR) qui représente une coloration d'un substrat (S), qui présente à son tour plusieurs cellules de Crithidia luciliae (CR), par un premier colorant fluorescent ainsi qu'en outre pour recevoir une deuxième image de fluorescence (SG) qui représente une coloration du substrat (S) par un deuxième colorant fluorescent, le deuxième colorant fluorescent provoquant une coloration dans les zones dans lesquelles les anticorps de l'échantillon de patient ont été liés à l'ADNdb dans la zone de kinétoplaste respective des cellules de Crithidia luciliae respectives,
- l'identification de premières images partielles (ETB) respectives dans la première image de fluorescence (SR), qui représente respectivement une cellule de Crithidia Luciliae (CR),
- la détermination de deuxièmes images partielles (ZTB) respectives de la deuxième image de fluorescence (SG), qui correspondent aux premières images partielles (ETB) respectives de la première image de fluorescence (SR),
- présentant en outre
- le traitement respectif d'au moins une quantité partielle des deuxièmes images partielles (ZTB) respectives par un réseau neuronal convolutif (CNN) préentraîné pour déterminer un taux de liaison (IBM1, IBM2) respectif, qui indique un degré respectif d'une liaison d'auto-anticorps dans une zone de kinétoplaste (K) respective d'une cellule de Crithidia luciliae (CR) respective d'une deuxième image partielle (ZTB) respective,
- le CNN traitant respectivement séparément, en soi, une deuxième image partielle (ZTB) respective pour déterminer, pour une deuxième image partielle (ZTB) respective, une sous-image respective qui représente une zone de kinétoplaste respective d'une cellule de Crithidia luciliae (CR) respective et
- le CNN déterminant le taux de liaison (IBM1) respectif sur la base de la sous-image (SUB) respective,
- ainsi qu'en outre la détermination d'un taux de liaison total (GBM) d'une liaison d'auto-anticorps de l'échantillon de patient à de l'acide désoxyribonucléique double brin sur la base des taux de liaison (IBM1, IBM2) respectifs,
**caractérisé en ce que** le CNN génère, au cours de la détermination du taux de liaison individuel respectif, pour une deuxième image partielle respective, une Feature-Map (FFM1) respective,
**en ce qu'**en outre, sur la base de la Feature-Map finale, un indice de confiance positif respectif concernant la présence de la liaison d'auto-anticorps dans une zone de kinétoplaste respective pour une deuxième image partielle respective est déterminé, **en ce qu'**en outre une quantité partielle des deuxièmes images partielles est choisie sur la base de l'indice de confiance positif respectif des deuxièmes images partielles respectives,
et **en ce qu'**en outre un taux de liaison total (GBM) concernant la liaison d'auto-anticorps de l'échantillon de patient à l'acide désoxyribonucléique double brin est déterminé sur la base des différents taux de liaison, qui sont associés aux deuxièmes images partielles choisies de la quantité partielle.

13. Produit programme informatique (CPP)
comprenant des instructions qui permettent, lors de l'exécution du programme par un ordinateur, de mettre en oeuvre le procédé pour le traitement d'image numérique selon la revendication 12.
